# EUROPEAN PATENT SPECIFICATION

(11) **EP 3 286 314 B1**
(45) Date of publication and mention of the grant of the patent: **26.06.2019**
(21) Application number: 16718650.1
(22) Date of filing: 22.04.2016
(51) Int. Cl.: C12N 15/10

(54) **COW ANTIBODY SCAFFOLD POLYPEPTIDE METHOD AND COMPOSITION**
KUH-ANTIKÖRPERGERÜSTPOLYPEPTIDVERFAHREN UND -ZUSAMMENSETZUNG
PROCÉDÉ ET COMPOSITION DE POLYPEPTIDE D'ÉCHAFAUDAGE D'ANTICORPS BOVIN

(30) Priority: 23.04.2015 US 201562152004 P
(43) Date of publication of application: 28.02.2018
(73) Proprietor: F. Hoffmann-La Roche AG, 4070 Basel (CH); Roche Diagnostics GmbH, 68305 Mannheim (DE)
(72) Inventor: ALBERT, Thomas, Danville, CA 94506 (US); LYAMICHEV, Victor, Madison, WI 53711 (US); PATEL, Jigar, Verona, WI 53593 (US)
(74) Representative: Burger, Alexander
(86) International application number: PCT/EP2016/058989
(87) International publication number: WO 2016/170101

(56) References cited:
- EP-A1- 2 474 613
- EP-A1- 2 615 455
- EP-A1- 2 647 704
- EP-A1- 2 653 543
- WO-A2-2010/039852
- FENG WANG ET AL: "Reshaping Antibody Diversity", CELL, vol. 153, no. 6, 1 June 2013 (2013-06-01), pages 1379-1393, XP055169986, ISSN: 0092-8674, DOI: 10.1016/j.cell.2013.04.049
- Christine Klinguer-Hamour, et al.: "World Antibody-Drug Conjugate Summit", , vol. 6, no. 1 15 October 2013 (2013-10-15), 16 October 2013 (2013-10-16), pages 18-29, XP002760209, San Francisco, CA. Retrieved from the Internet: URL:http://www.ncbi.nlm.nih.gov/pmc/articl es/PMC3929441/pdf/mabs-6-18.pdf [retrieved on 2015-02-16] -& Vaughan V. Smider: "Cow Antibodies: A New Structural Class of Antibody Using Ultralong CDR3s", World ADC , 16 October 2013 (2013-10-16), XP055170029, San Francisco, CA Retrieved from the Internet: URL:http://adc-summit.com/uploads/files/24 63_ADC_/Vaughn_Smider.pdf [retrieved on 2015-02-16]
- ISAO FUKUDA ET AL: "In vitro evolution of single-chain antibodies using mRNA display", NUCLEIC ACIDS RESEARCH, OXFORD UNIVERSITY PRESS, GB, vol. 34, no. 19, 1 November 2006 (2006-11-01), pages E127-1, XP002639193, ISSN: 1362-4962, DOI: 10.1093/NAR/GKL618 [retrieved on 2006-09-29]
- XU LIHUI ET AL: "Directed evolution of high-affinity antibody mimics using mRNA display", CHEMISTRY AND BIOLOGY, CURRENT BIOLOGY, LONDON, GB, vol. 9, no. 8, 1 August 2002 (2002-08-01), pages 933-942, XP002293439, ISSN: 1074-5521, DOI: 10.1016/S1074-5521(02)00187-4
- P. TIMMERMAN ET AL: "A Combinatorial Approach for the Design of Complementarity-determining Region-derived Peptidomimetics with in Vitro Anti-tumoral Activity", JOURNAL OF BIOLOGICAL CHEMISTRY, vol. 284, no. 49, 4 December 2009 (2009-12-04), pages 34126-34134, XP055278875, US ISSN: 0021-9258, DOI: 10.1074/jbc.M109.041459

### SEQUENCE LISTING

<110> ROCHE DIAGNOSTICS GMBH F. HOFFMANN-LA ROCHE AG
<120> COW ANTIBODY SCAFFOLD POLYPEPTIDE METHOD AND COMPOSITION
<130> P32658-WO
<140>
   <141>
<150> 62/152,004
   <151> 2015-04-23
<160> 402
<170> PatentIn version 3.5
<210> 1
   <211> 70
   <212> PRT
   <213> Artificial Sequence
<220>
   <223> Description of Artificial Sequence: Synthetic polypeptide
<220>
   <221> MOD_RES
   <222> (16)..(55)
   <223> Any natural or non-natural amino acid
<220>
   <221> MISC_FEATURE
   <222> (16)..(55)
   <223> This region may encompass 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, 1 to 20, 1 to 21, 1 to 22, 1 to 23, 1 to 24, 1 to 25, 1 to 26, 1 to 27, 1 to 28, 1 to 29, 1 to 30,
<220>
   <221> MISC_FEATURE
   <222> (16)..(55)
   <223> continued from above; 1 to 31, 1 to 32, 1 to 33, 1 to 34, 1 to 35, 1 to 36, 1 to 37, 1 to 38, 1 to 39, or 1 to 40 residues, wherein some positions may be absent
<400> 1
   Met Gly Cys Thr Ser Val His Gln Glu Thr Lys Lys Tyr Gln Ser Xaa
   1               5                   10                  15
   Xaa Xaa Xaa Xaa Xaa Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Ser Tyr Thr Tyr Asn Tyr Glu His Val
               50                  55                  60
   Asp Val Trp Gly Cys Gly
   65                  70
<210> 2
   <211> 80
   <212> PRT
   <213> Artificial Sequence
<220>
   <223> Description of Artificial Sequence: Synthetic polypeptide
<220>
   <221> MOD_RES
   <222> (16)..(55)
   <223> Any natural or non-natural amino acid
<220>
   <221> MISC_FEATURE
   <222> (16)..(55)
   <223> This region may encompass 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, 1 to 20, 1 to 21, 1 to 22, 1 to 23, 1 to 24, 1 to 25, 1 to 26, 1 to 27, 1 to 28, 1 to 29, 1 to 30,
<220>
   <221> MISC_FEATURE
   <222> (16)..(55)
   <223> continued from above; 1 to 31, 1 to 32, 1 to 33, 1 to 34, 1 to 35, 1 to 36, 1 to 37, 1 to 38, 1 to 39, or 1 to 40 residues, wherein some positions may be absent
<400> 2
   Met Gly Cys Thr Ser Val His Gln Glu Thr Lys Lys Tyr Gln Ser Xaa
   1               5                   10                  15
   Xaa Xaa Xaa Xaa Xaa Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Ser Tyr Thr Tyr Asn Tyr Glu His Val
       50                  55                  60
   Asp Val Trp Gly Cys Gly Ser Ala Asp Tyr Lys Asp Asp Asp Asp Lys
   65                  70                  75                  80
<210> 3
   <211> 82
   <212> PRT
   <213> Artificial Sequence
<220>
   <223> Description of Artificial Sequence: Synthetic polypeptide
<220>
   <221> MOD_RES
   <222> (16)..(55)
   <223> Any natural or non-natural amino acid
<220>
   <221> MISC_FEATURE
   <222> (16)..(55)
   <223> This region may encompass 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, 1 to 20, 1 to 21, 1 to 22, 1 to 23, 1 to 24, 1 to 25, 1 to 26, 1 to 27, 1 to 28, 1 to 29, 1 to 30,
<220>
   <221> MISC_FEATURE
   <222> (16)..(55)
   <223> continued from above; 1 to 31, 1 to 32, 1 to 33, 1 to 34, 1 to 35, 1 to 36, 1 to 37, 1 to 38, 1 to 39, or 1 to 40 residues, wherein some positions may be absent
<400> 3
   Met Gly Cys Thr Ser Val His Gln Glu Thr Lys Lys Tyr Gln Ser Xaa
   1               5                   10                  15
   Xaa Xaa Xaa Xaa Xaa Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Ser Tyr Thr Tyr Asn Tyr Glu His Val
       50                  55                  60
   Asp Val Trp Gly Cys Gly Ser Ala Asp Tyr Lys Asp Asp Asp Asp Lys
   65                  70                  75                  80
   Lys Lys
<210> 4
   <211> 10
   <212> PRT
   <213> Artificial Sequence
<220>
   <223> Description of Artificial Sequence: Synthetic peptide
<220>
   <221> MOD_RES
   <222> (1)..(1)
   <223> Trp or Tyr
<220>
   <221> MOD_RES
   <222> (2)..(3)
   <223> Asp or Glu
<220>
   <221> MOD_RES
   <222> (4)..(4)
   <223> Trp or Tyr
<220>
   <221> MOD_RES
   <222> (8)..(8)
   <223> Gly, Ser or Thr
<400> 4
   Xaa Xaa Xaa Xaa Ala Leu Gln Xaa Tyr Asp
   1               5                   10
<210> 5
   <211> 5
   <212> PRT
   <213> Artificial Sequence
<220>
   <223> Description of Artificial Sequence: Synthetic peptide
<400> 5
   Arg Ser Lys Leu Gly
   1 5
<210> 6
   <211> 6
   <212> PRT
   <213> Artificial Sequence
<220>
   <223> Description of Artificial Sequence: Synthetic peptide
<400> 6
   Cys Thr Thr Val His Gln
   1 5
<210> 7
   <211> 6
   <212> PRT
   <213> Artificial Sequence
<220>
   <223> Description of Artificial Sequence: Synthetic peptide
<400> 7
   Cys Thr Ser Val His Gln
   1 5
<210> 8
   <211> 6
   <212> PRT
   <213> Artificial Sequence
<220>
   <223> Description of Artificial Sequence: Synthetic peptide
<400> 8
   Cys Ser Ser Val Thr Gln
   1 5
<210> 9
   <211> 6
   <212> PRT
   <213> Artificial Sequence
<220>
   <223> Description of Artificial Sequence: Synthetic peptide
<400> 9
   Cys Ser Thr Val His Gln
   1 5
<210> 10
   <211> 6
   <212> PRT
   <213> Artificial Sequence
<220>
   <223> Description of Artificial Sequence: Synthetic peptide
<400> 10
   Cys Ala Thr Val Arg Gln
   1 5
<210> 11
   <211> 6
   <212> PRT
   <213> Artificial Sequence
<220>
   <223> Description of Artificial Sequence: Synthetic peptide
<400> 11
   Cys Ser Pro Val His Gln
   1 5
<210> 12
   <211> 6
   <212> PRT
   <213> Artificial Sequence
<220>
   <223> Description of Artificial Sequence: Synthetic peptide
<400> 12
   Cys Ala Thr Val Tyr Gln
   1 5
<210> 13
   <211> 6
   <212> PRT
   <213> Artificial Sequence
<220>
   <223> Description of Artificial Sequence: Synthetic peptide
<400> 13
   Cys Thr Ala Val Tyr Gln
   1 5
<210> 14
   <211> 6
   <212> PRT
   <213> Artificial Sequence
<220>
   <223> Description of Artificial Sequence: Synthetic peptide
<400> 14
   Cys Thr Asn Val His Gln
   1               5
<210> 15
   <211> 6
   <212> PRT
   <213> Artificial Sequence
<220>
   <223> Description of Artificial Sequence: Synthetic peptide
<400> 15
   Cys Ala Thr Val His Gln
   1               5
<210> 16
   <211> 6
   <212> PRT
   <213> Artificial Sequence
<220>
   <223> Description of Artificial Sequence: Synthetic peptide
<400> 16
   Cys Thr Thr Val Arg Gln
   1               5
<210> 17
   <211> 6
   <212> PRT
   <213> Artificial Sequence
<220>
   <223> Description of Artificial Sequence: Synthetic peptide
<400> 17
   Cys Ser Thr Val Tyr Gln
   1               5
<210> 18
   <211> 6
   <212> PRT
   <213> Artificial Sequence
<220>
   <223> Description of Artificial Sequence: Synthetic peptide
<400> 18
   Cys Thr Ile Val His Gln
   1 5
<210> 19
   <211> 6
   <212> PRT
   <213> Artificial Sequence
<220>
   <223> Description of Artificial Sequence: Synthetic peptide
<400> 19
   Cys Ala Ile Val Tyr Gln
   1 5
<210> 20
   <211> 6
   <212> PRT
   <213> Artificial Sequence
<220>
   <223> Description of Artificial Sequence: Synthetic peptide
<400> 20
   Cys Thr Thr Val Tyr Gln
   1 5
<210> 21
   <211> 6
   <212> PRT
   <213> Artificial Sequence
<220>
   <223> Description of Artificial Sequence: Synthetic peptide
<400> 21
   Cys Thr Thr Val Phe Gln
   1 5
<210> 22
   <211> 6
   <212> PRT
   <213> Artificial Sequence
<220>
   <223> Description of Artificial Sequence: Synthetic peptide
<400> 22
   Cys Ala Ala Val Phe Gln
   1 5
<210> 23
   <211> 6
   <212> PRT
   <213> Artificial Sequence
<220>
   <223> Description of Artificial Sequence: Synthetic peptide
<400> 23
   Cys Gly Thr Val His Gln
   1               5
<210> 24
   <211> 6
   <212> PRT
   <213> Artificial Sequence
<220>
   <223> Description of Artificial Sequence: Synthetic peptide
<400> 24
   Cys Ala Ser Val His Gln
   1               5
<210> 25
   <211> 6
   <212> PRT
   <213> Artificial Sequence
<220>
   <223> Description of Artificial Sequence: Synthetic peptide
<400> 25
   Cys Thr Ala Val Phe Gln
   1               5
<210> 26
   <211> 6
   <212> PRT
   <213> Artificial Sequence
<220>
   <223> Description of Artificial Sequence: Synthetic peptide
<400> 26
   Cys Ala Thr Val Phe Gln
   1               5
<210> 27
   <211> 6
   <212> PRT
   <213> Artificial Sequence
<220>
   <223> Description of Artificial Sequence: Synthetic peptide
<400> 27
   Cys Ala Ala Ala His Gln
   1 5
<210> 28
   <211> 6
   <212> PRT
   <213> Artificial Sequence
<220>
   <223> Description of Artificial Sequence: Synthetic peptide
<400> 28
   Cys Val Val Val Tyr Gln
   1 5
<210> 29
   <211> 6
   <212> PRT
   <213> Artificial Sequence
<220>
   <223> Description of Artificial Sequence: Synthetic peptide
<4> 29
<210> 30
   <211> 6
   <212> PRT
   <213> Artificial Sequence
<220>
   <223> Description of Artificial Sequence: Synthetic peptide
<400> 30
   Cys Gly Ala Val His Gln
   1 5
<210> 31
   <211> 6
   <212> PRT
   <213> Artificial Sequence
<220>
   <223> Description of Artificial Sequence: Synthetic peptide
<400> 31
   Cys Ala Thr Lys Lys Gln
   1 5
<210> 32
   <211> 6
   <212> PRT
   <213> Artificial Sequence
<220>
   <223> Description of Artificial Sequence: Synthetic peptide
<400> 32
   Cys Ile Thr Val His Gln
   1               5
<210> 33
   <211> 6
   <212> PRT
   <213> Artificial Sequence
<220>
   <223> Description of Artificial Sequence: Synthetic peptide
<400> 33
   Cys Thr Ile Val His Gln
   1               5
<210> 34
   <211> 6
   <212> PRT
   <213> Artificial Sequence
<220>
   <223> Description of Artificial Sequence: Synthetic peptide
<400> 34
   Cys Ile Thr Ala His Gln
   1               5
<210> 35
   <211> 6
   <212> PRT
   <213> Artificial Sequence
<220>
   <223> Description of Artificial Sequence: Synthetic peptide
<400> 35
   Cys Val Ile Val His Gln
   1               5
<210> 36
   <211> 6
   <212> PRT
   <213> Artificial Sequence
<220>
   <223> Description of Artificial Sequence: Synthetic peptide
<400> 36
   Cys Thr Ile Val Asn Gln
   1 5
<210> 37
   <211> 6
   <212> PRT
   <213> Artificial Sequence
<220>
   <223> Description of Artificial Sequence: Synthetic peptide
<400> 37
   Cys Ala Ala Val His Gln
   1 5
<210> 38
   <211> 6
   <212> PRT
   <213> Artificial Sequence
<220>
   <223> Description of Artificial Sequence: Synthetic peptide
<400> 38
   Cys Gly Thr Val Tyr Gln
   1 5
<210> 39
   <211> 6
   <212> PRT
   <213> Artificial Sequence
<220>
   <223> Description of Artificial Sequence: Synthetic peptide
<400> 39
   Cys Val Thr Val His Gln
   1 5
<210> 40
   <211> 6
   <212> PRT
   <213> Artificial Sequence
<220>
   <223> Description of Artificial Sequence: Synthetic peptide
<400> 40
   Cys Thr Thr Val Leu Gln
   1 5
<210> 41
   <211> 6
   <212> PRT
   <213> Artificial Sequence
<220>
   <223> Description of Artificial Sequence: Synthetic peptide
<400> 41
   Cys Thr Thr Thr His Gln
   1               5
<210> 42
   <211> 6
   <212> PRT
   <213> Artificial Sequence
<220>
   <223> Description of Artificial Sequence: Synthetic peptide
<400> 42
   Cys Thr Thr Asp Tyr Gln
   1               5
<210> 43
   <211> 7
   <212> PRT
   <213> Artificial Sequence
<220>
   <223> Description of Artificial Sequence: Synthetic peptide
<400> 43
   Glu Thr Lys Lys Tyr Gln Ser
   1               5
<210> 44
   <211> 5
   <212> PRT
   <213> Artificial Sequence
<220>
   <223> Description of Artificial Sequence: Synthetic peptide
<400> 44
   Glu Thr Arg Lys Thr
   1               5
<210> 45
   <211> 7
   <212> PRT
   <213> Artificial Sequence
<220>
   <223> Description of Artificial Sequence: Synthetic peptide
<400> 45
   Arg Thr His Val Ser Arg Ser
   1 5
<210> 46
   <211> 7
   <212> PRT
   <213> Artificial Sequence
<220>
   <223> Description of Artificial Sequence: Synthetic peptide
<400> 46
   Lys Thr Thr Arg Lys Thr Cys
   1 5
<210> 47
   <211> 2
   <212> PRT
   <213> Artificial Sequence
<220>
   <223> Description of Artificial Sequence: Synthetic peptide
<400> 47
   Ile Phe
1
<210> 48
   <211> 9
   <212> PRT
   <213> Artificial Sequence
<220>
   <223> Description of Artificial Sequence: Synthetic peptide
<400> 48
   Lys Thr Arg Thr Thr Gln Gly Asn Thr
   1 5
<210> 49
   <211> 5
   <212> PRT
   <213> Artificial Sequence
<220>
   <223> Description of Artificial Sequence: Synthetic peptide
<400> 49
   Thr Thr Leu Arg Asp
   1 5
<210> 50
   <211> 20
   <212> PRT
   <213> Artificial Sequence
<220>
   <223> Description of Artificial Sequence: Synthetic peptide
<400> 50 Leu Lys Asp Asp 20
<210> 51
   <211> 20
   <212> PRT
   <213> Artificial Sequence
<220>
   <223> Description of Artificial Sequence: Synthetic peptide
<400> 51
   Lys Thr Arg Thr Thr Gln Gly Asn Asn Leu Ser Leu Met Val Thr Leu
   1               5                   10                  15
   Leu Lys Asp Asp
               20
<210> 52
   <211> 9
   <212> PRT
   <213> Artificial Sequence
<220>
   <223> Description of Artificial Sequence: Synthetic peptide
<400> 52
   Lys Thr Arg Thr Thr Gln Gly Asn Thr
   1               5
<210> 53
   <211> 19
   <212> PRT
   <213> Artificial Sequence
<220>
   <223> Description of Artificial Sequence: Synthetic peptide
<400> 53
   Lys Pro Gly Gln His Lys Gly Ile Leu Val Leu Met Val Thr Leu Leu
   1               5                   10                  15
   Lys Asp Asp
<210> 54
   <211> 19
   <212> PRT
   <213> Artificial Sequence
<220>
   <223> Description of Artificial Sequence: Synthetic peptide
<400> 54
   Lys Thr Arg Thr Thr Gln Gly Ile Leu Val Leu Met Val Thr Leu Leu
   1               5                   10                  15
   Lys Asp Asp
<210> 55
   <211> 5
   <212> PRT
   <213> Artificial Sequence
<220>
   <223> Description of Artificial Sequence: Synthetic peptide
<400> 55
   Glu Thr Lys Lys Asn
   1               5
<210> 56
   <211> 5
   <212> PRT
   <213> Artificial Sequence
<220>
   <223> Description of Artificial Sequence: Synthetic peptide
<400> 56
   Glu Ile Arg Lys Cys
   1               5
<210> 57
   <211> 5
   <212> PRT
   <213> Artificial Sequence
<220>
   <223> Description of Artificial Sequence: Synthetic peptide
<400> 57
   Gln Thr Arg Lys Cys
   1               5
<210> 58
   <211> 5
   <212> PRT
   <213> Artificial Sequence
<220>
   <223> Description of Artificial Sequence: Synthetic peptide
<400> 58
   Gln Thr Arg Lys Ser
   1               5
<210> 59
   <211> 7
   <212> PRT
   <213> Artificial Sequence
<220>
   <223> Description of Artificial Sequence: Synthetic peptide
<400> 59
   Lys Thr Asn Gln Ser Lys Asn
   1               5
<210> 60
   <211> 7
   <212> PRT
   <213> Artificial Sequence
<220>
   <223> Description of Artificial Sequence: Synthetic peptide
<400> 60
   Thr Thr His Gln Ile His Thr
   1               5
<210> 61
   <211> 7
   <212> PRT
   <213> Artificial Sequence
<220>
   <223> Description of Artificial Sequence: Synthetic peptide
<400> 61
   Lys Thr Thr Ser Ile Arg Ser
   1               5
<210> 62
   <211> 5
   <212> PRT
   <213> Artificial Sequence
<220>
   <223> Description of Artificial Sequence: Synthetic peptide
<400> 62
   Lys Thr Lys Lys Thr
   1               5
<210> 63
   <211> 5
   <212> PRT
   <213> Artificial Sequence
<220>
   <223> Description of Artificial Sequence: Synthetic peptide
<400> 63
   Lys Thr Lys Lys Leu
   1               5
<210> 64
   <211> 6
   <212> PRT
   <213> Artificial Sequence
<220>
   <223> Description of Artificial Sequence: Synthetic peptide
<400> 64
   His Thr Asn Lys Lys Arg
   1               5
<210> 65
   <211> 6
   <212> PRT
   <213> Artificial Sequence
<220>
   <223> Description of Artificial Sequence: Synthetic peptide
<400> 65
   His Thr Asn Gln Asn Arg
   1               5
<210> 66
   <211> 5
   <212> PRT
   <213> Artificial Sequence
<220>
   <223> Description of Artificial Sequence: Synthetic peptide
<400> 66
   Lys Thr Asn Glu Arg
   1               5
<210> 67
   <211> 6
   <212> PRT
   <213> Artificial Sequence
<220>
   <223> Description of Artificial Sequence: Synthetic peptide
<400> 67
   Lys Thr Asn Glu Arg Cys
   1               5
<210> 68
   <211> 7
   <212> PRT
   <213> Artificial Sequence
<220>
   <223> Description of Artificial Sequence: Synthetic peptide
<400> 68
   Lys Thr Asn Arg Glu Arg Cys
   1               5
<210> 69
   <211> 6
   <212> PRT
   <213> Artificial Sequence
<220>
   <223> Description of Artificial Sequence: Synthetic peptide
<400> 69
   Ser Thr Asn Lys Lys Asp
      1               5
<210> 70
   <211> 5
   <212> PRT
   <213> Artificial Sequence
<220>
   <223> Description of Artificial Sequence: Synthetic peptide
<400> 70
   Glu Thr Leu Ile Arg
   1               5
<210> 71
   <211> 5
   <212> PRT
   <213> Artificial Sequence
<220>
   <223> Description of Artificial Sequence: Synthetic peptide
<400> 71
   Lys Thr Arg Thr Thr
   1               5
<210> 72
   <211> 7
   <212> PRT
   <213> Artificial Sequence
<220>
   <223> Description of Artificial Sequence: Synthetic peptide
<400> 72
   Lys Thr Asn Arg Glu Met Ser
   1               5
<210> 73
   <211> 5
   <212> PRT
   <213> Artificial Sequence
<220>
   <223> Description of Artificial Sequence: Synthetic peptide
<400> 73
   Glu Thr Lys Arg Ser
   1               5
<210> 74
   <211> 5
   <212> PRT
   <213> Artificial Sequence
<220>
   <223> Description of Artificial Sequence: Synthetic peptide
<400> 74
   Arg Thr Arg Gln Arg
   1               5
<210> 75
   <211> 5
   <212> PRT
   <213> Artificial Sequence
<220>
   <223> Description of Artificial Sequence: Synthetic peptide
<400> 75
   Lys Thr Glu Thr Arg
   1 5
<210> 76
   <211> 7
   <212> PRT
   <213> Artificial Sequence
<220>
   <223> Description of Artificial Sequence: Synthetic peptide
<400> 76
   Lys Thr Asn Lys Lys Glu Ser
   1 5
<210> 77
   <211> 7
   <212> PRT
   <213> Artificial Sequence
<220>
   <223> Description of Artificial Sequence: Synthetic peptide
<400> 77
   Lys Ser Arg Lys Glu Ser Ser
   1 5
<210> 78
   <211> 5
   <212> PRT
   <213> Artificial Sequence
<220>
   <223> Description of Artificial Sequence: Synthetic peptide
<400> 78
   Glu Thr Arg Thr Asn
   1 5
<210> 79
   <211> 5
   <212> PRT
   <213> Artificial Sequence
<220>
   <223> Description of Artificial Sequence: Synthetic peptide
<400> 79
   Lys Thr Glu Lys His
   1 5
<210> 80
   <211> 5
   <212> PRT
   <213> Artificial Sequence
<220>
   <223> Description of Artificial Sequence: Synthetic peptide
<400> 80
   Lys Thr Lys Glu Leu
   1               5
<210> 81
   <211> 5
   <212> PRT
   <213> Artificial Sequence
<220>
   <223> Description of Artificial Sequence: Synthetic peptide
<400> 81
   His Thr Glu Pro Thr
   1               5
<210> 82
   <211> 5
   <212> PRT
   <213> Artificial Sequence
<220>
   <223> Description of Artificial Sequence: Synthetic peptide
<400> 82
   Glu Thr Arg Lys Ser
   1               5
<210> 83
   <211> 5
   <212> PRT
   <213> Artificial Sequence
<220>
   <223> Description of Artificial Sequence: Synthetic peptide
<400> 83
   Glu Thr Arg Lys Asp
   1               5
<210> 84
   <211> 5
   <212> PRT
   <213> Artificial Sequence
<220>
   <223> Description of Artificial Sequence: Synthetic peptide
<400> 84
   Glu Thr Lys Lys Ser
   1               5
<210> 85
   <211> 9
   <212> PRT
   <213> Artificial Sequence
<220>
   <223> Description of Artificial Sequence: Synthetic peptide
<400> 85
   Lys Thr Arg Thr Thr Gln Gly Asn Thr
   1               5
<210> 86
   <211> 7
   <212> PRT
   <213> Artificial Sequence
<220>
   <223> Description of Artificial Sequence: Synthetic peptide
<400> 86
   Lys Thr Asn Ser Gln Lys Ser
   1               5
<210> 87
   <211> 7
   <212> PRT
   <213> Artificial Sequence
<220>
   <223> Description of Artificial Sequence: Synthetic peptide
<400> 87
   Gln Thr His Lys Val Arg Asp
        1               5
<210> 88
   <211> 5
   <212> PRT
   <213> Artificial Sequence
<220>
   <223> Description of Artificial Sequence: Synthetic peptide
<400> 88
   Arg Thr Gly Gln Lys
   1 5
<210> 89
   <211> 5
   <212> PRT
   <213> Artificial Sequence
<220>
   <223> Description of Artificial Sequence: Synthetic peptide
<400> 89
   Lys Thr Lys Gln Asn
   1 5
<210> 90
   <211> 7
   <212> PRT
   <213> Artificial Sequence
<220>
   <223> Description of Artificial Sequence: Synthetic peptide
<400> 90
   Gln Thr His Glu Lys Arg Ser
   1 5
<210> 91
   <211> 7
   <212> PRT
   <213> Artificial Sequence
<220>
   <223> Description of Artificial Sequence: Synthetic peptide
<400> 91
   Gln Thr Lys Arg Lys Ser Gly
   1 5
<210> 92
   <211> 5
   <212> PRT
   <213> Artificial Sequence
<220>
   <223> Description of Artificial Sequence: Synthetic peptide
<400> 92
   Glu Thr Lys Arg Thr
   1 5
<210> 93
   <211> 5
   <212> PRT
   <213> Artificial Sequence
<220>
   <223> Description of Artificial Sequence: Synthetic peptide
<400> 93
   Glu Thr Gln Lys Ser
   1               5
<210> 94
   <211> 5
   <212> PRT
   <213> Artificial Sequence
<220>
   <223> Description of Artificial Sequence: Synthetic peptide
<400> 94
   Glu Thr His Lys Arg
   1               5
<210> 95
   <211> 5
   <212> PRT
   <213> Artificial Sequence
<220>
   <223> Description of Artificial Sequence: Synthetic peptide
<400> 95
   Glu Thr His Lys Asn
   1               5
<210> 96
   <211> 7
   <212> PRT
   <213> Artificial Sequence
<220>
   <223> Description of Artificial Sequence: Synthetic peptide
<400> 96
   Gln Thr His Ala Thr Arg Arg
   1               5
<210> 97
   <211> 7
   <212> PRT
   <213> Artificial Sequence
<220>
   <223> Description of Artificial Sequence: Synthetic peptide
<400> 97
   Arg Thr Glu Gly Gln Gln Ser
   1               5
<210> 98
   <211> 7
   <212> PRT
   <213> Artificial Sequence
<220>
   <223> Description of Artificial Sequence: Synthetic peptide
<400> 98
   Glu Thr Lys Thr Lys Ser Gly
   1               5
<210> 99
   <211> 7
   <212> PRT
   <213> Artificial Sequence
<220>
   <223> Description of Artificial Sequence: Synthetic peptide
<400> 99
   His Thr Lys Glu Ile Lys Thr
   1               5
<210> 100
   <211> 7
   <212> PRT
   <213> Artificial Sequence
<220>
   <223> Description of Artificial Sequence: Synthetic peptide
<400> 100
   Glu Thr His Gln Gln Arg Gly
   1               5
<210> 101
   <211> 5
   <212> PRT
   <213> Artificial Sequence
<220>
   <223> Description of Artificial Sequence: Synthetic peptide
<400> 101
   Lys Thr Glu Lys Lys
   1               5
<210> 102
   <211> 5
   <212> PRT
   <213> Artificial Sequence
<220>
   <223> Description of Artificial Sequence: Synthetic peptide
<400> 102
   Arg Thr Gln Lys Ser
   1 5
<210> 103
   <211> 5
   <212> PRT
   <213> Artificial Sequence
<220>
   <223> Description of Artificial Sequence: Synthetic peptide
<400> 103
   Gln Thr Asn Lys Arg
   1 5
<210> 104
   <211> 6
   <212> PRT
   <213> Artificial Sequence
<220>
   <223> Description of Artificial Sequence: Synthetic peptide
<400> 104
   Glu Thr Gln Arg Thr Ser
   1 5
<210> 105
   <211> 4
   <212> PRT
   <213> Artificial Sequence
<220>
   <223> Description of Artificial Sequence: Synthetic peptide
<400> 105
   Lys Asp Lys His
   1
<210> 106
   <211> 8
   <212> PRT
   <213> Artificial Sequence
<220>
   <223> Description of Artificial Sequence: Synthetic peptide
<400> 106
   Gln Thr Thr Glu Lys Gly Lys Thr
   1 5
<210> 107
   <211> 5
   <212> PRT
   <213> Artificial Sequence
<220>
   <223> Description of Artificial Sequence: Synthetic peptide
<400> 107
   Lys Thr Asp Val Thr
   1 5
<210> 108
   <211> 7
   <212> PRT
   <213> Artificial Sequence
<220>
   <223> Description of Artificial Sequence: Synthetic peptide
<400> 108
   Glu Thr His Thr Gln Arg Thr
   1 5
<210> 109
   <211> 5
   <212> PRT
   <213> Artificial Sequence
<220>
   <223> Description of Artificial Sequence: Synthetic peptide
<400> 109
   Lys Thr Glu Lys Ser
   1 5
<210> 110
   <211> 7
   <212> PRT
   <213> Artificial Sequence
<220>
   <223> Description of Artificial Sequence: Synthetic peptide
<400> 110
   Lys Thr Asn Gln Lys Trp Gly
   1               5
<210> 111
   <211> 5
   <212> PRT
   <213> Artificial Sequence
<220>
   <223> Description of Artificial Sequence: Synthetic peptide
<400> 111
   Glu Thr Arg Thr Asn
   1               5
<210> 112
   <211> 7
   <212> PRT
   <213> Artificial Sequence
<220>
   <223> Description of Artificial Sequence: Synthetic peptide
<400> 112
   Lys Thr Thr Thr Thr Lys Ser
   1               5
<210> 113
   <211> 5
   <212> PRT
   <213> Artificial Sequence
<220>
   <223> Description of Artificial Sequence: Synthetic peptide
<400> 113
   Lys Thr Glu Gln Arg
   1               5
<210> 114
   <211> 5
   <212> PRT
   <213> Artificial Sequence
<220>
   <223> Description of Artificial Sequence: Synthetic peptide
<400> 114
   Met Thr Ile Lys Thr
   1 5
<210> 115
   <211> 7
   <212> PRT
   <213> Artificial Sequence
<220>
   <223> Description of Artificial Sequence: Synthetic peptide
<400> 115
   Lys Thr Glu Ser Val Arg Ser
   1 5
<210> 116
   <211> 5
   <212> PRT
   <213> Artificial Sequence
<220>
   <223> Description of Artificial Sequence: Synthetic peptide
<400> 116
   Gln Thr Thr Asn Arg
   1 5
<210> 117
   <211> 5
   <212> PRT
   <213> Artificial Sequence
<220>
   <223> Description of Artificial Sequence: Synthetic peptide
<400> 117
   Leu Thr Lys Lys Thr
   1 5
<210> 118
   <211> 6
   <212> PRT
   <213> Artificial Sequence
<220>
   <223> Description of Artificial Sequence: Synthetic peptide
<400> 118
   Lys Thr Thr Gln Gln Ser
   1 5
<210> 119
   <211> 6
   <212> PRT
   <213> Artificial Sequence
<220>
   <223> Description of Artificial Sequence: Synthetic peptide
<400> 119
   His Thr Asn Lys Lys Arg
   1 5
<210> 120
   <211> 5
   <212> PRT
   <213> Artificial Sequence
<220>
   <223> Description of Artificial Sequence: Synthetic peptide
<400> 120
   Gln Thr Arg Lys Ser
   1 5
<210> 121
   <211> 5
   <212> PRT
   <213> Artificial Sequence
<220>
   <223> Description of Artificial Sequence: Synthetic peptide
<400> 121
   Lys Thr Ala Arg Ser
   1 5
<210> 122
   <211> 2
   <212> PRT
   <213> Artificial Sequence
<220>
   <223> Description of Artificial Sequence: Synthetic peptide
<400> 122
   Ile Cys
   1
<210> 123
   <211> 5
   <212> PRT
   <213> Artificial Sequence
<220>
   <223> Description of Artificial Sequence: Synthetic peptide
<400> 123
   Gln Thr Thr Lys Arg
   1 5
<210> 124
   <211> 5
   <212> PRT
   <213> Artificial Sequence
<220>
   <223> Description of Artificial Sequence: Synthetic peptide
<400> 124
   Leu Thr Arg Ala His
   1 5
<210> 125
   <211> 5
   <212> PRT
   <213> Artificial Sequence
<220>
   <223> Description of Artificial Sequence: Synthetic peptide
<400> 125
   Arg Thr Glu Lys Ser
   1 5
<210> 126
   <211> 5
   <212> PRT
   <213> Artificial Sequence
<220>
   <223> Description of Artificial Sequence: Synthetic peptide
<400> 126
   Arg Thr Lys Arg Ser
   1 5
<210> 127
   <211> 5
   <212> PRT
   <213> Artificial Sequence
<220>
   <223> Description of Artificial Sequence: Synthetic peptide
<400> 127
   Ile Thr His Lys Glu
   1 5
<210> 128
   <211> 7
   <212> PRT
   <213> Artificial Sequence
<220>
   <223> Description of Artificial Sequence: Synthetic peptide
<400> 128
   His Thr Thr Thr Lys Asn Thr
   1               5
<210> 129
   <211> 6
   <212> PRT
   <213> Artificial Sequence
<220>
   <223> Description of Artificial Sequence: Synthetic peptide
<400> 129
   Lys Thr Leu Glu Lys Thr
   1               5
<210> 130
   <211> 6
   <212> PRT
   <213> Artificial Sequence
<220>
   <223> Description of Artificial Sequence: Synthetic peptide
<400> 130
   Glu Val Gln Lys Lys Thr
   1               5
<210> 131
   <211> 5
   <212> PRT
   <213> Artificial Sequence
<220>
   <223> Description of Artificial Sequence: Synthetic peptide
<400> 131
   Lys Thr Gln Arg Ser
   1               5
<210> 132
   <211> 7
   <212> PRT
   <213> Artificial Sequence
<220>
   <223> Description of Artificial Sequence: Synthetic peptide
<400> 132
   Glu Thr Lys Thr Arg Ser Thr
   1 5
<210> 133
   <211> 7
   <212> PRT
   <213> Artificial Sequence
<220>
   <223> Description of Artificial Sequence: Synthetic peptide
<400> 133
   Arg Thr Thr Thr Glu Arg Ser
   1 5
<210> 134
   <211> 5
   <212> PRT
   <213> Artificial Sequence
<220>
   <223> Description of Artificial Sequence: Synthetic peptide
<400> 134
   Lys Thr Gln Arg Thr
   1 5
<210> 135
   <211> 9
   <212> PRT
   <213> Artificial Sequence
<220>
   <223> Description of Artificial Sequence: Synthetic peptide
<400> 135
   Lys Thr Arg Thr Thr Gln Gly Asn Thr
   1 5
<210> 136
   <211> 8
   <212> PRT
   <213> Artificial Sequence
<220>
   <223> Description of Artificial Sequence: Synthetic peptide
<400> 136
   Cys Tyr Thr Tyr Asn Tyr Glu Phe
   1 5
<210> 137
   <211> 8
   <212> PRT
   <213> Artificial Sequence
<220>
   <223> Description of Artificial Sequence: Synthetic peptide
<400> 137
   His Tyr Thr Tyr Thr Tyr Asp Phe
   1 5
<210> 138
   <211> 8
   <212> PRT
   <213> Artificial Sequence
<220>
   <223> Description of Artificial Sequence: Synthetic peptide
<400> 138
   His Tyr Thr Tyr Thr Tyr Glu Trp
   1 5
<210> 139
   <211> 8
   <212> PRT
   <213> Artificial Sequence
<220>
   <223> Description of Artificial Sequence: Synthetic peptide
<400> 139
   Lys His Arg Tyr Thr Tyr Glu Trp
   1 5
<210> 140
   <211> 8
   <212> PRT
   <213> Artificial Sequence
<220>
   <223> Description of Artificial Sequence: Synthetic peptide
<400> 140
   Asn Tyr Ile Tyr Lys Tyr Ser Phe
   1 5
<210> 141
   <211> 8
   <212> PRT
   <213> Artificial Sequence
<220>
   <223> Description of Artificial Sequence: Synthetic peptide
<400> 141
   Pro Tyr Ile Tyr Thr Tyr Gln Phe
   1 5
<210> 142
   <211> 8
   <212> PRT
   <213> Artificial Sequence
<220>
   <223> Description of Artificial Sequence: Synthetic peptide
<400> 142
   Ser Phe Thr Tyr Thr Tyr Glu Trp
   1 5
<210> 143
   <211> 8
   <212> PRT
   <213> Artificial Sequence
<220>
   <223> Description of Artificial Sequence: Synthetic peptide
<400> 143
   Ser Tyr Ile Tyr Ile Tyr Gln Trp
   1 5
<210> 144
   <211> 8
   <212> PRT
   <213> Artificial Sequence
<220>
   <223> Description of Artificial Sequence: Synthetic peptide
<400> 144
   Ser Tyr Asn Tyr Thr Tyr Ser Trp
   1 5
<210> 145
   <211> 8
   <212> PRT
   <213> Artificial Sequence
<220>
   <223> Description of Artificial Sequence: Synthetic peptide
<400> 145
   Ser Tyr Ser Tyr Ser Tyr Glu Tyr
   1               5
<210> 146
   <211> 8
   <212> PRT
   <213> Artificial Sequence
<220>
   <223> Description of Artificial Sequence: Synthetic peptide
<400> 146
   Ser Tyr Thr Tyr Asn Tyr Asp Phe
   1               5
<210> 147
   <211> 8
   <212> PRT
   <213> Artificial Sequence
<220>
   <223> Description of Artificial Sequence: Synthetic peptide
<400> 147
   Ser Tyr Thr Tyr Asn Tyr Glu Trp
   1               5
<210> 148
   <211> 8
   <212> PRT
   <213> Artificial Sequence
<220>
   <223> Description of Artificial Sequence: Synthetic peptide
<400> 148
   Ser Tyr Thr Tyr Asn Tyr Gln Phe
   1               5
<210> 149
   <211> 8
   <212> PRT
   <213> Artificial Sequence
<220>
   <223> Description of Artificial Sequence: Synthetic peptide
<400> 149
   Ser Tyr Val Trp Thr His Asn Phe
   1               5
<210> 150
   <211> 8
   <212> PRT
   <213> Artificial Sequence
<220>
   <223> Description of Artificial Sequence: Synthetic peptide
<400> 150
   Thr Tyr Lys Tyr Val Tyr Glu Trp
   1 5
<210> 151
   <211> 8
   <212> PRT
   <213> Artificial Sequence
<220>
   <223> Description of Artificial Sequence: Synthetic peptide
<400> 151
   Thr Tyr Thr Tyr Thr Tyr Glu Phe
   1 5
<210> 152
   <211> 8
   <212> PRT
   <213> Artificial Sequence
<220>
   <223> Description of Artificial Sequence: Synthetic peptide
<400> 152
   Thr Tyr Thr Tyr Thr Tyr Glu Trp
   1 5
<210> 153
   <211> 8
   <212> PRT
   <213> Artificial Sequence
<220>
   <223> Description of Artificial Sequence: Synthetic peptide
<400> 153
   Val Phe Thr Tyr Thr Tyr Glu Phe
   1 5
<210> 154
   <211> 6
   <212> PRT
   <213> Artificial Sequence
<220>
   <223> Description of Artificial Sequence: Synthetic peptide
<400> 154
   Ala Tyr Thr Tyr Glu Trp
   1               5
<210> 155
   <211> 6
   <212> PRT
   <213> Artificial Sequence
<220>
   <223> Description of Artificial Sequence: Synthetic peptide
<400> 155
   Asp Tyr Ile Tyr Thr Tyr
   1               5
<210> 156
   <211> 6
   <212> PRT
   <213> Artificial Sequence
<220>
   <223> Description of Artificial Sequence: Synthetic peptide
<400> 156
   Ile His Ser Tyr Glu Phe
   1               5
<210> 157
   <211> 6
   <212> PRT
   <213> Artificial Sequence
<220>
   <223> Description of Artificial Sequence: Synthetic peptide
<400> 157
   Ser Phe Thr Tyr Glu Phe
   1               5
<210> 158
   <211> 6
   <212> PRT
   <213> Artificial Sequence
<220>
   <223> Description of Artificial Sequence: Synthetic peptide
<400> 158
   Ser His Ser Tyr Glu Phe
   1 5
<210> 159
   <211> 6
   <212> PRT
   <213> Artificial Sequence
<220>
   <223> Description of Artificial Sequence: Synthetic peptide
<400> 159
   Thr His Thr Tyr Glu Phe
   1 5
<210> 160
   <211> 6
   <212> PRT
   <213> Artificial Sequence
<220>
   <223> Description of Artificial Sequence: Synthetic peptide
<400> 160
   Thr Trp Thr Tyr Glu Phe
   1 5
<210> 161
   <211> 6
   <212> PRT
   <213> Artificial Sequence
<220>
   <223> Description of Artificial Sequence: Synthetic peptide
<400> 161
   Thr Tyr Asn Tyr Glu Trp
   1 5
<210> 162
   <211> 6
   <212> PRT
   <213> Artificial Sequence
<220>
   <223> Description of Artificial Sequence: Synthetic peptide
<400> 162
   Thr Tyr Ser Tyr Glu Phe
   1 5
<210> 163
   <211> 6
   <212> PRT
   <213> Artificial Sequence
<220>
   <223> Description of Artificial Sequence: Synthetic peptide
<400> 163
   Thr Tyr Ser Tyr Glu His
   1 5
<210> 164
   <211> 6
   <212> PRT
   <213> Artificial Sequence
<220>
   <223> Description of Artificial Sequence: Synthetic peptide
<400> 164
   Thr Tyr Thr Tyr Asp Phe
   1 5
<210> 165
   <211> 6
   <212> PRT
   <213> Artificial Sequence
<220>
   <223> Description of Artificial Sequence: Synthetic peptide
<400> 165
   Thr Tyr Thr Tyr Glu Phe
   1 5
<210> 166
   <211> 6
   <212> PRT
   <213> Artificial Sequence
<220>
   <223> Description of Artificial Sequence: Synthetic peptide
<400> 166
   Thr Tyr Thr Tyr Glu Trp
   1 5
<210> 167
   <211> 4
   <212> PRT
   <213> Artificial Sequence
<220>
   <223> Description of Artificial Sequence: Synthetic peptide
<400> 167
   Ala Tyr Glu Phe
   1
<210> 168
   <211> 4
   <212> PRT
   <213> Artificial Sequence
<220>
   <223> Description of Artificial Sequence: Synthetic peptide
<400> 168
   Ala Tyr Ser Phe
   1
<210> 169
   <211> 4
   <212> PRT
   <213> Artificial Sequence
<220>
   <223> Description of Artificial Sequence: Synthetic peptide
<400> 169
   Ala Tyr Ser Tyr
   1
<210> 170
   <211> 4
   <212> PRT
   <213> Artificial Sequence
<220>
   <223> Description of Artificial Sequence: Synthetic peptide
<400> 170
   Cys Tyr Ser Phe
   1
<210> 171
   <211> 4
   <212> PRT
   <213> Artificial Sequence
<220>
   <223> Description of Artificial Sequence: Synthetic peptide
<400> 171
   Asp Tyr Thr Tyr
   1
<210> 172
   <211> 4
   <212> PRT
   <213> Artificial Sequence
<220>
   <223> Description of Artificial Sequence: Synthetic peptide
<400> 172
   Lys Tyr Glu His
   1
<210> 173
   <211> 4
   <212> PRT
   <213> Artificial Sequence
<220>
   <223> Description of Artificial Sequence: Synthetic peptide
<400> 173
   Lys Tyr Glu Trp
   1
<210> 174
   <211> 4
   <212> PRT
   <213> Artificial Sequence
<220>
   <223> Description of Artificial Sequence: Synthetic peptide
<400> 174
   Met Tyr Glu Phe
   1
<210> 175
   <211> 4
   <212> PRT
   <213> Artificial Sequence
<220>
   <223> Description of Artificial Sequence: Synthetic peptide
<400> 175
   Asn Trp Ile Tyr
   1
<210> 176
   <211> 4
   <212> PRT
   <213> Artificial Sequence
<220>
   <223> Description of Artificial Sequence: Synthetic peptide
<400> 176
   Asn Tyr Asp Tyr
   1
<210> 177
   <211> 4
   <212> PRT
   <213> Artificial Sequence
<220>
   <223> Description of Artificial Sequence: Synthetic peptide
<400> 177
   Asn Tyr Gln Trp
   1
<210> 178
   <211> 4
   <212> PRT
   <213> Artificial Sequence
<220>
   <223> Description of Artificial Sequence: Synthetic peptide
<400> 178
   Asn Tyr Ser Phe
   1
<210> 179
   <211> 4
   <212> PRT
   <213> Artificial Sequence
<220>
   <223> Description of Artificial Sequence: Synthetic peptide
<400> 179
   Pro Tyr Glu Trp
   1
<210> 180
   <211> 4
   <212> PRT
   <213> Artificial Sequence
<220>
   <223> Description of Artificial Sequence: Synthetic peptide
<400> 180
   Arg Tyr Asn Trp
   1
<210> 181
   <211> 4
   <212> PRT
   <213> Artificial Sequence
<220>
   <223> Description of Artificial Sequence: Synthetic peptide
<400> 181
   Arg Tyr Thr Tyr
   1
<210> 182
   <211> 4
   <212> PRT
   <213> Artificial Sequence
<220>
   <223> Description of Artificial Sequence: Synthetic peptide
<400> 182
   Ser Tyr Glu Phe
   1
<210> 183
   <211> 4
   <212> PRT
   <213> Artificial Sequence
<220>
   <223> Description of Artificial Sequence: Synthetic peptide
<400> 183
   Ser Tyr Glu His
   1
<210> 184
   <211> 4
   <212> PRT
   <213> Artificial Sequence
<220>
   <223> Description of Artificial Sequence: Synthetic peptide
<400> 184
   Ser Tyr Glu Trp
   1
<210> 185
   <211> 4
   <212> PRT
   <213> Artificial Sequence
<220>
   <223> Description of Artificial Sequence: Synthetic peptide
<400> 185
   Ser Tyr Lys Trp
   1
<210> 186
   <211> 4
   <212> PRT
   <213> Artificial Sequence
<220>
   <223> Description of Artificial Sequence: Synthetic peptide
<400> 186
   Ser Tyr Thr Tyr
   1
<210> 187
   <211> 4
   <212> PRT
   <213> Artificial Sequence
<220>
   <223> Description of Artificial Sequence: Synthetic peptide
<400> 187
   Thr Tyr Asp Phe
   1
<210> 188
   <211> 4
   <212> PRT
   <213> Artificial Sequence
<220>
   <223> Description of Artificial Sequence: Synthetic peptide
<400> 188
   Thr Tyr Glu Phe
   1
<210> 189
   <211> 4
   <212> PRT
   <213> Artificial Sequence
<220>
   <223> Description of Artificial Sequence: Synthetic peptide
<400> 189
   Thr Tyr Glu Trp
   1
<210> 190
   <211> 4
   <212> PRT
   <213> Artificial Sequence
<220>
   <223> Description of Artificial Sequence: Synthetic peptide
<400> 190
   Thr Tyr Gln Trp
   1
<210> 191
   <211> 4
   <212> PRT
   <213> Artificial Sequence
<220>
   <223> Description of Artificial Sequence: Synthetic peptide
<400> 191
   Thr Tyr Thr Tyr
   1
<210> 192
   <211> 4
   <212> PRT
   <213> Artificial Sequence
<220>
   <223> Description of Artificial Sequence: Synthetic peptide
<400> 192
   Val Tyr Glu Trp
   1
<210> 193
   <211> 5
   <212> PRT
   <213> Artificial Sequence
<220>
   <223> Description of Artificial Sequence: Synthetic peptide
<400> 193
   Tyr Val Asp Ala Trp
   1 5
<210> 194
   <211> 5
   <212> PRT
   <213> Artificial Sequence
<220>
   <223> Description of Artificial Sequence: Synthetic peptide
<400> 194
   His Val Asp Val Trp
   1 5
<210> 195
   <211> 5
   <212> PRT
   <213> Artificial Sequence
<220>
   <223> Description of Artificial Sequence: Synthetic peptide
<400> 195
   His Val Asp Ala Trp
   1 5
<210> 196
   <211> 5
   <212> PRT
   <213> Artificial Sequence
<220>
   <223> Description of Artificial Sequence: Synthetic peptide
<400> 196
   Gly Val Asp Ala Trp
   1 5
<210> 197
   <211> 5
   <212> PRT
   <213> Artificial Sequence
<220>
   <223> Description of Artificial Sequence: Synthetic peptide
<400> 197
   Tyr Ile Asp Ala Trp
   1 5
<210> 198
   <211> 5
   <212> PRT
   <213> Artificial Sequence
<220>
   <223> Description of Artificial Sequence: Synthetic peptide
<400> 198
   His Val Asp Ser Trp
   1               5
<210> 199
   <211> 5
   <212> PRT
   <213> Artificial Sequence
<220>
   <223> Description of Artificial Sequence: Synthetic peptide
<400> 199
   His Ile Asp Ala Trp
   1               5
<210> 200
   <211> 5
   <212> PRT
   <213> Artificial Sequence
<220>
   <223> Description of Artificial Sequence: Synthetic peptide
<400> 200
   Tyr Val Asp Thr Trp
   1               5
<210> 201
   <211> 5
   <212> PRT
   <213> Artificial Sequence
<220>
   <223> Description of Artificial Sequence: Synthetic peptide
<400> 201
   Asn Val Asp Ala Trp
   1               5
<210> 202
   <211> 5
   <212> PRT
   <213> Artificial Sequence
<220>
   <223> Description of Artificial Sequence: Synthetic peptide
<400> 202
   His Val Asn Ala Trp
   1 5
<210> 203
   <211> 5
   <212> PRT
   <213> Artificial Sequence
<220>
   <223> Description of Artificial Sequence: Synthetic peptide
<400> 203
   Tyr Val Thr Ala Trp
   1 5
<210> 204
   <211> 5
   <212> PRT
   <213> Artificial Sequence
<220>
   <223> Description of Artificial Sequence: Synthetic peptide
<400> 204
   Tyr Ile Thr Ala Trp
   1 5
<210> 205
   <211> 5
   <212> PRT
   <213> Artificial Sequence
<220>
   <223> Description of Artificial Sequence: Synthetic peptide
<400> 205
   Tyr Val Glu Ala Trp
   1 5
<210> 206
   <211> 5
   <212> PRT
   <213> Artificial Sequence
<220>
   <223> Description of Artificial Sequence: Synthetic peptide
<400> 206
   His Val Asp Glu Trp
   1 5
<210> 207
   <211> 5
   <212> PRT
   <213> Artificial Sequence
<220>
   <223> Description of Artificial Sequence: Synthetic peptide
<400> 207
   His Val Asp Thr Trp
   1 5
<210> 208
   <211> 5
   <212> PRT
   <213> Artificial Sequence
<220>
   <223> Description of Artificial Sequence: Synthetic peptide
<400> 208
   Tyr Ala Asp Ala Trp
   1 5
<210> 209
   <211> 5
   <212> PRT
   <213> Artificial Sequence
<220>
   <223> Description of Artificial Sequence: Synthetic peptide
<400> 209
   Tyr Gly Asp Ala Trp
   1 5
<210> 210
   <211> 5
   <212> PRT
   <213> Artificial Sequence
<220>
   <223> Description of Artificial Sequence: Synthetic peptide
<400> 210
   His Ala Asp Ala Trp
   1 5
<210> 211
   <211> 5
   <212> PRT
   <213> Artificial Sequence
<220>
   <223> Description of Artificial Sequence: Synthetic peptide
<400> 211
   Tyr Val Glu Ala Trp
   1 5
<210> 212
   <211> 5
   <212> PRT
   <213> Artificial Sequence
<220>
   <223> Description of Artificial Sequence: Synthetic peptide
<400> 212
   Asn Val Asp Ser Trp
   1 5
<210> 213
   <211> 5
   <212> PRT
   <213> Artificial Sequence
<220>
   <223> Description of Artificial Sequence: Synthetic peptide
<400> 213
   Asn Val Asp Ala Trp
   1 5
<210> 214
   <211> 5
   <212> PRT
   <213> Artificial Sequence
<220>
   <223> Description of Artificial Sequence: Synthetic peptide
<400> 214
   Gly Val Asp Ala Trp
   1 5
<210> 215
   <211> 5
   <212> PRT
   <213> Artificial Sequence
<220>
   <223> Description of Artificial Sequence: Synthetic peptide
<400> 215
   Asn Ile Asp Ala Trp
   1 5
<210> 216
   <211> 5
   <212> PRT
   <213> Artificial Sequence
<220>
   <223> Description of Artificial Sequence: Synthetic peptide
<400> 216
   Arg Ile Asp Val Met
   1 5
<210> 217
   <211> 5
   <212> PRT
   <213> Artificial Sequence
<220>
   <223> Description of Artificial Sequence: Synthetic peptide
<400> 217
   Tyr Val Glu Thr Trp
   1 5
<210> 218
   <211> 5
   <212> PRT
   <213> Artificial Sequence
<220>
   <223> Description of Artificial Sequence: Synthetic peptide
<400> 218
   Tyr Val Asn Ala Trp
   1 5
<210> 219
   <211> 5
   <212> PRT
   <213> Artificial Sequence
<220>
   <223> Description of Artificial Sequence: Synthetic peptide
<400> 219
   His Ala Asp Val Trp
   1 5
<210> 220
   <211> 5
   <212> PRT
   <213> Artificial Sequence
<220>
   <223> Description of Artificial Sequence: Synthetic peptide
<400> 220
   His Val Glu Ser Trp
   1 5
<210> 221
   <211> 5
   <212> PRT
   <213> Artificial Sequence
<220>
   <223> Description of Artificial Sequence: Synthetic peptide
<400> 221
   His Val Glu Thr Trp
   1 5
<210> 222
   <211> 5
   <212> PRT
   <213> Artificial Sequence
<220>
   <223> Description of Artificial Sequence: Synthetic peptide
<400> 222
   Asn Val Glu Ala Trp
   1 5
<210> 223
   <211> 5
   <212> PRT
   <213> Artificial Sequence
<220>
   <223> Description of Artificial Sequence: Synthetic peptide
<400> 223
   Tyr Val Asp Ser Trp
   1 5
<210> 224
   <211> 5
   <212> PRT
   <213> Artificial Sequence
<220>
   <223> Description of Artificial Sequence: Synthetic peptide
<400> 224
   Arg Val Asp Thr Trp
   1               5
<210> 225
   <211> 9
   <212> PRT
   <213> Artificial Sequence
<220>
   <223> Description of Artificial Sequence: Synthetic peptide
<400> 225
   Gly Asp Tyr Ala Leu Gln Gly Pro Gly
   1               5
<210> 226
   <211> 11
   <212> PRT
   <213> Artificial Sequence
<220>
   <223> Description of Artificial Sequence: Synthetic peptide
<400> 226
   Cys Gly Gly Asp Tyr Ala Leu Gln Gly Pro Gly
   1               5                   10
<210> 227
   <211> 11
   <212> PRT
   <213> Artificial Sequence
<220>
   <223> Description of Artificial Sequence: Synthetic peptide
<400> 227
   Trp Gly Gly Asp Tyr Ala Leu Gln Gly Pro Gly
   1               5                   10
<210> 228
   <211> 11
   <212> PRT
   <213> Artificial Sequence
<220>
   <223> Description of Artificial Sequence: Synthetic peptide
<400> 228
   Tyr Gly Gly Asp Tyr Ala Leu Gln Gly Pro Gly
   1 5 10
<210> 229
   <211> 11
   <212> PRT
   <213> Artificial Sequence
<220>
   <223> Description of Artificial Sequence: Synthetic peptide
<400> 229
   Asp Gly Gly Asp Tyr Ala Leu Gln Gly Pro Gly
   1 5 10
<210> 230
   <211> 11
   <212> PRT
   <213> Artificial Sequence
<220>
   <223> Description of Artificial Sequence: Synthetic peptide
<400> 230
   Gly Asp Gly Asp Tyr Ala Leu Gln Gly Pro Gly
   1 5 10
<210> 231
   <211> 11
   <212> PRT
   <213> Artificial Sequence
<220>
   <223> Description of Artificial Sequence: Synthetic peptide
<400> 231
   Asn Gly Gly Asp Tyr Ala Leu Gln Gly Pro Gly
   1 5 10
<210> 232
   <211> 11
   <212> PRT
   <213> Artificial Sequence
<220>
   <223> Description of Artificial Sequence: Synthetic peptide
<400> 232
   Gly Cys Gly Asp Tyr Ala Leu Gln Gly Pro Gly
   1 5 10
<210> 233
   <211> 11
   <212> PRT
   <213> Artificial Sequence
<220>
   <223> Description of Artificial Sequence: Synthetic peptide
<400> 233
   Glu Gly Gly Asp Tyr Ala Leu Gln Gly Pro Gly
   1               5                   10
<210> 234
   <211> 11
   <212> PRT
   <213> Artificial Sequence
<220>
   <223> Description of Artificial Sequence: Synthetic peptide
<400> 234
   Pro Gly Gly Asp Tyr Ala Leu Gln Gly Pro Gly
   1               5                   10
<210> 235
   <211> 11
   <212> PRT
   <213> Artificial Sequence
<220>
   <223> Description of Artificial Sequence: Synthetic peptide
<400> 235
   Thr Gly Gly Asp Tyr Ala Leu Gln Gly Pro Gly
   1               5                   10
<210> 236
   <211> 11
   <212> PRT
   <213> Artificial Sequence
<220>
   <223> Description of Artificial Sequence: Synthetic peptide
<400> 236
   Gln Gly Gly Asp Tyr Ala Leu Gln Gly Pro Gly
   1               5                   10
<210> 237
   <211> 11
   <212> PRT
   <213> Artificial Sequence
<220>
   <223> Description of Artificial Sequence: Synthetic peptide
<400> 237
   Ile Gly Gly Asp Tyr Ala Leu Gln Gly Pro Gly
   1 5 10
<210> 238
   <211> 11
   <212> PRT
   <213> Artificial Sequence
<220>
   <223> Description of Artificial Sequence: Synthetic peptide
<400> 238
   Phe Gly Gly Asp Tyr Ala Leu Gln Gly Pro Gly
   1 5 10
<210> 239
   <211> 11
   <212> PRT
   <213> Artificial Sequence
<220>
   <223> Description of Artificial Sequence: Synthetic peptide
<400> 239
   His Gly Gly Asp Tyr Ala Leu Gln Gly Pro Gly
   1 5 10
<210> 240
   <211> 11
   <212> PRT
   <213> Artificial Sequence
<220>
   <223> Description of Artificial Sequence: Synthetic peptide
<400> 240
   Leu Gly Gly Asp Tyr Ala Leu Gln Gly Pro Gly
   1 5 10
<210> 241
   <211> 11
   <212> PRT
   <213> Artificial Sequence
<220>
   <223> Description of Artificial Sequence: Synthetic peptide
<400> 241
   Val Gly Gly Asp Tyr Ala Leu Gln Gly Pro Gly
   1 5 10
<210> 242
   <211> 11
   <212> PRT
   <213> Artificial Sequence
<220>
   <223> Description of Artificial Sequence: Synthetic peptide
<400> 242
   Arg Gly Gly Asp Tyr Ala Leu Gln Gly Pro Gly
   1 5 10
<210> 243
   <211> 11
   <212> PRT
   <213> Artificial Sequence
<220>
   <223> Description of Artificial Sequence: Synthetic peptide
<400> 243
   Gly Trp Gly Asp Tyr Ala Leu Gln Gly Pro Gly
   1 5 10
<210> 244
   <211> 11
   <212> PRT
   <213> Artificial Sequence
<220>
   <223> Description of Artificial Sequence: Synthetic peptide
<400> 244
   Met Gly Gly Asp Tyr Ala Leu Gln Gly Pro Gly
   1 5 10
<210> 245
   <211> 11
   <212> PRT
   <213> Artificial Sequence
<220>
   <223> Description of Artificial Sequence: Synthetic peptide
<400> 245
   Ser Gly Gly Asp Tyr Ala Leu Gln Gly Pro Gly
   1 5 10
<210> 246
   <211> 11
   <212> PRT
   <213> Artificial Sequence
<220>
   <223> Description of Artificial Sequence: Synthetic peptide
<400> 246
   Ala Gly Gly Asp Tyr Ala Leu Gln Gly Pro Gly
   1 5 10
<210> 247
   <211> 11
   <212> PRT
   <213> Artificial Sequence
<220>
   <223> Description of Artificial Sequence: Synthetic peptide
<400> 247
   Gly Tyr Gly Asp Tyr Ala Leu Gln Gly Pro Gly
   1 5 10
<210> 248
   <211> 11
   <212> PRT
   <213> Artificial Sequence
<220>
   <223> Description of Artificial Sequence: Synthetic peptide
<400> 248
   Gly Glu Gly Asp Tyr Ala Leu Gln Gly Pro Gly
   1 5 10
<210> 249
   <211> 11
   <212> PRT
   <213> Artificial Sequence
<220>
   <223> Description of Artificial Sequence: Synthetic peptide
<400> 249
   Gly Pro Gly Asp Tyr Ala Leu Gln Gly Pro Gly
   1 5 10
<210> 250
   <211> 11
   <212> PRT
   <213> Artificial Sequence
<220>
   <223> Description of Artificial Sequence: Synthetic peptide
<400> 250
   Gly His Gly Asp Tyr Ala Leu Gln Gly Pro Gly
   1 5 10
<210> 251
   <211> 11
   <212> PRT
   <213> Artificial Sequence
<220>
   <223> Description of Artificial Sequence: Synthetic peptide
<400> 251
   Gly Asn Gly Asp Tyr Ala Leu Gln Gly Pro Gly
   1 5 10
<210> 252
   <211> 5
   <212> PRT
   <213> Artificial Sequence
<220>
   <223> Description of Artificial Sequence: Synthetic peptide
<400> 252
   Asp Tyr Ala Leu Gln
   1 5
<210> 253
   <211> 11
   <212> PRT
   <213> Artificial Sequence
<220>
   <223> Description of Artificial Sequence: Synthetic peptide
<400> 253
   Gly Gly Gly Asp Tyr Ala Leu Gln Gly Gly Gly
   1 5 10
<210> 254
   <211> 11
   <212> PRT
   <213> Artificial Sequence
<220>
   <223> Description of Artificial Sequence: Synthetic peptide
<400> 254
   Trp Asp Gly Asp Tyr Ala Leu Gln Gly Gly Gly
   1 5 10
<210> 255
   <211> 13
   <212> PRT
   <213> Artificial Sequence
<220>
   <223> Description of Artificial Sequence: Synthetic peptide
<400> 255
   Gly Gly Gly Gly Asp Tyr Ala Leu Gln Gly Gly Gly Gly
   1 5 10
<210> 256
   <211> 12
   <212> PRT
   <213> Artificial Sequence
<220>
   <223> Description of Artificial Sequence: Synthetic peptide
<400> 256
   Gly Gly Gly Asp Tyr Ala Leu Gln Gly Gly Gly Gly
   1 5 10
<210> 257
   <211> 5
   <212> PRT
   <213> Artificial Sequence
<220>
   <223> Description of Artificial Sequence: Synthetic peptide
<220>
   <221> MOD_RES
   <222> (1)..(1)
   <223> Tyr or Phe
<220>
   <221> MOD_RES
   <222> (2) .. (2)
   <223> Val or Ala
<400> 257
   Xaa Xaa Leu Gln Gly
   1 5
<210> 258
   <211> 5
   <212> PRT
   <213> Artificial Sequence
<220>
   <223> Description of Artificial Sequence: Synthetic peptide
<400> 258
   Asp Tyr Val Leu Gln
   1 5
<210> 259
   <211> 5
   <212> PRT
   <213> Artificial Sequence
<220>
   <223> Description of Artificial Sequence: Synthetic peptide
<400> 259
   Asn Tyr Ala Leu Gln
   1 5
<210> 260
   <211> 5
   <212> PRT
   <213> Artificial Sequence
<220>
   <223> Description of Artificial Sequence: Synthetic peptide
<400> 260
   Glu Tyr Ala Leu Gln
   1 5
<210> 261
   <211> 5
   <212> PRT
   <213> Artificial Sequence
<220>
   <223> Description of Artificial Sequence: Synthetic peptide
<400> 261
   Pro Tyr Ala Leu Gln
   1 5
<210> 262
   <211> 5
   <212> PRT
   <213> Artificial Sequence
<220>
   <223> Description of Artificial Sequence: Synthetic peptide
<400> 262
   Glu Tyr Val Leu Gln
   1 5
<210> 263
   <211> 5
   <212> PRT
   <213> Artificial Sequence
<220>
   <223> Description of Artificial Sequence: Synthetic peptide
<400> 263
   Asp Phe Ala Leu Gln
   1 5
<210> 264
   <211> 5
   <212> PRT
   <213> Artificial Sequence
<220>
   <223> Description of Artificial Sequence: Synthetic peptide
<400> 264
   Phe Tyr Ala Leu Gln
   1 5
<210> 265
   <211> 5
   <212> PRT
   <213> Artificial Sequence
<220>
   <223> Description of Artificial Sequence: Synthetic peptide
<400> 265
   Asn Tyr Val Leu Gln
   1 5
<210> 266
   <211> 5
   <212> PRT
   <213> Artificial Sequence
<220>
   <223> Description of Artificial Sequence: Synthetic peptide
<400> 266
   Arg Tyr Ala Leu Gln
   1 5
<210> 267
   <211> 5
   <212> PRT
   <213> Artificial Sequence
<220>
   <223> Description of Artificial Sequence: Synthetic peptide
<400> 267
   Tyr Phe Ala Leu Gln
   1 5
<210> 268
   <211> 5
   <212> PRT
   <213> Artificial Sequence
<220>
   <223> Description of Artificial Sequence: Synthetic peptide
<400> 268
   Pro Tyr Val Leu Gln
   1 5
<210> 269
   <211> 5
   <212> PRT
   <213> Artificial Sequence
<220>
   <223> Description of Artificial Sequence: Synthetic peptide
<400> 269
   Trp Tyr Ala Leu Gln
   1 5
<210> 270
   <211> 5
   <212> PRT
   <213> Artificial Sequence
<220>
   <223> Description of Artificial Sequence: Synthetic peptide
<400> 270
   Ser Tyr Ala Leu Gln
   1 5
<210> 271
   <211> 5
   <212> PRT
   <213> Artificial Sequence
<220>
   <223> Description of Artificial Sequence: Synthetic peptide
<400> 271
   His Tyr Ala Leu Gln
   1 5
<210> 272
   <211> 5
   <212> PRT
   <213> Artificial Sequence
<220>
   <223> Description of Artificial Sequence: Synthetic peptide
<400> 272
   Glu Phe Ala Leu Gln
   1 5
<210> 273
   <211> 5
   <212> PRT
   <213> Artificial Sequence
<220>
   <223> Description of Artificial Sequence: Synthetic peptide
<400> 273
   Asn Phe Val Leu Gln
   1 5
<210> 274
   <211> 5
   <212> PRT
   <213> Artificial Sequence
<220>
   <223> Description of Artificial Sequence: Synthetic peptide
<400> 274
   Asp Tyr Phe Leu Gln
   1 5
<210> 275
   <211> 5
   <212> PRT
   <213> Artificial Sequence
<220>
   <223> Description of Artificial Sequence: Synthetic peptide
<400> 275
   Glu Tyr Val Ala Gln
   1 5
<210> 276
   <211> 5
   <212> PRT
   <213> Artificial Sequence
<220>
   <223> Description of Artificial Sequence: Synthetic peptide
<400> 276
   Asp Tyr Val Ala Gln
   1 5
<210> 277
   <211> 5
   <212> PRT
   <213> Artificial Sequence
<220>
   <223> Description of Artificial Sequence: Synthetic peptide
<400> 277
   Asp Phe Tyr Leu Gln
   1 5
<210> 278
   <211> 5
   <212> PRT
   <213> Artificial Sequence
<220>
   <223> Description of Artificial Sequence: Synthetic peptide
<400> 278
   Glu Tyr Phe Leu Gln
   1 5
<210> 279
   <211> 4
   <212> PRT
   <213> Artificial Sequence
<220>
   <223> Description of Artificial Sequence: Synthetic peptide
<220>
   <221> MOD_RES
   <222> (3)..(3)
   <223> Leu or Ser
<400> 279
   Ser Lys Xaa Lys
   1
<210> 280
   <211> 4
   <212> PRT
   <213> Artificial Sequence
<220>
   <223> Description of Artificial Sequence: Synthetic peptide
<220>
   <221> MOD_RES
   <222> (1)..(1)
   <223> Lys or Arg
<220>
   <221> MOD_RES
   <222> (2) .. (2)
   <223> Ser or Thr
<400> 280
   Xaa Xaa Lys Leu
   1
<210> 281
   <211> 5
   <212> PRT
   <213> Artificial Sequence
<220>
   <223> Description of Artificial Sequence: Synthetic peptide
<400> 281
   Ala Arg Ser Lys Leu
   1 5
<210> 282
   <211> 5
   <212> PRT
   <213> Artificial Sequence
<220>
   <223> Description of Artificial Sequence: Synthetic peptide
<400> 282
   Lys Ser Lys Leu Ala
   1 5
<210> 283
   <211> 5
   <212> PRT
   <213> Artificial Sequence
<220>
   <223> Description of Artificial Sequence: Synthetic peptide
<400> 283
   Thr Lys Ser Lys Leu
   1 5
<210> 284
   <211> 5
   <212> PRT
   <213> Artificial Sequence
<220>
   <223> Description of Artificial Sequence: Synthetic peptide
<400> 284
   Lys Leu Ser Lys Leu
   1               5
<210> 285
   <211> 5
   <212> PRT
   <213> Artificial Sequence
<220>
   <223> Description of Artificial Sequence: Synthetic peptide
<400> 285
   Arg Ser Lys Leu Gly
   1               5
<210> 286
   <211> 5
   <212> PRT
   <213> Artificial Sequence
<220>
   <223> Description of Artificial Sequence: Synthetic peptide
<400> 286
   Arg Gly Ser Lys Leu
   1               5
<210> 287
   <211> 5
   <212> PRT
   <213> Artificial Sequence
<220>
   <223> Description of Artificial Sequence: Synthetic peptide
<400> 287
   Arg Ser Lys Ser Lys
   1               5
<210> 288
   <211> 5
   <212> PRT
   <213> Artificial Sequence
<220>
   <223> Description of Artificial Sequence: Synthetic peptide
<400> 288
   Ser Lys Ser Lys Leu
   1 5
<210> 289
   <211> 5
   <212> PRT
   <213> Artificial Sequence
<220>
   <223> Description of Artificial Sequence: Synthetic peptide
<400> 289
   Pro Lys Thr Lys Leu
   1 5
<210> 290
   <211> 5
   <212> PRT
   <213> Artificial Sequence
<220>
   <223> Description of Artificial Sequence: Synthetic peptide
<400> 290
   Arg Ser Lys Leu Ala
   1 5
<210> 291
   <211> 5
   <212> PRT
   <213> Artificial Sequence
<220>
   <223> Description of Artificial Sequence: Synthetic peptide
<400> 291
   Gly Arg Ser Lys Leu
   1 5
<210> 292
   <211> 5
   <212> PRT
   <213> Artificial Sequence
<220>
   <223> Description of Artificial Sequence: Synthetic peptide
<400> 292
   Ser Lys Leu Ser Lys
   1 5
<210> 293
   <211> 5
   <212> PRT
   <213> Artificial Sequence
<220>
   <223> Description of Artificial Sequence: Synthetic peptide
<400> 293
   Phe Thr Lys Ser Lys
   1 5
<210> 294
   <211> 5
   <212> PRT
   <213> Artificial Sequence
<220>
   <223> Description of Artificial Sequence: Synthetic peptide
<400> 294
   Arg Leu Lys Ser Lys
   1 5
<210> 295
   <211> 5
   <212> PRT
   <213> Artificial Sequence
<220>
   <223> Description of Artificial Sequence: Synthetic peptide
<400> 295
   Lys Leu Gly Ala Lys
   1 5
<210> 296
   <211> 5
   <212> PRT
   <213> Artificial Sequence
<220>
   <223> Description of Artificial Sequence: Synthetic peptide
<400> 296
   Gln Arg Ser Lys Leu
   1 5
<210> 297
   <211> 5
   <212> PRT
   <213> Artificial Sequence
<220>
   <223> Description of Artificial Sequence: Synthetic peptide
<400> 297
   Leu Ser Lys Leu Lys
   1 5
<210> 298
   <211> 5
   <212> PRT
   <213> Artificial Sequence
<220>
   <223> Description of Artificial Sequence: Synthetic peptide
<400> 298
   Asn Arg Thr Lys Leu
   1 5
<210> 299
   <211> 5
   <212> PRT
   <213> Artificial Sequence
<220>
   <223> Description of Artificial Sequence: Synthetic peptide
<400> 299
   Gln Arg Thr Lys Leu
   1 5
<210> 300
   <211> 11
   <212> PRT
   <213> Artificial Sequence
<220>
   <223> Description of Artificial Sequence: Synthetic peptide
<400> 300
   Gly Gly Gly Arg Ser Lys Leu Ala Gly Gly Gly
   1 5 10
<210> 301
   <211> 12
   <212> PRT
   <213> Artificial Sequence
<220>
   <223> Description of Artificial Sequence: Synthetic peptide
<400> 301
   Gly Gly Gly Ala Arg Ser Lys Leu Gly Gly Gly Gly
   1 5 10
<210> 302
   <211> 5
   <212> PRT
   <213> Artificial Sequence
<220>
   <223> Description of Artificial Sequence: Synthetic peptide
<400> 302
   Arg Gly Thr Lys Leu
   1 5
<210> 303
   <211> 5
   <212> PRT
   <213> Artificial Sequence
<220>
   <223> Description of Artificial Sequence: Synthetic peptide
<400> 303
   Phe Pro Lys Leu Lys
   1 5
<210> 304
   <211> 5
   <212> PRT
   <213> Artificial Sequence
<220>
   <223> Description of Artificial Sequence: Synthetic peptide
<400> 304
   Lys Leu Lys Tyr Lys
   1 5
<210> 305
   <211> 5
   <212> PRT
   <213> Artificial Sequence
<220>
   <223> Description of Artificial Sequence: Synthetic peptide
<400> 305
   Arg Ala Lys Tyr Lys
   1 5
<210> 306
   <211> 5
   <212> PRT
   <213> Artificial Sequence
<220>
   <223> Description of Artificial Sequence: Synthetic peptide
<400> 306
   Lys Thr Lys Tyr Lys
   1 5
<210> 307
   <211> 5
   <212> PRT
   <213> Artificial Sequence
<220>
   <223> Description of Artificial Sequence: Synthetic peptide
<400> 307
   Gly Tyr Lys Leu Lys
   1 5
<210> 308
   <211> 4
   <212> PRT
   <213> Artificial Sequence
<220>
   <223> Description of Artificial Sequence: Synthetic peptide
<220>
   <221> MOD_RES
   <222> (1)..(1)
   <223> Phe or Tyr
<220>
   <221> MOD_RES
   <222> (2)..(2)
   <223> Phe, Tyr or Thr
<400> 308
   Xaa Xaa Leu Gln
   1
<210> 309
   <211> 4
   <212> PRT
   <213> Artificial Sequence
<220>
   <223> Description of Artificial Sequence: Synthetic peptide
<220>
   <221> MOD_RES
   <222> (1)..(1)
   <223> Tyr or Phe
<400> 309
   Xaa Val Ala Gln
   1
<210> 310
   <211> 3
   <212> PRT
   <213> Artificial Sequence
<220>
   <223> Description of Artificial Sequence: Synthetic peptide
<220>
   <221> MOD_RES
   <222> (2) .. (2)
   <223> Tyr, Leu or Ser
<400> 310
   Lys Xaa Lys
   1
<210> 311
   <211> 3
   <212> PRT
   <213> Artificial Sequence
<220>
   <223> Description of Artificial Sequence: Synthetic peptide
<400> 311
   Thr Lys Leu
   1
<210> 312
   <211> 5
   <212> PRT
   <213> Artificial Sequence
<220>
   <223> Description of Artificial Sequence: Synthetic peptide
<220>
   <221> MOD_RES
   <222> (2) .. (2)
   <223> Thr or Ser
<400> 312
   Thr Xaa Val His Gln
   1 5
<210> 313
   <211> 52
   <212> PRT
   <213> Artificial Sequence
<220>
   <223> Description of Artificial Sequence: Synthetic polypeptide
<220>
   <221> MOD_RES
   <222> (16)..(25)
   <223> Any of the 20 standard amino acids
<400> 313
   Met Gly Cys Thr Ser Val His Gln Glu Thr Lys Lys Tyr Gln Ser Xaa
   1               5                   10                  15
   Xaa Xaa Xaa Xaa Xaa Xaa Xaa Xaa Xaa Ser Tyr Thr Tyr Asn Tyr Glu
               20                  25                  30
   His Val Asp Val Trp Gly Cys Gly Ser Ala Asp Tyr Lys Asp Asp Asp
           35                  40                  45
   Asp Lys Lys Lys
       50
<210> 314
   <211> 3
   <212> PRT
   <213> Artificial Sequence
<220>
   <223> Description of Artificial Sequence: Synthetic peptide
<400> 314
   Asn Asn Ser
   1
<210> 315
   <211> 3
   <212> PRT
   <213> Artificial Sequence
<220>
   <223> Description of Artificial Sequence: Synthetic peptide
<400> 315
   Asn Asn Cys
   1
<210> 316
   <211> 3
   <212> PRT
   <213> Artificial Sequence
<220>
   <223> Description of Artificial Sequence: Synthetic peptide
<400> 316
   Asn Asn Gly
   1
<210> 317
   <211> 36
   <212> DNA
   <213> Artificial Sequence
<220>
   <223> Description of Artificial Sequence: Synthetic oligonucleotide
<400> 317
   tgcattatca aaaagagccg cgatccgggc cgctgc 36
<210> 318
   <211> 12
   <212> PRT
   <213> Artificial Sequence
<220>
   <223> Description of Artificial Sequence: Synthetic peptide
<400> 318
   Cys Ile Ile Lys Lys Ser Arg Asp Pro Gly Arg Cys
   1 5 10
<210> 319
   <211> 35
   <212> PRT
   <213> Artificial Sequence
<220>
   <223> Description of Artificial Sequence: Synthetic polypeptide
<220>
   <221> MOD_RES
   <222> (14)..(23)
   <223> Any of the 20 standard amino acids
<400> 319
   Cys Thr Ser Val His Gln Glu Thr Lys Lys Tyr Gln Ser Xaa Xaa Xaa
   1 5 10 15
   Xaa Xaa Xaa Xaa Xaa Xaa Xaa Ser Tyr Thr Tyr Asn Tyr Glu His Val
   20 25 30
   Asp Val Trp
   35
<210> 320
   <211> 13
   <212> PRT
   <213> Artificial Sequence
<220>
   <223> Description of Artificial Sequence: Synthetic peptide
<400> 320
   Cys Thr Ser Val His Gln Glu Thr Lys Lys Tyr Gln Ser
   1               5                   10
<210> 321
   <211> 52
   <212> PRT
   <213> Artificial Sequence
<220>
   <223> Description of Artificial Sequence: Synthetic polypeptide
<220>
   <221> MOD_RES
   <222> (16)..(25)
   <223> Any of the 20 standard amino acids
<400> 321
   Met Gly Cys Thr Ser Val His Gln Glu Thr Lys Lys Tyr Gln Ser Xaa
   1               5                   10                  15
   Xaa Xaa Xaa Xaa Xaa Xaa Xaa Xaa Xaa Ser Tyr Thr Tyr Asn Tyr Glu
               20                  25                  30
   His Val Asp Val Trp Gly Cys Gly Ser Ala Asp Tyr Lys Asp Asp Asp
           35                  40                  45
   Asp Lys Lys Lys
       50
<210> 322
   <211> 8
   <212> PRT
   <213> Artificial Sequence
<220>
   <223> Description of Artificial Sequence: Synthetic peptide
<400> 322
   Asp Tyr Lys Asp Asp Asp Asp Lys
   1               5
<210> 323
   <211> 185
   <212> RNA
   <213> Artificial Sequence
<220>
   <223> Description of Artificial Sequence: Synthetic polynucleotide
<220>
   <221> modified_base
   <222> (75)..(76)
   <223> a, c, u, g, unknown or other
<220>
   <221> modified_base
   <222> (78)..(79)
   <223> a, c, u, g, unknown or other
<220>
   <221> modified_base
   <222> (81)..(82)
   <223> a, c, u, g, unknown or other
<220>
   <221> modified_base
   <222> (84)..(85)
   <223> a, c, u, g, unknown or other
<220>
   <221> modified_base
   <222> (87)..(88)
   <223> a, c, u, g, unknown or other
<220>
   <221> modified_base
   <222> (90)..(91)
   <223> a, c, u, g, unknown or other
<220>
   <221> modified_base
   <222> (93)..(94)
   <223> a, c, u, g, unknown or other
<220>
   <221> modified_base
   <222> (96)..(97)
   <223> a, c, u, g, unknown or other
<220>
   <221> modified_base
   <222> (99)..(100)
   <223> a, c, u, g, unknown or other
<220>
   <221> modified_base
   <222> (102)..(103)
   <223> a, c, u, g, unknown or other
<400> 323
   ggguuaacuu uaguaaggag gacagcuaaa uggguugcac cagcgugcau caggagacca 60
   agaaauacca gagcnnsnns nnsnnsnnsn nsnnsnnsnn snnsagcuac accuacaacu 120
   acgaacaugu ggauguaugg gguugcggcu ccgcugacua caaagaugac gacgauaaga 180
   aaaaa 185
<210> 324
   <211> 3
   <212> RNA
   <213> Artificial Sequence
<220>
   <223> Description of Artificial Sequence: Synthetic oligonucleotide
<400> 324
   aug 3
<210> 325
   <211> 182
   <212> DNA
   <213> Artificial Sequence
<220>
   <223> Description of Artificial Sequence: Synthetic polynucleotide
<220>
   <221> modified_base
   <222> (92)..(93)
   <223> a, c, t, g, unknown or other
<220>
   <221> modified_base
   <222> (95)..(96)
   <223> a, c, t, g, unknown or other
<220>
   <221> modified_base
   <222> (98)..(99)
   <223> a, c, t, g, unknown or other
<220>
   <221> modified_base
   <222> (101)..(102)
   <223> a, c, t, g, unknown or other
<220>
   <221> modified_base
   <222> (104)..(105)
   <223> a, c, t, g, unknown or other
<220>
   <221> modified_base
   <222> (107)..(108)
   <223> a, c, t, g, unknown or other
<220>
   <221> modified_base
   <222> (110)..(111)
   <223> a, c, t, g, unknown or other
<220>
   <221> modified_base
   <222> (113)..(114)
   <223> a, c, t, g, unknown or other
<220>
   <221> modified_base
   <222> (116)..(117)
   <223> a, c, t, g, unknown or other
<220>
   <221> modified_base
   <222> (119)..(120)
   <223> a, c, t, g, unknown or other
<400> 325
   taatacgact cactataggg ttaactttag taaggaggac agctaaatgg gttgcaccag 60
   cgtgcatcag gagaccaaga aataccagag cnnsnnsnns nnsnnsnnsn nsnnsnnsnn 120
   sagctacacc tacaactacg aacatgtgga tgtatggggt tgcggctccg ctgactacaa 180
   ag 182
<210> 326
   <211> 20
   <212> DNA
   <213> Artificial Sequence
<220>
   <223> Description of Artificial Sequence: Synthetic oligonucleotide
<400> 326
   atgacgacga taagaaaaaa 20
<210> 327
   <211> 20
   <212> DNA
   <213> Artificial Sequence
<220>
   <223> Description of Artificial Sequence: Synthetic oligonucleotide
<400> 327
   taatacgact cactataggg 20
<210> 328
   <211> 162
   <212> DNA
   <213> Artificial Sequence
<220>
   <223> Description of Artificial Sequence: Synthetic polynucleotide
<220>
   <221> modified_base
   <222> (62)..(63)
   <223> a, c, t, g, unknown or other
<220>
   <221> modified_base
   <222> (65)..(66)
   <223> a, c, t, g, unknown or other
<220>
   <221> modified_base
   <222> (68)..(69)
   <223> a, c, t, g, unknown or other
<220>
   <221> modified_base
   <222> (71)..(72)
   <223> a, c, t, g, unknown or other
<220>
   <221> modified_base
   <222> (74)..(75)
   <223> a, c, t, g, unknown or other
<220>
   <221> modified_base
   <222> (77)..(78)
   <223> a, c, t, g, unknown or other
<220>
   <221> modified_base
   <222> (80)..(81)
   <223> a, c, t, g, unknown or other
<220>
   <221> modified_base
   <222> (83)..(84)
   <223> a, c, t, g, unknown or other
<220>
   <221> modified_base
   <222> (86)..(87)
   <223> a, c, t, g, unknown or other
<220>
   <221> modified_base
   <222> (89)..(90)
   <223> a, c, t, g, unknown or other
<400> 328
   taaggaggac agctaaatgg gttgcaccag cgtgcatcag gagaccaaga aataccagag 60
   cnnsnnsnns nnsnnsnnsn nsnnsnnsnn sagctacacc tacaactacg aacatgtgga 120
   tgtatggggt tgcggctccg ctgactacaa agatgacgac ga 162
<210> 329
   <211> 49
   <212> DNA
   <213> Artificial Sequence
<220>
   <223> Description of Artificial Sequence: Synthetic oligonucleotide
<400> 329
   taatacgact cactataggg ttaactttag taaggaggac agctaaatg 49
<210> 330
   <211> 30
   <212> DNA
   <213> Artificial Sequence
<220>
   <223> Description of Artificial Sequence: Synthetic oligonucleotide
<400> 330
   ttttttctta tcgtcgtcat ctttgtagtc 30
<210> 331
   <211> 5
   <212> PRT
   <213> Artificial Sequence
<220>
   <223> Description of Artificial Sequence: Synthetic peptide
<400> 331
   Arg Asp Pro Gly Arg
   1 5
<210> 332
   <211> 10
   <212> PRT
   <213> Artificial Sequence
<220>
   <223> Description of Artificial Sequence: Synthetic peptide
<400> 332
   Arg Asp Pro Gly Arg Leu Ile Phe Gln Ser
   1 5 10
<210> 333
   <211> 10
   <212> PRT
   <213> Artificial Sequence
<220>
   <223> Description of Artificial Sequence: Synthetic peptide
<400> 333
   Ser Gln Ser Glu Arg Ala Thr Pro Arg Tyr
   1               5                   10
<210> 334
   <211> 10
   <212> PRT
   <213> Artificial Sequence
<220>
   <223> Description of Artificial Sequence: Synthetic peptide
<400> 334
   Arg Phe His Leu Phe Ile Leu Gly Thr Ser
   1               5                   10
<210> 335
   <211> 10
   <212> PRT
   <213> Artificial Sequence
<220>
   <223> Description of Artificial Sequence: Synthetic peptide
<400> 335
   Pro Pro Val Leu Pro Gln Leu Val Leu Leu
   1               5                   10
<210> 336
   <211> 10
   <212> PRT
   <213> Artificial Sequence
<220>
   <223> Description of Artificial Sequence: Synthetic peptide
<400> 336
   Pro Leu Ile His Pro Arg Arg Gly Tyr Ala
   1               5                   10
<210> 337
   <211> 10
   <212> PRT
   <213> Artificial Sequence
<220>
   <223> Description of Artificial Sequence: Synthetic peptide
<400> 337
   His Arg Leu Gln Ile Ala Thr Val Tyr Thr
   1 5 10
<210> 338
   <211> 10
   <212> PRT
   <213> Artificial Sequence
<220>
   <223> Description of Artificial Sequence: Synthetic peptide
<400> 338
   Thr Gln Pro Lys Leu Thr Ile Glu Gln Arg
   1 5 10
<210> 339
   <211> 10
   <212> PRT
   <213> Artificial Sequence
<220>
   <223> Description of Artificial Sequence: Synthetic peptide
<400> 339
   His Cys Asp Thr Leu Glu Leu Arg Pro Ala
   1 5 10
<210> 340
   <211> 10
   <212> PRT
   <213> Artificial Sequence
<220>
   <223> Description of Artificial Sequence: Synthetic peptide
<400> 340
   Leu Lys Gln Thr Asp Phe Arg Thr Pro Ile
   1 5 10
<210> 341
   <211> 10
   <212> PRT
   <213> Artificial Sequence
<220>
   <223> Description of Artificial Sequence: Synthetic peptide
<400> 341
   Lys Cys Cys Thr Ser Met Val Ile Gln Leu
   1 5 10
<210> 342
   <211> 10
   <212> PRT
   <213> Artificial Sequence
<220>
   <223> Description of Artificial Sequence: Synthetic peptide
<400> 342
   Leu Gln Arg Ala Ile Ser Ser Tyr Ser Arg
   1 5 10
<210> 343
   <211> 10
   <212> PRT
   <213> Artificial Sequence
<220>
   <223> Description of Artificial Sequence: Synthetic peptide
<400> 343
   Arg Asp Pro Gly Arg Leu Ile Phe Gln Ser
   1 5 10
<210> 344
   <211> 10
   <212> PRT
   <213> Artificial Sequence
<220>
   <223> Description of Artificial Sequence: Synthetic peptide
<400> 344
   Arg Asp Pro Gly Arg Val Ile Phe Glu Ile
   1 5 10
<210> 345
   <211> 10
   <212> PRT
   <213> Artificial Sequence
<220>
   <223> Description of Artificial Sequence: Synthetic peptide
<400> 345
   Arg Asp Pro Gly Arg Ile Val Phe Asn Asn
   1 5 10
<210> 346
   <211> 10
   <212> PRT
   <213> Artificial Sequence
<220>
   <223> Description of Artificial Sequence: Synthetic peptide
<400> 346
   Arg Asp Pro Tyr Asn Val Leu Ile Ser Leu
   1 5 10
<210> 347
   <211> 10
   <212> PRT
   <213> Artificial Sequence
<220>
   <223> Description of Artificial Sequence: Synthetic peptide
<400> 347
   His Arg Leu Gln Ile Ala Thr Val Tyr Thr
   1 5 10
<210> 348
   <211> 10
   <212> PRT
   <213> Artificial Sequence
<220>
   <223> Description of Artificial Sequence: Synthetic peptide
<400> 348
   Glu Thr Arg Ile Tyr Ile Arg Phe His Thr
   1 5 10
<210> 349
   <211> 5
   <212> PRT
   <213> Artificial Sequence
<220>
   <223> Description of Artificial Sequence: Synthetic peptide
<400> 349
   Arg Thr Lys Ile Lys
   1 5
<210> 350
   <211> 10
   <212> PRT
   <213> Artificial Sequence
<220>
   <223> Description of Artificial Sequence: Synthetic peptide
<400> 350
   Val Thr Ser Thr Tyr Met Phe Met Tyr Ala
   1 5 10
<210> 351
   <211> 9
   <212> PRT
   <213> Artificial Sequence
<220>
   <223> Description of Artificial Sequence: Synthetic peptide
<400> 351
   Ala Ala Val Arg Leu Asn Ser Ser Ser
   1               5
<210> 352
   <211> 10
   <212> PRT
   <213> Artificial Sequence
<220>
   <223> Description of Artificial Sequence: Synthetic peptide
<400> 352
   Val Val Glu Phe His Phe Lys Trp Cys Ile
   1               5                   10
<210> 353
   <211> 10
   <212> PRT
   <213> Artificial Sequence
<220>
   <223> Description of Artificial Sequence: Synthetic peptide
<400> 353
   Arg Asp Pro Gly Arg Leu Ile Phe Gln Ser
   1               5                   10
<210> 354
   <211> 10
   <212> PRT
   <213> Artificial Sequence
<220>
   <223> Description of Artificial Sequence: Synthetic peptide
<400> 354
   Arg Asp Pro Gly Arg Val Ile Phe Glu Ile
   1               5                   10
<210> 355
   <211> 10
   <212> PRT
   <213> Artificial Sequence
<220>
   <223> Description of Artificial Sequence: Synthetic peptide
<400> 355
   Arg Asp Pro Gly Arg Ile Val Phe Asn Asn
   1 5 10
<210> 356
   <211> 10
   <212> PRT
   <213> Artificial Sequence
<220>
   <223> Description of Artificial Sequence: Synthetic peptide
<400> 356
   Arg Asp Pro Tyr Asn Val Leu Ile Ser Leu
   1 5 10
<210> 357
   <211> 10
   <212> PRT
   <213> Artificial Sequence
<220>
   <223> Description of Artificial Sequence: Synthetic peptide
<400> 357
   Arg Ser His Gly Gln Phe Ser Phe Thr Phe
   1 5 10
<210> 358
   <211> 10
   <212> PRT
   <213> Artificial Sequence
<220>
   <223> Description of Artificial Sequence: Synthetic peptide
<400> 358
   Ser Leu Phe Val Glu Tyr Arg Ile Thr Tyr
   1 5 10
<210> 359
   <211> 10
   <212> PRT
   <213> Artificial Sequence
<220>
   <223> Description of Artificial Sequence: Synthetic peptide
<400> 359
   Gln Met Ser Phe Arg Phe Glu Val Arg Val
   1 5 10
<210> 360
   <211> 10
   <212> PRT
   <213> Artificial Sequence
<220>
   <223> Description of Artificial Sequence: Synthetic peptide
<400> 360
   Ser Ile Glu Phe Gly Leu Thr Phe Ser Phe
   1               5                   10
<210> 361
   <211> 10
   <212> PRT
   <213> Artificial Sequence
<220>
   <223> Description of Artificial Sequence: Synthetic peptide
<400> 361
   Val Thr Ser Thr Tyr Met Phe Met Tyr Ala
   1               5                   10
<210> 362
   <211> 10
   <212> PRT
   <213> Artificial Sequence
<220>
   <223> Description of Artificial Sequence: Synthetic peptide
<400> 362
   Gln Phe His Leu Glu Phe Ala Phe Thr Leu
   1               5                   10
<210> 363
   <211> 52
   <212> PRT
   <213> Artificial Sequence
<220>
   <223> Description of Artificial Sequence: Synthetic polypeptide
<400> 363
   Met Gly Cys Thr Ser Val His Gln Glu Thr Lys Lys Tyr Gln Ser Arg
   1               5                   10                  15
   Asp Pro Gly Arg Leu Ile Phe Gln Ser Ser Tyr Thr Tyr Asn Tyr Glu
               20                  25                  30
   His Val Asp Val Trp Gly Cys Gly Ser Ala Trp Ser His Pro Gln Phe
           35                  40                  45
   Glu Lys Gly Ser
       50
<210> 364
   <211> 156
   <212> DNA
   <213> Artificial Sequence
<220>
   <223> Description of Artificial Sequence: Synthetic polynucleotide
<400> 364
   atgggttgca ccagcgtgca tcaggagacc aagaaatacc agagcagaga tcctggtaga        60
   ttaatatttc aatctagcta cacctacaac tacgaacatg tggatgtatg gggttgcggc        120
   tccgcttgga gccatccgca gttcgaaaag ggtagt                                  156
<210> 365
   <211> 60
   <212> DNA
   <213> Artificial Sequence
<220>
   <223> Description of Artificial Sequence: Synthetic primer
<400> 365
   taatacgact cactataggg ttaactttag taaggaggac agctaaatgg gttgcaccag 60
<210> 366
   <211> 33
   <212> DNA
   <213> Artificial Sequence
<220>
   <223> Description of Artificial Sequence: Synthetic primer
<400> 366
   ttaactaccc ttttcgaact gcggatggct cca 33
<210> 367
   <211> 39
   <212> PRT
   <213> Artificial Sequence
<220>
   <223> Description of Artificial Sequence: Synthetic polypeptide
<220>
   <223> N-term Biotin
<400> 367
   Gly Cys Thr Ser Val His Gln Glu Thr Lys Lys Tyr Gln Ser Arg Asp
   1               5                   10                  15
   Pro Gly Arg Leu Ile Phe Gln Ser Ser Tyr Thr Tyr Asn Tyr Glu His
               20                  25                  30
   Val Asp Val Trp Gly Cys Gly
           35
<210> 368
   <211> 20
   <212> PRT
   <213> Artificial Sequence
<220>
   <223> Description of Artificial Sequence: Synthetic peptide
<220>
   <223> N-term Biotin
<400> 368
   Gly Gly Gly Gly Ser Gly Gly Gly Gly Ser Arg Asp Pro Gly Arg Leu
   1               5                   10                  15
   Ile Phe Gln Ser
               20
<210> 369
   <211> 66
   <212> PRT
   <213> Artificial Sequence
<220>
   <223> Description of Artificial Sequence: Synthetic polypeptide
<400> 369
   Met Gly Cys Thr Ser Val His Gln Glu Thr Lys Lys Tyr Gln Ser Cys
   1 5 10 15
   Ile Ile Lys Lys Ser Arg Asp Pro Gly Arg Cys Ser Tyr Thr Tyr Asn
   20 25 30
   Tyr Glu His Val Asp Val Trp Gly Cys Gly Ser Ala Trp Ser His Pro
   35 40 45
   Gln Phe Glu Lys Gly Ser Ala Glu Gln Lys Leu Ile Ser Glu Glu Asp
   50 55 60
   Leu Gly
   65
<210> 370
   <211> 12
   <212> PRT
   <213> Artificial Sequence
<220>
   <223> Description of Artificial Sequence: Synthetic peptide
<400> 370
   Ser Tyr Thr Tyr Asn Tyr Glu His Val Asp Val Trp
   1 5 10
<210> 371
   <211> 3
   <212> **PRT**
   <213> Artificial Sequence
<220>
   <223> Description of Artificial Sequence: Synthetic peptide
<400> 371
   Asn Gly Leu
   1
<210> 372
   <211> 5
   <212> PRT
   <213> Artificial Sequence
<220>
   <223> Description of Artificial Sequence: Synthetic peptide
<400> 372
   Met Met Met Met Met
   1 5
<210> 373
   <211> 5
   <212> PRT
   <213> Artificial Sequence
<220>
   <223> Description of Artificial Sequence: Synthetic peptide
<400> 373
   Ala Met Met Met Met
   1               5
<210> 374
   <211> 5
   <212> PRT
   <213> Artificial Sequence
<220>
   <223> Description of Artificial Sequence: Synthetic peptide
<400> 374
   Pro Met Met Met Met
   1               5
<210> 375
   <211> 5
   <212> PRT
   <213> Artificial Sequence
<220>
   <223> Description of Artificial Sequence: Synthetic peptide
<400> 375
   Val Met Met Met Met
   1               5
<210> 376
   <211> 5
   <212> PRT
   <213> Artificial Sequence
<220>
   <223> Description of Artificial Sequence: Synthetic peptide
<400> 376
   Gln Met Met Met Met
   1               5
<210> 377
   <211> 5
   <212> PRT
   <213> Artificial Sequence
<220>
   <223> Description of Artificial Sequence: Synthetic peptide
<400> 377
   Met Ala Met Met Met
   1 5
<210> 378
   <211> 5
   <212> PRT
   <213> Artificial Sequence
<220>
   <223> Description of Artificial Sequence: Synthetic peptide
<400> 378
   Met Gln Met Met Met
   1 5
<210> 379
   <211> 5
   <212> PRT
   <213> Artificial Sequence
<220>
   <223> Description of Artificial Sequence: Synthetic peptide
<400> 379
   Met Pro Met Met Met
   1 5
<210> 380
   <211> 5
   <212> PRT
   <213> Artificial Sequence
<220>
   <223> Description of Artificial Sequence: Synthetic peptide
<400> 380
   Met Asn Met Met Met
   1 5
<210> 381
   <211> 5
   <212> PRT
   <213> Artificial Sequence
<220>
   <223> Description of Artificial Sequence: Synthetic peptide
<400> 381
   Pro Ala Met Met Met
   1 5
<210> 382
   <211> 5
   <212> PRT
   <213> Artificial Sequence
<220>
   <223> Description of Artificial Sequence: Synthetic peptide
<400> 382
   Pro Phe Met Met Met
   1               5
<210> 383
   <211> 5
   <212> PRT
   <213> Artificial Sequence
<220>
   <223> Description of Artificial Sequence: Synthetic peptide
<400> 383
   Pro Val Met Met Met
   1               5
<210> 384
   <211> 5
   <212> PRT
   <213> Artificial Sequence
<220>
   <223> Description of Artificial Sequence: Synthetic peptide
<400> 384
   Pro Glu Met Met Met
   1               5
<210> 385
   <211> 5
   <212> PRT
   <213> Artificial Sequence
<220>
   <223> Description of Artificial Sequence: Synthetic peptide
<400> 385
   Val Ala Met Met Met
   1               5
<210> 386
   <211> 5
   <212> PRT
   <213> Artificial Sequence
<220>
   <223> Description of Artificial Sequence: Synthetic peptide
<400> 386
   Val Phe Met Met Met
   1               5
<210> 387
   <211> 5
   <212> PRT
   <213> Artificial Sequence
<220>
   <223> Description of Artificial Sequence: Synthetic peptide
<400> 387
   Pro Val Met Met Met
   1               5
<210> 388
   <211> 5
   <212> PRT
   <213> Artificial Sequence
<220>
   <223> Description of Artificial Sequence: Synthetic peptide
<400> 388
   Val Glu Met Met Met
   1               5
<210> 389
   <211> 5
   <212> PRT
   <213> Artificial Sequence
<220>
   <223> Description of Artificial Sequence: Synthetic peptide
<400> 389
   Ala Ala Met Met Met
   1               5
<210> 390
   <211> 5
   <212> PRT
   <213> Artificial Sequence
<220>
   <223> Description of Artificial Sequence: Synthetic peptide
<400> 390
   Ala Phe Met Met Met
   1               5
<210> 391
   <211> 5
   <212> PRT
   <213> Artificial Sequence
<220>
   <223> Description of Artificial Sequence: Synthetic peptide
<400> 391
   Ala Val Met Met Met
   1 5
<210> 392
   <211> 5
   <212> PRT
   <213> Artificial Sequence
<220>
   <223> Description of Artificial Sequence: Synthetic peptide
<400> 392
   Ala Glu Met Met Met
   1 5
<210> 393
   <211> 4
   <212> PRT
   <213> Artificial Sequence
<220>
   <223> Description of Artificial Sequence: Synthetic peptide
<400> 393
   Met Met Met Met
   1
<210> 394
   <211> 6
   <212> PRT
   <213> Artificial Sequence
<220>
   <223> Description of Artificial Sequence: Synthetic peptide
<220>
   <221> MOD_RES
   <222> (1)..(1)
   <223> Any of the 20 standard amino acids
<220>
   <223> See specification as filed for detailed description of substitutions and preferred embodiments
<400> 394
   Xaa Met Met Met Met Met
   1 5
<210> 395
   <211> 6
   <212> PRT
   <213> Artificial Sequence
<220>
   <223> Description of Artificial Sequence: Synthetic peptide
<220>
   <221> MOD_RES
   <222> (2)..(2)
   <223> Any of the 20 standard amino acids
<220>
   <223> See specification as filed for detailed description of substitutions and preferred embodiments
<400> 395
   Met Xaa Met Met Met Met
   1 5
<210> 396
   <211> 6
   <212> PRT
   <213> Artificial Sequence
<220>
   <223> Description of Artificial Sequence: Synthetic peptide
<220>
   <221> MOD_RES
   <222> (3)..(3)
   <223> Any of the 20 standard amino acids
<220>
   <223> See specification as filed for detailed description of substitutions and preferred embodiments
<400> 396
   Met Met Xaa Met Met Met
   1 5
<210> 397
   <211> 6
   <212> PRT
   <213> Artificial Sequence
<220>
   <223> Description of Artificial Sequence: Synthetic peptide
<220>
   <221> MOD_RES
   <222> (4)..(4)
   <223> Any of the 20 standard amino acids
<220>
   <223> See specification as filed for detailed description of substitutions and preferred embodiments
<400> 397
   Met Met Met Xaa Met Met
   1 5
<210> 398
   <211> 6
   <212> PRT
   <213> Artificial Sequence
<220>
   <223> Description of Artificial Sequence: Synthetic peptide
<220>
   <221> MOD_RES
   <222> (5)..(5)
   <223> Any of the 20 standard amino acids
<220>
   <223> See specification as filed for detailed description of substitutions and preferred embodiments
<400> 398
   Met Met Met Met Xaa Met
   1 5
<210> 399
   <211> 6
   <212> PRT
   <213> Artificial Sequence
<220>
   <223> Description of Artificial Sequence: Synthetic peptide
<220>
   <221> MOD_RES
   <222> (6)..(6)
   <223> Any of the 20 standard amino acids
<220>
   <223> See specification as filed for detailed description of substitutions and preferred embodiments
<400> 399
   Met Met Met Met Met Xaa
   1 5
<210> 400
   <211> 15
   <212> PRT
   <213> Artificial Sequence
<220>
   <223> Description of Artificial Sequence: Synthetic peptide
<400> 400
   Met Gly Cys Thr Ser Val His Gln Glu Thr Lys Lys Tyr Gln Ser
   1               5                   10                  15
<210> 401
   <211> 27
   <212> PRT
   <213> Artificial Sequence
<220>
   <223> Description of Artificial Sequence: Synthetic peptide
<400> 401
   Ser Tyr Thr Tyr Asn Tyr Glu His Val Asp Val Trp Gly Cys Gly Ser
   1               5                   10                  15
   Ala Asp Tyr Lys Asp Asp Asp Asp Lys Lys Lys
               20                  25
<210> 402
   <211> 15
   <212> PRT
   <213> Artificial Sequence
<220>
   <223> Description of Artificial Sequence: Synthetic peptide
<400> 402
   Gly Cys Gly Ser Ala Asp Tyr Lys Asp Asp Asp Asp Lys Lys Lys
   1               5                   10                  15

## Description

### TECHNICAL FIELD

This invention relates to a method of selecting a peptide of interest using an mRNA-displayed cow antibody scaffold polypeptide. The invention also relates to the mRNA-displayed cow antibody scaffold polypeptide comprising a peptide of interest and to a peptide microarray comprising the cow antibody scaffold polypeptide comprising a peptide of interest or to a peptide microarray comprising a peptide of interest as described herein.

### BACKGROUND

With the identification of cellular pathways and targets that play key roles in metabolism and disease progression, the understanding of disease states continues to expand exponentially. However, our ability to treat diseases lags behind due to the limitations inherent in existing drug platforms. At present, the available drug platforms are based primarily on small molecules and therapeutic proteins, which address only about 10 to 20 percent of the identified therapeutic targets for treatment of diseases. The majority of targets and diseases are presently "undruggable" using existing therapeutic modalities.

Peptides combine the high specificity of biological drugs with the bioavailability of small molecules, and, thus, offer exciting opportunities to address difficult targets for disease treatment. In fact, peptides have proven to be effective when used to target extracellular receptors and numerous efforts have been made to use peptides to modulate intracellular processes. However, great challenges still remain as peptides are rapidly metabolized by proteolytic enzymes, and they typically do not readily cross cell membranes.

With their conformation rigidity, cyclic peptides show great promise to overcome these limitations. Cyclic peptides are polypeptide chains taking cyclic ring structure. The ring structure can be formed by linking one end of the peptide to the other with an amide bond, or other chemically stable bonds such as lactone, ether, thioether, disulfide, etc. The rigidity of cyclic peptides decreases the free energy and, therefore, allows enhanced binding to target molecules. In addition, due to their lack of free termini, cyclic peptides are more resistant to digestion. Also, it has previously been shown that cyclic peptides have better cell-penetrating capability than their linear counterparts. These unique properties have made cyclic peptides attractive candidates for drug discovery. In the past, cyclic peptides have been isolated from large combinatorial libraries using library screening tools, such as phage display and mRNA display. mRNA display is an attractive possibility for peptide screening due to its *in vitro* nature, large library size, and the capability to include natural, non-natural, and modified amino acids.

In this context, a skilled person prior to the invention was made was aware of the following prior art:
FENG WANG ET AL: "Reshaping Antibody Diversity", CELL, vol. 153, no. 6, 1 June 2013 (2013-06-01), pages 1379-1393,
"World ADC Cow Antibodies: A New Structural Class of Antibody Using Ultra long CDR3s", Scripps Res Inst, Dept Integrat Struct and Computat Biol, La Jolla, CA 92037 USA;
"In vitro evolution of single-chain antibodies using mRNA display", NUCLEIC ACIDS RESEARCH, OXFORD UNIVERSITY PRESS, GB, vol. 34, no. 19, 1 November 2006
"Directed evolution of high-affinity antibody mimics using mRNA display", CHEMISTRY AND BIOLOGY, CURRENT BIOLOGY, LONDON, GB, vol. 9, no. 8, 1 August 2002
WO 2010/039852 A2
EP 2 647 704 A1
EP 2 615 455 A1

### SUMMARY

Applicants have created novel display and selection methods for cyclic peptides using a cow antibody scaffold polypeptide to present a peptide library in a structurally constrained manner. Bovine antibodies have the longest CDR H3 regions currently known, with an ultralong subset that ranges in length from 50 to 67 amino acids. These unusual CDR H3 regions often have multiple cysteines and confer a novel stalk-and-knob conformation. By adopting the unique sequences encoding the stalk and knob region, Applicants have created cow antibody scaffold polypeptides that can present confined peptides by utilizing the protruding knob compartment of cow antibodies. The use of Applicants' unique cow antibody scaffold polypeptides with high peptide versatility may not only allow selection and optimization of cyclic peptide therapeutics, but may also allow selection of cyclic peptides that can penetrate dome clefts in the target molecule (e.g., a protein) and can bind with high-affinity and specificity due to adjacent areas of surface complementarity.

In one embodiment, a method of selecting a peptide of interest is provided. The method comprises the steps of a) preparing a peptide library using an mRNA-displayed cow antibody scaffold polypeptide comprising the peptide of interest to be identified, b) selecting the peptide of interest from the peptide library by contacting a target molecule with the peptide of interest wherein the target molecule is immobilized on a solid support or is in solution, and c) identifying the amino acid sequence of the peptide of interest.

In another embodiment, an mRNA-displayed cow antibody scaffold polypeptide comprising a peptide of interest is provided.

In yet another embodiment, a peptide microarray is provided comprising a cow antibody scaffold polypeptide comprising a peptide of interest. In another embodiment, a peptide microarray comprising a peptide of interest as described herein is provided.

Several embodiments of the invention are also described by the following enumerated clauses:
1. A method of selecting a peptide of interest, the method comprising the steps of
   a) preparing a peptide library using an mRNA-displayed cow antibody scaffold polypeptide comprising the peptide of interest to be identified;
   b) selecting the peptide of interest from the peptide library by contacting a target molecule with the peptide of interest wherein the target molecule is immobilized on a solid support or is in solution; and
   c) identifying the amino acid sequence of the peptide of interest.
2. The method of clause 1 wherein the step of preparing the peptide library comprises the step of *in vitro* transcription of a DNA library to form an mRNA library.
3. The method of clause 2 further comprising the step of digesting the DNA library with DNase.
4. The method of clause 2 or 3 further comprising the step of conjugating the mRNA from the mRNA library to a puromycin oligonucleotide linker.
5. The method of any one of clauses 2 to 4 further comprising the step of translating *in vitro* the mRNA from the mRNA library to form mRNA-cow antibody scaffold polypeptide fusion conjugates comprising the cow antibody scaffold polypeptide wherein the cow antibody scaffold polypeptide comprises a purification tag.
6. The method of clause 5 further comprising the step of purifying the mRNA-cow antibody scaffold polypeptide fusion conjugates using the purification tag.
7. The method of clause 5 or 6 further comprising the step of reverse transcribing the mRNA from the mRNA library to form mRNA-DNA duplexes of the mRNA-cow antibody scaffold polypeptide fusion conjugates.
8. The method of clause 7 wherein the step of selecting comprises contacting the mRNA-DNA duplexes of the mRNA-cow antibody scaffold polypeptide fusion conjugates with the target molecule.
9. The method of clause 8 further comprising the step of regenerating the DNA from the mRNA-DNA duplexes of the mRNA-cow antibody scaffold polypeptide fusion conjugates.
10. The method of any one of clauses 2 to 9 wherein the DNA library is a cDNA library.
11. The method of any one of clauses 2 to 10 wherein the *in vitro* transcription is performed using T7 RNA polymerase.
12. The method of any one of clauses 4 to 11 wherein the puromycin oligonucleotide linker is linked to the mRNA at the 3' end of the mRNA.
13. The method of any one of clauses 5 to 12 wherein the purification tag is a FLAG tag.
14. The method of any one of clauses 2 to 13 wherein members of the DNA library comprise an RNA polymerase promoter sequence, an enhancer sequence, and a purification tag sequence.
15. The method of any one of clauses 5 to 13 wherein the purification tag is a C-terminal tag.
18. The cow antibody scaffold polypeptide has to comprise the sequence MGCTSVHQETKKYQS(X*)_{c}SYTYNYEHVDVWGCGSADYKDDDDKKK (SEQ ID NO: 3) wherein (X*)_{c} is a random sequence of amino acids, and wherein X* is an amino acid sequence and c is the number of amino acids in the random sequence of amino acids.
19. The method of any one of clauses 18 wherein X* comprises a natural amino acid.
20. The method of any one of clauses 18 wherein X* comprises a non-natural amino acid.
21. The method of any one of clauses 18 to 20 wherein c is an integer from 1 to 40.
22. The method of any one of clauses 18 to 20 wherein c is an integer from 1 to 10.
23. The method of any one of clauses above further comprising the steps of:
   a) synthesizing the peptide of interest, or derivatives thereof, on a first peptide microarray, wherein the derivatives of the peptide of interest include at least one alteration in the sequence of the peptide of interest selected from a single amino acid substitution, a double amino acid substitution, a deletion of one or more amino acids, and an insertion of one or more amino acids, whereby functionalized peptides are generated on the first peptide microarray;
   b) forming from the functionalized peptides, wherein the functionalized peptides are in linear form, cyclic peptides of formula I 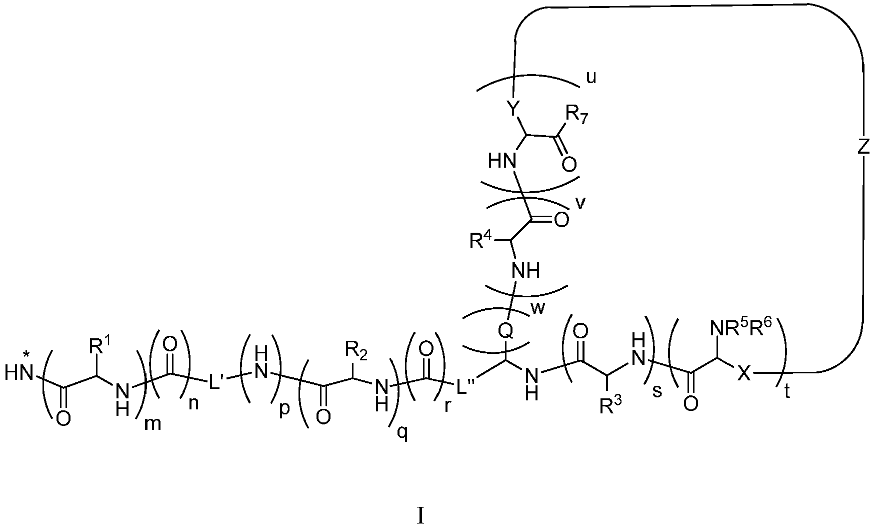
      wherein each R¹, R², R³ and R⁴ is independently a natural amino acid side chain or a non-natural amino acid side chain;
      each R⁵ and R⁶ is independently selected from the group consisting of hydrogen, an N-terminal capping group, and a N-terminal protecting group;
      R⁷ is selected from the group consisting of -OH, a C-terminal capping group, a C-terminal protecting group, and 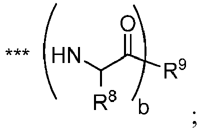
      each R⁸ is independently a natural amino acid side chain or a non-natural amino acid side chain;
      R⁹ is selected from the group consisting of -OH, a C-terminal capping group, and a C-terminal protecting group;
      Q is selected from the group consisting of a bond, a carbonyl, a natural amino acid side chain, and a non-natural amino acid side chain;
      each X and Y is independently selected from the group consisting of a bond, a natural amino acid side chain covalently attached to Z, and a non-natural amino acid side chain covalently attached to Z;
      Z is a group comprising a moiety selected from the group consisting of an amide bond, a disulfide bond, an isopeptide bond, a 1,2,3-triazole, and an optionally substituted 1,2-quinone;
      each L' and L" is independently an optional bivalent linking group or a bond;
      b is an integer from 0 to 50;
      m is an integer from 0 to 6;
      n is 0 or 1;
      p is 0 or 1;
      q is an integer from 0 to 6;
      r is 0 or 1;
      s is an integer from 0 to 100;
      t is 0 or 1;
      u is 0 or 1;
      v is an integer from 0 to 100;
      w is 0 or 1; and * is a point of connection connecting the cyclic peptide to an array support having a reactive surface; and *** is a point of connection to the rest of the functionalized peptide;
      the method comprising the step of reacting a functionalized peptide of formula II under conditions that cause Z to form 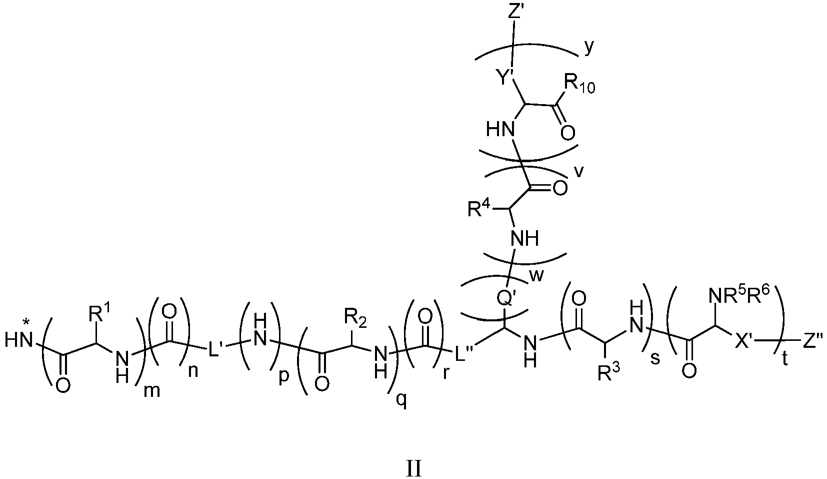
      wherein R¹, R² R³, R⁴, R⁵, R⁶, m, n, p, q, r, s, t, v, w, L', L", *, and *** are as defined for formula I;
      R¹⁰ is selected from the group consisting of -OH, a C-terminal capping group, a C-terminal protecting group, and 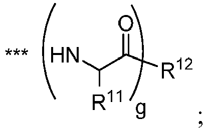
      each R¹¹ is independently a natural amino acid side chain or a non-natural amino acid side chain;
      R¹² is selected from the group consisting of -OH, a C-terminal capping group, and a C-terminal protecting group;
      Q' is selected from the group consisting of a bond, a carbonyl, a natural amino acid side chain covalently attached to Z", and a non-natural amino acid side chain covalently attached to Z";
      X' is selected from the group consisting of a bond, a natural amino acid side chain covalently attached to Z", and a non-natural amino acid side chain covalently attached to Z";
      Y' is selected from the group consisting of a bond, a natural amino acid side chain covalently attached to Z', and a non-natural amino acid side chain covalently attached to Z';
      each Z' and Z" is independently selected from the group consisting of a bond, -OH, hydrogen, a thiol, an amine, a carboxylic acid, an amide, an alkyne, an azide, an optionally substituted aminophenol, a natural amino acid side chain, a non-natural amino acid side chain, an N-terminal protecting group, and a C-terminal protecting group, provided that Z' and Z" are complementary groups that combine to form Z;
      g is an integer from 0 to 50; and
      y is 0 or 1;
      wherein the functionalized peptides and the cyclic peptides are immobilized to the reactive surface;
   c) exposing the cyclic peptides to the target molecule, whereby the target molecule binds to at least one cyclic peptide;
   d) identifying one or more of the cyclic peptides demonstrating strong binding to the target molecule, whereby a matured core binder sequence is determined;
   e) performing at least one of N-terminal and C-terminal extension of the matured core binder sequence determined in step d to provide a matured, extended core binder sequence on a second peptide microarray;
   f) exposing the target molecule to the second peptide microarray comprising a population of matured, extended core binder sequence peptides generated in step e wherein the population of matured, extended core binder sequence peptides comprises cyclic peptides formed as in step b; and
   g) identifying a matured, extended cyclic peptide with strong binding to the target molecule.
24. The method of clause 23, wherein Z comprises a moiety selected from the group consisting of an amide bond, 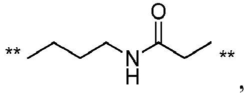 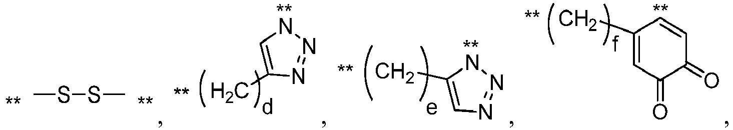 and 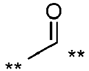 wherein d is an integer from 0 to 6, e is an integer from 0 to 6, and f is an integer from 0 to 6, and ** is a point of connection to the rest of the cyclic peptide.
25. The method of clause 23 or 24, wherein Z comprises a peptide bond, Z" comprises an N-terminal protecting group, t is 0, u is 0, and y is 0.
26. The method of clause 25, further comprising removing Z" from the rest of the functionalized peptide to cause the peptide bond to form.
27. The method of clause 23 or 24, wherein Q and X are bonds to Z, Z comprises ** -S-S- **, X' is a bond to Z", Q' is a bond to Z', Z' and Z" comprise cysteine side chains, t is 1, u is 0, v is 0, w is 1, and y is 0.
28. The method of clause 27, further comprising subjecting the functionalized peptide to oxidative conditions to cause ** -S-S- ** to form.
29. The method of clause 23 or 24, wherein X and Y are bonds to Z, Z comprises ** -S-S- **, X' is a bond to Z", Y' is a bond to Z', Z' and Z" comprise cysteine side chains, t is 1, u is 1, and y is 1.
30. The method of clause 29, further comprising subjecting the functionalized peptide to oxidative conditions to cause ** -S-S- ** to form.
31. The method of clause 23 or 24, wherein Q is a bond to Z, Z comprises 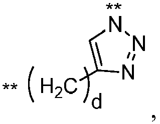 Q' is a bond to Z', Z' comprises 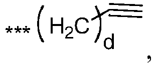 Z" comprises an azide, d is 1, u is 0, v is 0, w is 1, and y is 0.
32. The method of clause 31, further comprising contacting the functionalized peptide with a copper catalyst to cause 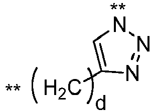 to form.
33. The method of clause 23 or 24, wherein Y is a bond to Z, Z comprises 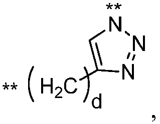 Y' is a bond to Z', Z' comprises 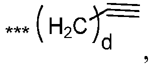 Z" comprises an azide, d is 1, u is 1, and y is 1.
34. The method of clause 33, further comprising contacting the functionalized peptide with a copper catalyst to cause 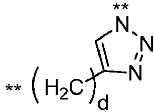 to form.
35. The method of clause 23 or 24, wherein Q is a bond to Z, Z comprises 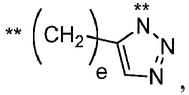 Q' is a bond to Z', Z' comprises 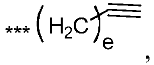 Z" comprises an azide, e is 1, u is 0, v is 0, w is 1, and y is 0.
36. The method of clause 35, further comprising contacting the functionalized peptide with a copper catalyst to cause 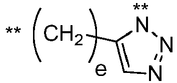 to form.
37. The method of clause 23 or 24, wherein Y is a bond to Z, Z comprises 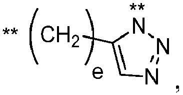 Y' is a bond to Z', Z' comprises 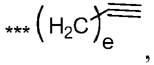 Z" comprises an azide, e is 1, u is 1, and y is 1.
38. The method of clause 37, further comprising contacting the functionalized peptide with a copper catalyst to cause 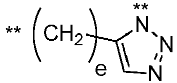 to form.
39. The method of clause 23 or 24, wherein Q is a bond to Z, Z comprises 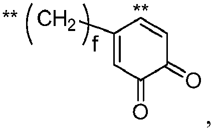 Q' is a bond to Z', Z' comprises 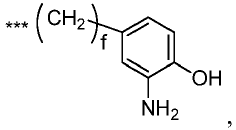 Z" comprises an amine, f is 1, u is 0, v is 0, w is 1, and y is 0.
40. The method of clause 39, further comprising contacting the functionalized peptide with a potassium ferricyanide to cause 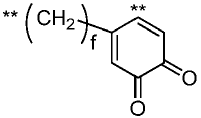 to form.
41. The method of clause 23 or 24, wherein Y is a bond to Z, Z comprises 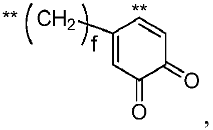 Z" comprises an amine, Y' is a bond to Z', Z' comprises 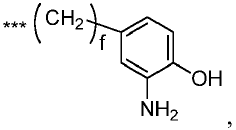 f is 1, u is 1, and y is 1.
42. The method of clause 41, further comprising contacting the functionalized peptide with a potassium ferricyanide to cause 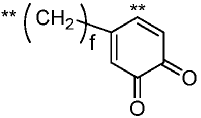 to form.
43. The method of clause 23 or 24, wherein R⁴, R¹⁰, and R¹¹ are defined such that the functionalized peptide comprises a butelase 1 recognition sequence, Y is a bond to Z, Z comprises 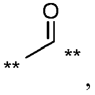 Y' is a bond to Z', Z' is an asparagine or aspartic acid side chain, u is 1, and y is 1.
44. The method of clause 43, further comprising contacting the functionalized peptide with butelase 1 to cause 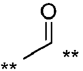 to form.
45. The method of clause 23 or 24, wherein Q and X are bonds to Z, Z comprises 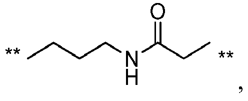 Q' is a bond to Z', X' is a bond to Z", Z' is a glutamine side chain and Z" is a lysine side chain or Z' is a lysine side chain and Z" is a glutamine side chain, t is 1, u is 0, v is 0, w is 1, and y is 0.
46. The method of clause 45, further comprising contacting the functionalized peptide with a microbial transglutaminase to cause 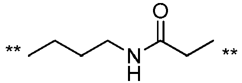 to form.
47. The method of clause 23 or 24, wherein X and Y are bonds to Z, Z comprises 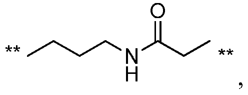 X' is a bond to Z", Y' is a bond to Z', Z' is a glutamine side chain and Z" is a lysine side chain or Z' is a lysine side chain and Z" is a glutamine side chain, t is 1, u is 1, and y is 1.
48. The method of clause 47, further comprising contacting the functionalized peptide with a microbial transglutaminase to cause 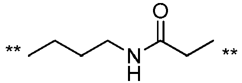 to form.
49. The method of any one of clauses 23 to 48, wherein each L' and L" is independently of the formula V 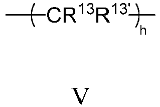 wherein each R¹³ and R^{13'} is independently selected from the group consisting of H, D, halogen, C₁-C₆ alkyl, C₂-C₆ alkenyl, C₂-C₆ alkynyl, C₃-C₆ cycloalkyl, 3- to 7-membered heterocycloalkyl, C₆-C₁₀ aryl, 5- to 7-membered heteroaryl, -OR¹⁴, -OC(O)R¹⁴, -NR¹⁴R^{14'}, -NR¹⁴C(O)R¹⁵, -C(O)R¹⁴, -C(O)OR¹⁴, and -C(O)NR¹⁴R^{14'}, wherein each hydrogen atom in C₁-C₆ alkyl, C₂-C₆ alkenyl, C₂-C₆ alkynyl, C₃-C₆ cycloalkyl, 3- to 7-membered heterocycloalkyl, C₆-C₁₀ aryl and 5- to 7-membered heteroaryl is independently optionally substituted by halogen, C₁-C₆ alkyl, C₂-C₆ alkenyl, C₂-C₆ alkynyl, -OR¹⁶; each R¹⁴, R^{14'}, R¹⁵, and R¹⁶ is independently selected from the group consisting of H, D, hydroxyl, C₁-C₇ alkyl, C₂-C₇ alkenyl, C₂-C₇ alkynyl, C₃-C₆ cycloalkyl, 3- to 7-membered heterocycloalkyl, C₆-C₁₀ aryl and 5- to 7-membered heteroaryl; and h is an integer from 1 to 10; or the formula VI or VII 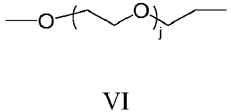 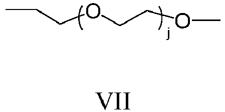 wherein j is an integer from 0 to 30.
50. The method of clause 49, wherein each R¹³ and R^{13'} is hydrogen.
51. The method of clause 49 or 50, wherein L' is present, h is 5, m is 0, n is 1, and p is 1.
52. The method of clause 49, wherein at least one of L' and L" is of the formula VI or VII 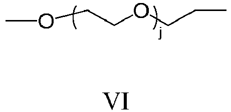 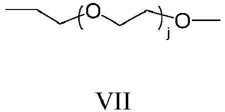 wherein j is 7.
53. The method of any one of clauses 23 to 52, wherein the N-terminal protecting group is a photoprotecting group.
54. The method of any one of clauses 23 to 53, wherein the N-terminal protecting group is 2-(2-nitrophenyl)propyloxycarbonyl.
55. The method of any one of clauses 1 to 22 further comprising the step of synthesizing the cow antibody scaffold polypeptide on one or more peptide microarrays wherein the cow antibody scaffold polypeptide comprises the peptide of interest.
56. The method of clause 55 comprising the steps of:
   a) synthesizing the cow antibody scaffold polypeptide comprising the peptide of interest, or derivatives of the peptide of interest, on a first peptide microarray, wherein the derivatives of the peptide of interest include at least one alteration in the sequence of the peptide of interest selected from a single amino acid substitution, a double amino acid substitution, a deletion of one or more amino acids, and an insertion of one or more amino acids, whereby functionalized peptides are generated on the first peptide microarray;
   b) forming from the functionalized peptides, wherein the functionalized peptides are in linear form, cyclic peptides of formula VIII 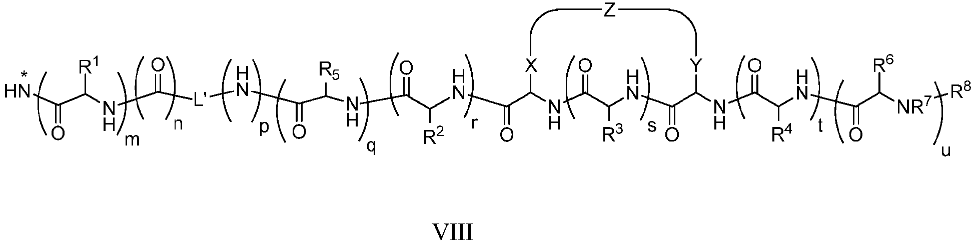
      wherein each R¹, R², R³, and R⁴ is independently a natural amino acid side chain or a non-natural amino acid side chain;
      each R⁵ and R⁶ is independently a natural amino acid side chain or a non-natural amino acid side chain selected such that 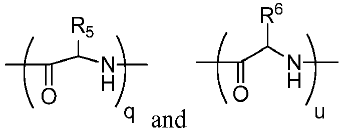 can form a beta-sheet;
      each R⁷ is independently selected from the group consisting of hydrogen, an N-terminal capping group, and an N-terminal protecting group;
      R⁸ is selected from the group consisting of hydrogen, an N-terminal capping group, and a protecting group;
      each X and Y is independently selected from the group consisting of a bond, a natural amino acid side chain covalently attached to Z, and a non-natural amino acid side chain covalently attached to Z;
      Z is a group comprising a moiety selected from the group consisting of an amide bond, a disulfide bond, an isopeptide bond, a 1,2,3-triazole, and an optionally substituted 1,2-quinone;
      L' is an optional bivalent linking group or a bond;
      m is an integer from 0 to 6;
      n is 0 or 1;
      p is 0 or 1;
      q is an integer from 0 to 50;
      r is an integer from 0 to 50;
      s is an integer from 0 to 50;
      t is an integer from 0 to 50;
      u is an integer from 0 to 50; and * is a point of connection connecting the cyclic peptide to an array support having a reactive surface;
      the method comprising the step of reacting a functionalized peptide of formula IX under conditions that cause Z to form 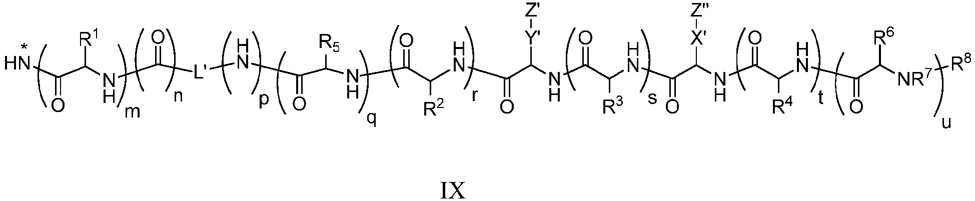
      wherein R¹, R² R³, R⁴, R⁵, R⁶, R⁷, and R⁸, m, n, p, q, r, s, t, u, L' and * are as defined for formula VIII;
      X' is selected from the group consisting of a bond, a natural amino acid side chain covalently attached to Z", and a non-natural amino acid side chain covalently attached to Z";
      Y' is selected from the group consisting of a bond, a natural amino acid side chain covalently attached to Z', and a non-natural amino acid side chain covalently attached to Z'; and
      each Z' and Z" is independently selected from the group consisting of a bond, -OH, hydrogen, a thiol, an amine, a carboxylic acid, an amide, an alkyne, an azide, an optionally substituted aminophenol, a natural amino acid side chain, a non-natural amino acid side chain, an N-terminal protecting group, and a C-terminal protecting group, provided that Z' and Z" are complementary groups that combine to form Z;
      wherein the functionalized peptides and the cyclic peptides are immobilized to the reactive surface;
   c) exposing the cyclic peptides to the target molecule, whereby the target molecule binds to at least one cyclic peptide;
   d) identifying one or more of the cyclic peptides demonstrating strong binding to the target molecule, whereby a matured core binder sequence is determined;
   e) performing at least one of N-terminal and C-terminal extension of the matured core binder sequence determined in step d to provide a matured, extended core binder sequence on a second peptide microarray;
   f) exposing the target molecule to the second peptide microarray comprising a population of matured, extended core binder sequence peptides generated in step e wherein the population of matured, extended core binder sequence peptides comprises cyclic peptides formed as in step b; and
   g) identifying a matured, extended cyclic peptide with strong binding to the target molecule.
57. The method of clause 56, wherein Z comprises a moiety selected from the group consisting of an amide bond, 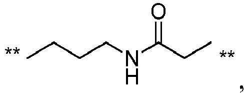 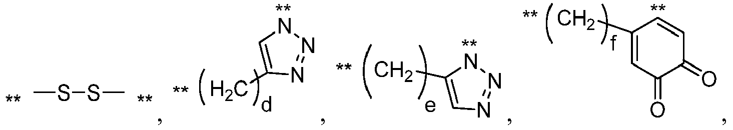 and 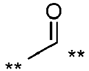 wherein d is an integer from 0 to 6, e is an integer from 0 to 6, and f is an integer from 0 to 6, and ** is a point of connection to the rest of the cyclic peptide.
58. The method of clause 56 or 57, wherein X and Y are bonds to Z, Z comprises ** -S-S- **, X' is a bond to Z", Y' is a bond to Z', and Z' and Z" comprise cysteine side chains.
59. The method of clause 58, further comprising subjecting the functionalized peptide to oxidative conditions to cause ** -S-S- ** to form.
60. The method of claim 56 or 57, wherein Y is a bond to Z, Z comprises 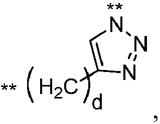 Y' is a bond to Z', Z' comprises 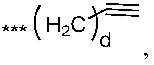 Z" comprises an azide, and d is 1.
61. The method of clause 60, further comprising contacting the functionalized peptide with a copper catalyst to cause 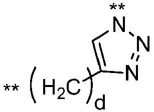 to form.
62. The method of clause 56 or 57, wherein Y is a bond to Z, Z comprises 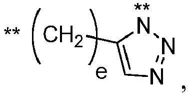 Y' is a bond to Z', Z' comprises 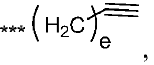 Z" comprises an azide, and e is 1.
63. The method of clause 62, further comprising contacting the functionalized peptide with a copper catalyst to cause 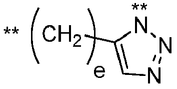 to form.
64. The method of clause 56 or 57, wherein Y is a bond to Z, Z comprises 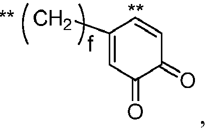 Y' is a bond to Z', Z' comprises 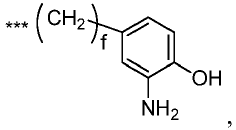 Z" comprises an amine, and f is 1.
65. The method of clause 64, further comprising contacting the functionalized peptide with a potassium ferricyanide to cause 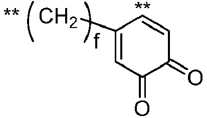 to form.
66. The method of clause 56 or 57, wherein X and Y are bonds to Z, Z comprises 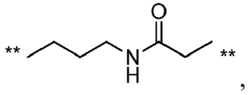 X' is a bond to Z", Y' is a bond to Z', and Z' is a glutamine side chain and Z" is a lysine side chain or Z' is a lysine side chain and Z" is a glutamine side chain.
67. The method of clause 66, further comprising contacting the functionalized peptide with a microbial transglutaminase to cause 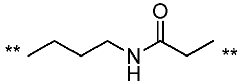 to form.
68. The method of any one of clauses 56 to 67, wherein L' is of the formula (X) 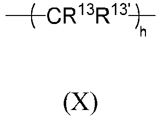 wherein each R¹³ and R^{13'} is independently selected from the group consisting of H, D, halogen, C₁-C₆ alkyl, C₂-C₆ alkenyl, C₂-C₆ alkynyl, C₃-C₆ cycloalkyl, 3- to 7-membered heterocycloalkyl, C₆-C₁₀ aryl, 5- to 7-membered heteroaryl, -OR¹⁴,-OC(O)R¹⁴, -NR¹⁴R^{14'}, -NR¹⁴C(O)R¹⁵, -C(O)R¹⁴, -C(O)OR¹⁴, and -C(O)NR¹⁴R^{14'}, wherein each hydrogen atom in C₁-C₆ alkyl, C₂-C₆ alkenyl, C₂-C₆ alkynyl, C₃-C₆ cycloalkyl, 3- to 7-membered heterocycloalkyl, C₆-C₁₀ aryl and 5- to 7-membered heteroaryl is independently optionally substituted by halogen, C₁-C₆ alkyl, C₂-C₆ alkenyl, C₂-C₆ alkynyl, -OR¹⁶; each R¹⁴, R^{14'}, R¹⁵, and R¹⁶ is independently selected from the group consisting of H, D, hydroxyl, C₁-C₇ alkyl, C₂-C₇ alkenyl, C₂-C₇ alkynyl, C₃-C₆ cycloalkyl, 3- to 7-membered heterocycloalkyl, C₆-C₁₀ aryl and 5- to 7-membered heteroaryl; and h is an integer from 1 to 10; or the formula XI or XII 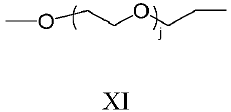 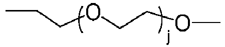 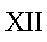 wherein j is an integer from 0 to 30.
69. The method of clause 68, wherein each R¹³ and R^{13'} is hydrogen.
70. The method of clause 68 or 69, wherein L' is present, h is 5, m is 0, n is 1, and p is 1.
71. The method of clause 68, wherein L' is of the formula XI or XII 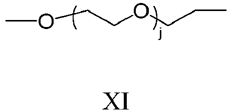 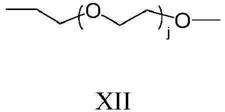 wherein j is 7.
72. The method of any one of clauses 56 to 71, wherein the N-terminal protecting group is a photoprotecting group.
73. The method of any one of clauses 56 to 72, wherein the N-terminal protecting group is 2-(2-nitrophenyl)propyloxycarbonyl.
74. The method of any one of clauses 23 to 54 and 56 to 73, wherein at least one of a label-free and an affinity analysis of the matured, extended core binder sequence peptides is performed.
75. The method of any one of clauses 23 to 54 and 56 to 74, wherein the first or second peptide microarray comprises at least one of glass, plastic, and carbon composite.
76. The method any one of clauses 23 to 54 and 56 to 75, wherein the functionalized peptides and the cyclic peptides on the first or the second peptide microarray comprise the same number of amino acids.
77. The method of any one of clauses 23 to 54 and 56 to 76, wherein the functionalized peptides and the cyclic peptides on the first or the second peptide microarray do not include the amino acid cysteine or methionine, or histidine-proline-glutamine motifs, or amino acid repeats of 2 or more amino acids.
78. The method of any one of clauses 23 to 54 and 56 to 77, wherein the population of matured, extended core binder sequence peptides includes at least one of an N-terminal wobble synthesis oligopeptide and a C-terminal wobble synthesis oligopeptide.
79. The method of any one of clauses 23 to 54 and 56 to 78 wherein the first or second peptide microarray comprises one or more linear peptides and wherein the method further comprises the step of contacting the one or more linear peptides on the first or second peptide microarray with a protease capable of digesting the one or more linear peptides.
80. The method of clause 79 wherein the protease is an amino protease or a mixture of amino proteases.
81. The method of clause 79 wherein the protease is dipeptidyl peptidase IV, aminopeptidase m, or a combination thereof.
82. An mRNA-displayed cow antibody scaffold polypeptide comprising a peptide of interest.
83. The mRNA-displayed cow antibody scaffold polypeptide of clause 82 comprising the sequence MGCTSVHQETKKYQS(X*)_{c}SYTYNYEHVDVWGCG (SEQ ID NO: 1) wherein (X*)_{c} is a random sequence of amino acids, and wherein X* is an amino acid sequence and c is the number of amino acids in the random sequence of amino acids.
84. The mRNA-displayed cow antibody scaffold polypeptide of clause 82 comprising the sequence MGCTSVHQETKKYQS(X*)_{c}SYTYNYEHVDVWGCGSADYKDDDDK (SEQ ID NO: 2) wherein (X*)_{c} is a random sequence of amino acids, and wherein X* is an amino acid sequence and c is the number of amino acids in the random sequence of amino acids.
85. The mRNA-displayed cow antibody scaffold polypeptide of clause 82 comprising the sequence MGCTSVHQETKKYQS(X*)_{c}SYTYNYEHVDVWGCGSADYKDDDDKKK (SEQ ID NO: 3) wherein (X*)_{c} is a random sequence of amino acids, and wherein X* is an amino acid sequence and c is the number of amino acids in the random sequence of amino acids.
86. The mRNA-displayed cow antibody scaffold polypeptide of any one of clauses 83 to 85 wherein X* comprises a natural amino acid.
87. The mRNA-displayed cow antibody scaffold polypeptide of any one of clauses 83 to 85 wherein X* comprises a non-natural amino acid.
88. The mRNA-displayed cow antibody scaffold polypeptide of any one of clauses 83 to 87 wherein c is an integer from 1 to 40.
89. The mRNA-displayed cow antibody scaffold polypeptide of any one of clauses 83 to 87 wherein c is an integer from 1 to 10.
90. The mRNA-displayed cow antibody scaffold polypeptide of any one of clauses 83 to 89 wherein (X*)_{c} comprises the sequence of the peptide of interest.
91. The mRNA-displayed cow antibody scaffold polypeptide of clause 90 wherein the peptide of interest is a therapeutic peptide.
92. A peptide microarray comprising a cow antibody scaffold polypeptide comprising a peptide of interest.
93. The peptide microarray of clause 92 wherein the cow antibody scaffold polypeptide comprises the sequence MGCTSVHQETKKYQS(X*)_{c}SYTYNYEHVDVWGCG (SEQ ID NO: 1) wherein (X*)_{c} is a random sequence of amino acids, and wherein X* is an amino acid sequence and c is the number of amino acids in the random sequence of amino acids.
94. The peptide microarray of clause 92 wherein the cow antibody scaffold polypeptide comprises the sequence MGCTSVHQETKKYQS(X*)_{c}SYTYNYEHVDVWGCGSADYKDDDDK (SEQ ID NO: 2) wherein (X*)_{c} is a random sequence of amino acids, and wherein X* is an amino acid sequence and c is the number of amino acids in the random sequence of amino acids.
95. The peptide microarray of clause 92 wherein the cow antibody scaffold polypeptide comprises the sequence MGCTSVHQETKKYQS(X*)_{c}SYTYNYEHVDVWGCGSADYKDDDDKKK (SEQ ID NO: 3) wherein (X*)_{c} is a random sequence of amino acids, and wherein X* is an amino acid sequence and c is the number of amino acids in the random sequence of amino acids.
96. The peptide microarray of any one of clauses 93 to 95 wherein X* comprises a natural amino acid.
97. The peptide microarray of any one of clauses 93 to 95 wherein X* comprises a non-natural amino acid.
98. The peptide microarray of any one of clauses 93 to 97 wherein c is an integer from 1 to 40.
99. The peptide microarray of any one of clauses 93 to 97 wherein c is an integer from 1 to 10.
100. The peptide microarray of any one of clauses 93 to 99 wherein (X*)_{c} comprises the sequence of the peptide of interest.
101. The peptide microarray of clause 100 wherein the peptide of interest is a therapeutic peptide.
102. The method, mRNA-displayed cow antibody scaffold polypeptide, or peptide microarray of any one of clauses 1 to 101 wherein the cow antibody scaffold polypeptide comprises a random amino acid sequence cassette and the random amino acid sequence cassette comprises the sequence of the peptide of interest.
103. The method, mRNA-displayed cow antibody scaffold polypeptide, or peptide microarray of clause 102 wherein the random amino acid sequence cassette does not include the sequence of an antibody, a fragment of an antibody, or a peptide fragment derived from an antibody.
104. The method of clause 43 or 44 wherein the butelase 1 recognition sequence is NHV.
105. The method of clause 45, 46, 66, or 67 wherein the glutamine side chain is part of the sequence [WY][DE][DE][YW]ALQ[GST]YD (SEQ ID NO:4) and the lysine side chain is part of the sequence RSKLG (SEQ ID NO:5).
106. The method of any one of clauses 1 to 22 further comprising the step of synthesizing the cow antibody scaffold polypeptide comprising the peptide of interest on a peptide microarray to mature and/or extend the peptide of interest or the step of synthesizing the peptide of interest on a peptide microarray to mature and/or extend the peptide of interest.
107. The method, mRNA-displayed cow antibody scaffold polypeptide, or peptide microarray of any one of clauses 1 to 101 wherein the cow antibody scaffold polypeptide comprises a random amino acid sequence cassette wherein the random amino acid sequence cassette comprises the sequence of the peptide of interest and wherein the random amino acid sequence cassette is used for selection of peptides of interest.
108. A cow antibody scaffold polypeptide comprising a random amino acid sequence cassette.

### BRIEF DESCRIPTION OF THE DRAWINGS

**Figure 1** shows a schematic drawing of a cow antibody scaffold polypeptide. Figure 1 discloses SEQ ID NOS 400 and 401, respectively, in order of appearance.
**Figure 2** shows a denaturing gel of a single thrombin-binding peptide grafted on a cow antibody scaffold polypeptide.
**Figure 3** shows agarose gel electrophoresis of various PCR cycles to determine detection levels of amplification products.
**Figure 4** shows a Biacore sensorgram of a single cycle kinetics measurement of crude peptides from a translation mixture and derived binding constants.
**Figure 5** shows Biacore sensorgrams of multiple cycle kinetics measurements of selected thrombin binders. Figure 5 discloses SEQ ID NOS 367-368, respectively, in order of appearance.
**Figure 6** shows a schematic drawing of an N (N-terminal base) motif, an A (ascending beta-strand) motif, an R (random) motif, a D (descending beta-strand) motif, and a C (C-terminal base) motif (SEQ ID NO: 402) in a cow antibody scaffold polypeptide.

### DETAILED DESCRIPTION OF ILLUSTRATIVE EMBODIMENTS

As used herein, "cow antibody scaffold polypeptide" means a polypeptide comprising sequences from the CDR H3 region of a cow antibody. The cow antibody scaffold polypeptides described herein are useful for mRNA display of peptides of interest and *in situ* synthesis on a peptide microarray of the peptides of interest to generate linear or cyclic peptides.

In one embodiment, the term "peptide" or "peptides" in the phrases "peptide of interest", "peptide microarray", "first peptide microarray", "second peptide microarray", "cyclic peptide", "linear peptide", "functionalized peptide", "core binder peptide", "mature cyclic peptide of interest", "mature, extended core binder sequence peptide", "mature, extended cyclic peptide", and "peptide library" does not mean an antibody, a fragment of an antibody, or a peptide fragment derived from an antibody.

In various embodiments, the peptides of interest, cyclic peptides, linear peptides, functionalized peptides, core binder peptides, mature cyclic peptides of interest, mature, extended core binder peptides, and mature, extended cyclic peptides described herein can be from 4 to 50 amino acids, from 4 to 40 amino acids, from 4 to 30 amino acids, from 4 to 20 amino acids, from 4 to 19 amino acids, from 4 to 18 amino acids, from 4 to 17 amino acids, from 4 to 16 amino acids, from 4 to 15 amino acids, from 4 to 14 amino acids, from 4 to 13 amino acids, from 4 to 12 amino acids, from 4 to 11 amino acids, from 4 to 10 amino acids, from 4 to 9 amino acids, from 4 to 8 amino acids, from 4 to 7 amino acids, from 4 to 6 amino acids, or the peptides can be 5 amino acids in length or about 5 amino acids in length. The amino acids described herein can be natural or non-natural amino acids.

In the embodiments described herein, the term "natural amino acid" refers to one of the 20 amino acids typically found in proteins and used for protein biosynthesis as well as other amino acids which can be incorporated into proteins during translation (including pyrrolysine and selenocysteine). The 20 natural amino acids include histidine, alanine, valine, glycine, leucine, isoleucine, aspartic acid, glutamic acid, serine, glutamine, asparagine, threonine, arginine, proline, phenylalanine, tyrosine, tryptophan, cysteine, methionine and lysine.

In the embodiments described herein, the term "non-natural amino acid" refers to an organic compound that is not among those encoded by the standard genetic code, or incorporated into proteins during translation. Therefore, non-natural amino acids include, but are not limited to, amino acids or analogs of amino acids, the D-isostereomers of amino acids, the beta-amino-analogs of amino acids, citrulline, homocitrulline, homoarginine, hydroxyproline, homoproline, ornithine, 4-amino-phenylalanine, cyclohexylalanine, α-aminoisobutyric acid, N-methyl-alanine, N-methyl-glycine, norleucine, N-methyl-glutamic acid, tert-butylglycine, α-aminobutyric acid, tert-butylalanine, 2-aminoisobutyric acid, α-aminoisobutyric acid, 2-aminoindane-2-carboxylic acid, selenomethionine, dehydroalanine, lanthionine, γ-amino butyric acid, and derivatives thereof wherein the amine nitrogen has been mono- or di-alkylated.

Several embodiments of the invention are described in the Summary section of this patent application and each of the embodiments described in this Detailed Description section of the application applies to the embodiments described in the Summary, including the embodiments described by the enumerated clauses below.
1. A method of selecting a peptide of interest, the method comprising the steps of
   a) preparing a peptide library using an mRNA-displayed cow antibody scaffold polypeptide comprising the peptide of interest to be identified;
   b) selecting the peptide of interest from the peptide library by contacting a target molecule with the peptide of interest wherein the target molecule is immobilized on a solid support or is in solution; and
   c) identifying the amino acid sequence of the peptide of interest.
2. The method of clause 1 wherein the step of preparing the peptide library comprises the step of *in vitro* transcription of a DNA library to form an mRNA library.
3. The method of clause 2 further comprising the step of digesting the DNA library with DNase.
4. The method of clause 2 or 3 further comprising the step of conjugating the mRNA from the mRNA library to a puromycin oligonucleotide linker.
5. The method of any one of clauses 2 to 4 further comprising the step of translating *in vitro* the mRNA from the mRNA library to form mRNA-cow antibody scaffold polypeptide fusion conjugates comprising the cow antibody scaffold polypeptide wherein the cow antibody scaffold polypeptide comprises a purification tag.
6. The method of clause 5 further comprising the step of purifying the mRNA-cow antibody scaffold polypeptide fusion conjugates using the purification tag.
7. The method of clause 5 or 6 further comprising the step of reverse transcribing the mRNA from the mRNA library to form mRNA-DNA duplexes of the mRNA-cow antibody scaffold polypeptide fusion conjugates.
8. The method of clause 7 wherein the step of selecting comprises contacting the mRNA-DNA duplexes of the mRNA-cow antibody scaffold polypeptide fusion conjugates with the target molecule.
9. The method of clause 8 further comprising the step of regenerating the DNA from the mRNA-DNA duplexes of the mRNA-cow antibody scaffold polypeptide fusion conjugates.
10. The method of any one of clauses 2 to 9 wherein the DNA library is a cDNA library.
11. The method of any one of clauses 2 to 10 wherein the *in vitro* transcription is performed using T7 RNA polymerase.
12. The method of any one of clauses 4 to 11 wherein the puromycin oligonucleotide linker is linked to the mRNA at the 3' end of the mRNA.
13. The method of any one of clauses 5 to 12 wherein the purification tag is a FLAG tag.
14. The method of any one of clauses 2 to 13 wherein members of the DNA library comprise an RNA polymerase promoter sequence, an enhancer sequence, and a purification tag sequence.
15. The method of any one of clauses 5 to 13 wherein the purification tag is a C-terminal tag.
16. The method of any one of clauses 1 to 15 wherein the cow antibody scaffold polypeptide comprises the sequence MGCTSVHQETKKYQS(X*)_{c}SYTYNYEHVDVWGCG (SEQ ID NO: 1) wherein (X*)_{c} is a random sequence of amino acids, and wherein X* is an amino acid sequence and c is the number of amino acids in the random sequence of amino acids.
17. The method of clause 16 wherein the cow antibody scaffold polypeptide comprises the sequence MGCTSVHQETKKYQS(X*)_{c}SYTYNYEHVDVWGCGSADYKDDDDK (SEQ ID NO: 2) wherein (X*)_{c} is a random sequence of amino acids, and wherein X* is an amino acid sequence and c is the number of amino acids in the random sequence of amino acids.
18. The method of clause 17 wherein the cow antibody scaffold polypeptide comprises the sequence MGCTSVHQETKKYQS(X*)_{c}SYTYNYEHVDVWGCGSADYKDDDDKKK (SEQ ID NO: 3) wherein (X*)_{c} is a random sequence of amino acids, and wherein X* is an amino acid sequence and c is the number of amino acids in the random sequence of amino acids.
19. The method of any one of clauses 16 to 18 wherein X* comprises a natural amino acid.
20. The method of any one of clauses 16 to 18 wherein X* comprises a non-natural amino acid.
21. The method of any one of clauses 16 to 20 wherein c is an integer from 1 to 40.
22. The method of any one of clauses 16 to 20 wherein c is an integer from 1 to 10.
23. The method of any one of clauses 1 to 22 further comprising the steps of:
   a) synthesizing the peptide of interest, or derivatives thereof, on a first peptide microarray, wherein the derivatives of the peptide of interest include at least one alteration in the sequence of the peptide of interest selected from a single amino acid substitution, a double amino acid substitution, a deletion of one or more amino acids, and an insertion of one or more amino acids, whereby functionalized peptides are generated on the first peptide microarray;
   b) forming from the functionalized peptides, wherein the functionalized peptides are in linear form, cyclic peptides of formula I 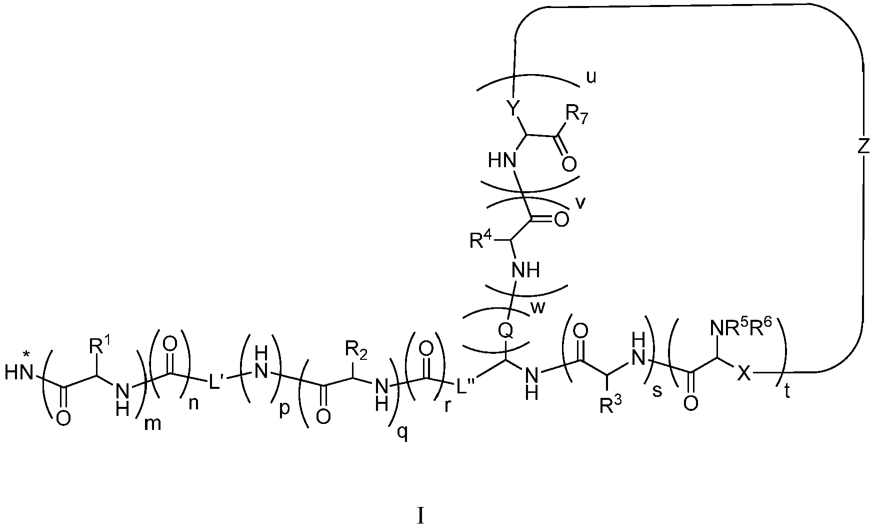
      wherein each R¹, R², R³ and R⁴ is independently a natural amino acid side chain or a non-natural amino acid side chain;
      each R⁵ and R⁶ is independently selected from the group consisting of hydrogen, an N-terminal capping group, and a N-terminal protecting group;
      R⁷ is selected from the group consisting of -OH, a C-terminal capping group, a C-terminal protecting group, and 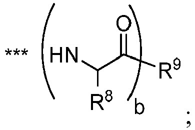
      each R⁸ is independently a natural amino acid side chain or a non-natural amino acid side chain;
      R⁹ is selected from the group consisting of -OH, a C-terminal capping group, and a C-terminal protecting group;
      Q is selected from the group consisting of a bond, a carbonyl, a natural amino acid side chain, and a non-natural amino acid side chain;
      each X and Y is independently selected from the group consisting of a bond, a natural amino acid side chain covalently attached to Z, and a non-natural amino acid side chain covalently attached to Z;
      Z is a group comprising a moiety selected from the group consisting of an amide bond, a disulfide bond, an isopeptide bond, a 1,2,3-triazole, and an optionally substituted 1,2-quinone;
      each L' and L" is independently an optional bivalent linking group or a bond;
      b is an integer from 0 to 50;
      m is an integer from 0 to 6;
      n is 0 or 1;
      p is 0 or 1;
      q is an integer from 0 to 6;
      r is 0 or 1;
      s is an integer from 0 to 100;
      t is 0 or 1;
      u is 0 or 1;
      v is an integer from 0 to 100;
      w is 0 or 1; and * is a point of connection connecting the cyclic peptide to an array support having a reactive surface; and *** is a point of connection to the rest of the functionalized peptide;
      the method comprising the step of reacting a functionalized peptide of formula II under conditions that cause Z to form 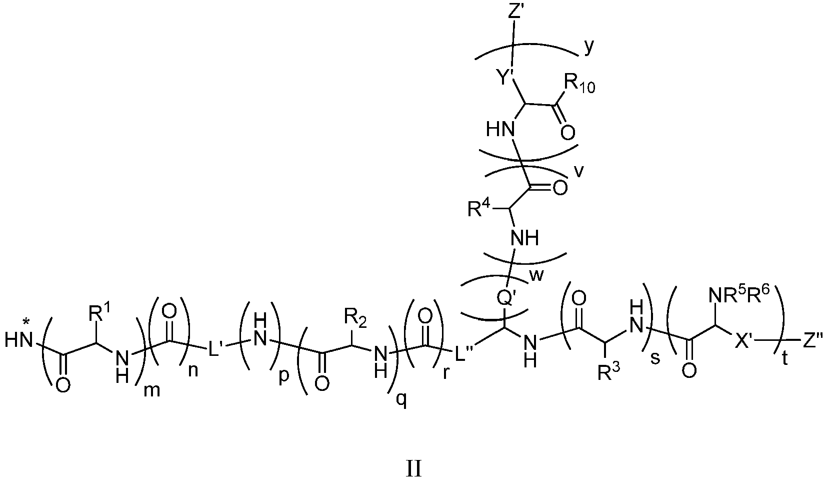
      wherein R¹, R² R³, R⁴, R⁵, R⁶, m, n, p, q, r, s, t, v, w, L', L", *, and *** are as defined for formula I;
      R¹⁰ is selected from the group consisting of -OH, a C-terminal capping group, a C-terminal protecting group, and 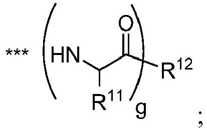
      each R¹¹ is independently a natural amino acid side chain or a non-natural amino acid side chain;
      R¹² is selected from the group consisting of -OH, a C-terminal capping group, and a C-terminal protecting group;
      Q' is selected from the group consisting of a bond, a carbonyl, a natural amino acid side chain covalently attached to Z", and a non-natural amino acid side chain covalently attached to Z";
      X' is selected from the group consisting of a bond, a natural amino acid side chain covalently attached to Z", and a non-natural amino acid side chain covalently attached to Z";
      Y' is selected from the group consisting of a bond, a natural amino acid side chain covalently attached to Z', and a non-natural amino acid side chain covalently attached to Z';
      each Z' and Z" is independently selected from the group consisting of a bond, -OH, hydrogen, a thiol, an amine, a carboxylic acid, an amide, an alkyne, an azide, an optionally substituted aminophenol, a natural amino acid side chain, a non-natural amino acid side chain, an N-terminal protecting group, and a C-terminal protecting group, provided that Z' and Z" are complementary groups that combine to form Z;
      g is an integer from 0 to 50; and
      y is 0 or 1;
      wherein the functionalized peptides and the cyclic peptides are immobilized to the reactive surface;
   c) exposing the cyclic peptides to the target molecule, whereby the target molecule binds to at least one cyclic peptide;
   d) identifying one or more of the cyclic peptides demonstrating strong binding to the target molecule, whereby a matured core binder sequence is determined;
   e) performing at least one of N-terminal and C-terminal extension of the matured core binder sequence determined in step d to provide a matured, extended core binder sequence on a second peptide microarray;
   f) exposing the target molecule to the second peptide microarray comprising a population of matured, extended core binder sequence peptides generated in step e wherein the population of matured, extended core binder sequence peptides comprises cyclic peptides formed as in step b; and
   g) identifying a matured, extended cyclic peptide with strong binding to the target molecule.
24. The method of clause 23, wherein Z comprises a moiety selected from the group consisting of an amide bond, 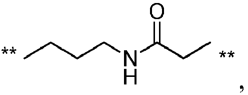 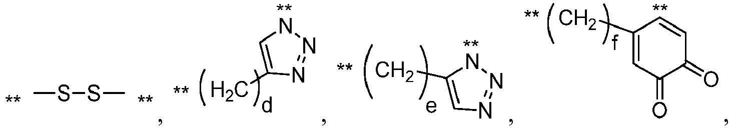 and 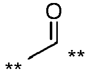 wherein d is an integer from 0 to 6, e is an integer from 0 to 6, and f is an integer from 0 to 6, and ** is a point of connection to the rest of the cyclic peptide.
25. The method of clause 23 or 24, wherein Z comprises a peptide bond, Z" comprises an N-terminal protecting group, t is 0, u is 0, and y is 0.
26. The method of clause 25, further comprising removing Z" from the rest of the functionalized peptide to cause the peptide bond to form.
27. The method of clause 23 or 24, wherein Q and X are bonds to Z, Z comprises ** -S-S- **, X' is a bond to Z", Q' is a bond to Z', Z' and Z" comprise cysteine side chains, t is 1, u is 0, v is 0, w is 1, and y is 0.
28. The method of clause 27, further comprising subjecting the functionalized peptide to oxidative conditions to cause ** -S-S- ** to form.
29. The method of clause 23 or 24, wherein X and Y are bonds to Z, Z comprises ** -S-S- **, X' is a bond to Z", Y' is a bond to Z', Z' and Z" comprise cysteine side chains, t is 1, u is 1, and y is 1.
30. The method of clause 29, further comprising subjecting the functionalized peptide to oxidative conditions to cause ** -S-S- ** to form.
31. The method of clause 23 or 24, wherein Q is a bond to Z, Z comprises 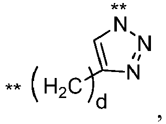 Q' is a bond to Z', Z' comprises 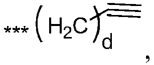 Z" comprises an azide, d is 1, u is 0, v is 0, w is 1, and y is 0.
32. The method of clause 31, further comprising contacting the functionalized peptide with a copper catalyst to cause 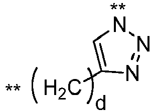 to form.
33. The method of clause 23 or 24, wherein Y is a bond to Z, Z comprises 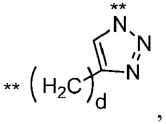 Y' is a bond to Z', Z' comprises 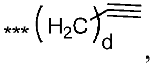 Z" comprises an azide, d is 1, u is 1, and y is 1.
34. The method of clause 33, further comprising contacting the functionalized peptide with a copper catalyst to cause 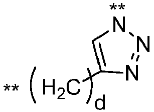 to form.
35. The method of clause 23 or 24, wherein Q is a bond to Z, Z comprises 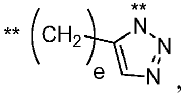 Q' is a bond to Z', Z' comprises 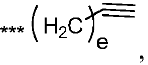 Z" comprises an azide, e is 1, u is 0, v is 0, w is 1, and y is 0.
36. The method of clause 35, further comprising contacting the functionalized peptide with a copper catalyst to cause 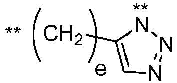 to form.
37. The method of clause 23 or 24, wherein Y is a bond to Z, Z comprises 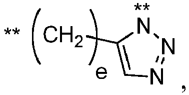 Y' is a bond to Z', Z' comprises 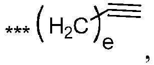 Z" comprises an azide, e is 1, u is 1, and y is 1.
38. The method of clause 37, further comprising contacting the functionalized peptide with a copper catalyst to cause 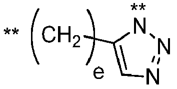 to form.
39. The method of clause 23 or 24, wherein Q is a bond to Z, Z comprises 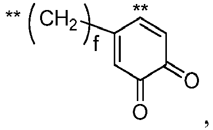 Q' is a bond to Z', Z' comprises 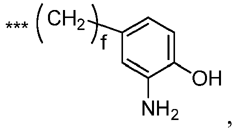 Z" comprises an amine, f is 1, u is 0, v is 0, w is 1, and y is 0.
40. The method of clause 39, further comprising contacting the functionalized peptide with a potassium ferricyanide to cause 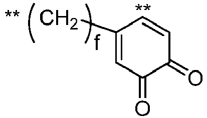 to form.
41. The method of clause 23 or 24, wherein Y is a bond to Z, Z comprises 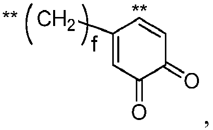 Z" comprises an amine, Y' is a bond to Z', Z' comprises 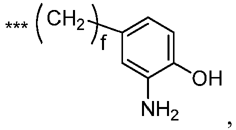 f is 1, u is 1, and y is 1.
42. The method of clause 41, further comprising contacting the functionalized peptide with a potassium ferricyanide to cause 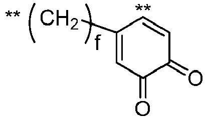 to form.
43. The method of clause 23 or 24, wherein R⁴, R¹⁰, and R¹¹ are defined such that the functionalized peptide comprises a butelase 1 recognition sequence, Y is a bond to Z, Z comprises 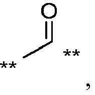 Y' is a bond to Z', Z' is an asparagine or aspartic acid side chain, u is 1, and y is 1.
44. The method of clause 43, further comprising contacting the functionalized peptide with butelase 1 to cause 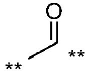 to form.
45. The method of clause 23 or 24, wherein Q and X are bonds to Z, Z comprises 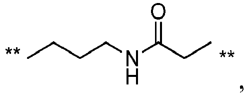 Q' is a bond to Z', X' is a bond to Z", Z' is a glutamine side chain and Z" is a lysine side chain or Z' is a lysine side chain and Z" is a glutamine side chain, t is 1, u is 0, v is 0, w is 1, and y is 0.
46. The method of clause 45, further comprising contacting the functionalized peptide with a microbial transglutaminase to cause 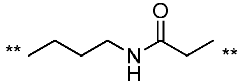 to form.
47. The method of clause 23 or 24, wherein X and Y are bonds to Z, Z comprises 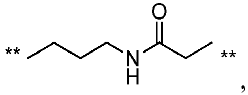 X' is a bond to Z", Y' is a bond to Z', Z' is a glutamine side chain and Z" is a lysine side chain or Z' is a lysine side chain and Z" is a glutamine side chain, t is 1, u is 1, and y is 1.
48. The method of clause 47, further comprising contacting the functionalized peptide with a microbial transglutaminase to cause 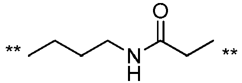 to form.
49. The method of any one of clauses 23 to 48, wherein each L' and L" is independently of the formula V 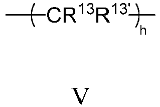 wherein each R¹³ and R^{13'} is independently selected from the group consisting of H, D, halogen, C₁-C₆ alkyl, C₂-C₆ alkenyl, C₂-C₆ alkynyl, C₃-C₆ cycloalkyl, 3- to 7-membered heterocycloalkyl, C₆-C₁₀ aryl, 5- to 7-membered heteroaryl, -OR¹⁴,-OC(O)R¹⁴, -NR¹⁴R^{14'}, -NR¹⁴C(O)R¹⁵, -C(O)R¹⁴, -C(O)OR¹⁴, and -C(O)NR¹⁴R^{14'}, wherein each hydrogen atom in C₁-C₆ alkyl, C₂-C₆ alkenyl, C₂-C₆ alkynyl, C₃-C₆ cycloalkyl, 3- to 7-membered heterocycloalkyl, C₆-C₁₀ aryl and 5- to 7-membered heteroaryl is independently optionally substituted by halogen, C₁-C₆ alkyl, C₂-C₆ alkenyl, C₂-C₆ alkynyl, -OR¹⁶; each R¹⁴, R^{14'}, R¹⁵, and R¹⁶ is independently selected from the group consisting of H, D, hydroxyl, C₁-C₇ alkyl, C₂-C₇ alkenyl, C₂-C₇ alkynyl, C₃-C₆ cycloalkyl, 3- to 7-membered heterocycloalkyl, C₆-C₁₀ aryl and 5- to 7-membered heteroaryl; and h is an integer from 1 to 10; or the formula VI or VII 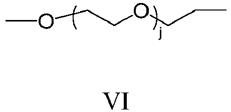 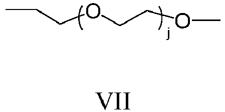 wherein j is an integer from 0 to 30.
50. The method of clause 49, wherein each R¹³ and R^{13'} is hydrogen.
51. The method of clause 49 or 50, wherein L' is present, h is 5, m is 0, n is 1, and p is 1.
52. The method of clause 49, wherein at least one of L' and L" is of the formula VI or VII 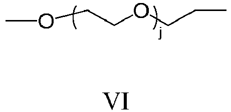 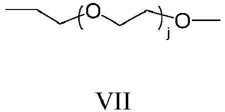 wherein j is 7.
53. The method of any one of clauses 23 to 52, wherein the N-terminal protecting group is a photoprotecting group.
54. The method of any one of clauses 23 to 53, wherein the N-terminal protecting group is 2-(2-nitrophenyl)propyloxycarbonyl.
55. The method of any one of clauses 1 to 22 further comprising the step of synthesizing the cow antibody scaffold polypeptide on one or more peptide microarrays wherein the cow antibody scaffold polypeptide comprises the peptide of interest.
56. The method of clause 55 comprising the steps of:
   a) synthesizing the cow antibody scaffold polypeptide comprising the peptide of interest, or derivatives of the peptide of interest, on a first peptide microarray, wherein the derivatives of the peptide of interest include at least one alteration in the sequence of the peptide of interest selected from a single amino acid substitution, a double amino acid substitution, a deletion of one or more amino acids, and an insertion of one or more amino acids, whereby functionalized peptides are generated on the first peptide microarray;
   b) forming from the functionalized peptides, wherein the functionalized peptides are in linear form, cyclic peptides of formula VIII 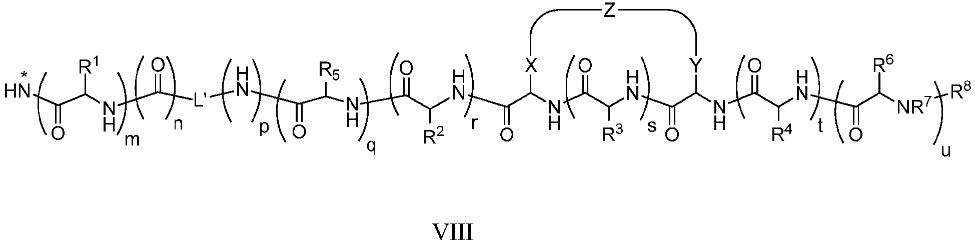
      wherein each R¹, R², R³, and R⁴ is independently a natural amino acid side chain or a non-natural amino acid side chain;
      each R⁵ and R⁶ is independently a natural amino acid side chain or a non-natural amino acid side chain selected such that 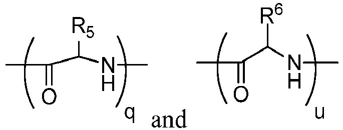 can form a beta-sheet;
      each R⁷ is independently selected from the group consisting of hydrogen, an N-terminal capping group, and an N-terminal protecting group;
      R⁸ is selected from the group consisting of hydrogen, an N-terminal capping group, and a protecting group;
      each X and Y is independently selected from the group consisting of a bond, a natural amino acid side chain covalently attached to Z, and a non-natural amino acid side chain covalently attached to Z;
      Z is a group comprising a moiety selected from the group consisting of an amide bond, a disulfide bond, an isopeptide bond, a 1,2,3-triazole, and an optionally substituted 1,2-quinone;
      L' is an optional bivalent linking group or a bond;
      m is an integer from 0 to 6;
      n is 0 or 1;
      p is 0 or 1;
      q is an integer from 0 to 50;
      r is an integer from 0 to 50;
      s is an integer from 0 to 50;
      t is an integer from 0 to 50;
      u is an integer from 0 to 50; and * is a point of connection connecting the cyclic peptide to an array support having a reactive surface;
      the method comprising the step of reacting a functionalized peptide of formula IX under conditions that cause Z to form 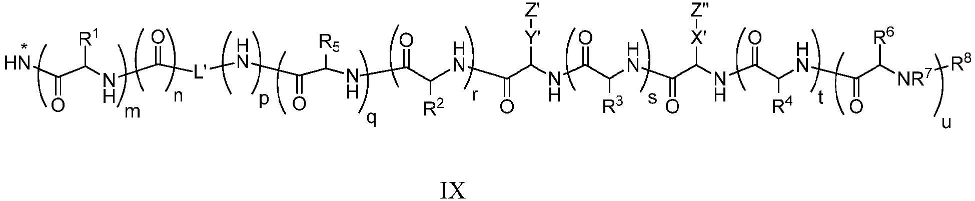
      wherein R¹, R² R³, R⁴, R⁵, R⁶, R⁷, and R⁸, m, n, p, q, r, s, t, u, L' and * are as defined for formula VIII;
      X' is selected from the group consisting of a bond, a natural amino acid side chain covalently attached to Z", and a non-natural amino acid side chain covalently attached to Z";
      Y' is selected from the group consisting of a bond, a natural amino acid side chain covalently attached to Z', and a non-natural amino acid side chain covalently attached to Z'; and
      each Z' and Z" is independently selected from the group consisting of a bond, -OH, hydrogen, a thiol, an amine, a carboxylic acid, an amide, an alkyne, an azide, an optionally substituted aminophenol, a natural amino acid side chain, a non-natural amino acid side chain, an N-terminal protecting group, and a C-terminal protecting group, provided that Z' and Z" are complementary groups that combine to form Z;
      wherein the functionalized peptides and the cyclic peptides are immobilized to the reactive surface;
   c) exposing the cyclic peptides to the target molecule, whereby the target molecule binds to at least one cyclic peptide;
   d) identifying one or more of the cyclic peptides demonstrating strong binding to the target molecule, whereby a matured core binder sequence is determined;
   e) performing at least one of N-terminal and C-terminal extension of the matured core binder sequence determined in step d to provide a matured, extended core binder sequence on a second peptide microarray;
   f) exposing the target molecule to the second peptide microarray comprising a population of matured, extended core binder sequence peptides generated in step e wherein the population of matured, extended core binder sequence peptides comprises cyclic peptides formed as in step b; and
   g) identifying a matured, extended cyclic peptide with strong binding to the target molecule.
57. The method of clause 56, wherein Z comprises a moiety selected from the group consisting of an amide bond, 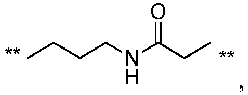 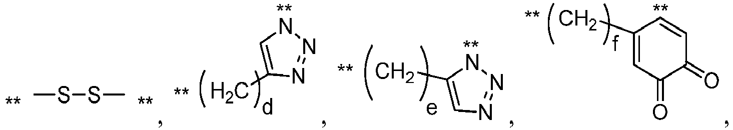 and 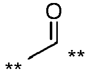 wherein d is an integer from 0 to 6, e is an integer from 0 to 6, and f is an integer from 0 to 6, and ** is a point of connection to the rest of the cyclic peptide.
58. The method of clause 56 or 57, wherein X and Y are bonds to Z, Z comprises ** -S-S- **, X' is a bond to Z", Y' is a bond to Z', and Z' and Z" comprise cysteine side chains.
59. The method of clause 58, further comprising subjecting the functionalized peptide to oxidative conditions to cause ** -S-S- ** to form.
60. The method of claim 56 or 57, wherein Y is a bond to Z, Z comprises 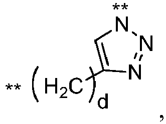 Y' is a bond to Z', Z' comprises 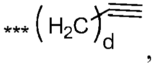 Z" comprises an azide, and d is 1.
61. The method of clause 60, further comprising contacting the functionalized peptide with a copper catalyst to cause 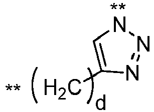 to form.
62. The method of clause 56 or 57, wherein Y is a bond to Z, Z comprises 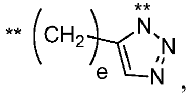 Y' is a bond to Z', Z' comprises 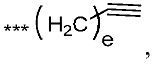 Z" comprises an azide, and e is 1.
63. The method of clause 62, further comprising contacting the functionalized peptide with a copper catalyst to cause 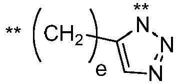 to form.
64. The method of clause 56 or 57, wherein Y is a bond to Z, Z comprises 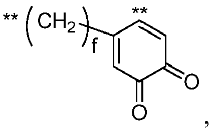 Y' is a bond to Z', Z' comprises 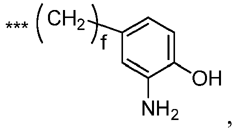 Z" comprises an amine, and f is 1.
65. The method of clause 64, further comprising contacting the functionalized peptide with a potassium ferricyanide to cause 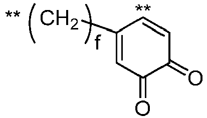 to form.
66. The method of clause 56 or 57, wherein X and Y are bonds to Z, Z comprises 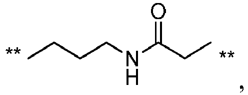 X' is a bond to Z", Y' is a bond to Z', and Z' is a glutamine side chain and Z" is a lysine side chain or Z' is a lysine side chain and Z" is a glutamine side chain.
67. The method of clause 66, further comprising contacting the functionalized peptide with a microbial transglutaminase to cause 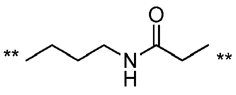 to form.
68. The method of any one of clauses 56 to 67, wherein L' is of the formula (X) 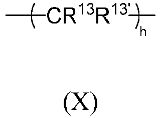 wherein each R¹³ and R^{13'} is independently selected from the group consisting of H, D, halogen, C₁-C₆ alkyl, C₂-C₆ alkenyl, C₂-C₆ alkynyl, C₃-C₆ cycloalkyl, 3- to 7-membered heterocycloalkyl, C₆-C₁₀ aryl, 5- to 7-membered heteroaryl, -OR¹⁴,-OC(O)R¹⁴, -NR¹⁴R^{14'}, -NR¹⁴C(O)R¹⁵, -C(O)R¹⁴, -C(O)OR¹⁴, and -C(O)NR¹⁴R^{14'}, wherein each hydrogen atom in C₁-C₆ alkyl, C₂-C₆ alkenyl, C₂-C₆ alkynyl, C₃-C₆ cycloalkyl, 3- to 7-membered heterocycloalkyl, C₆-C₁₀ aryl and 5- to 7-membered heteroaryl is independently optionally substituted by halogen, C₁-C₆ alkyl, C₂-C₆ alkenyl, C₂-C₆ alkynyl, -OR¹⁶; each R¹⁴, R^{14'}, R¹⁵, and R¹⁶ is independently selected from the group consisting of H, D, hydroxyl, C₁-C₇ alkyl, C₂-C₇ alkenyl, C₂-C₇ alkynyl, C₃-C₆ cycloalkyl, 3- to 7-membered heterocycloalkyl, C₆-C₁₀ aryl and 5- to 7-membered heteroaryl; and h is an integer from 1 to 10; or the formula XI or XII 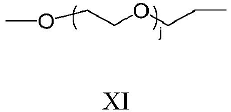 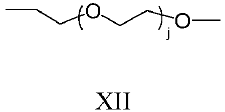 wherein j is an integer from 0 to 30.
69. The method of clause 68, wherein each R¹³ and R^{13'} is hydrogen.
70. The method of clause 68 or 69, wherein L' is present, h is 5, m is 0, n is 1, and p is 1.
71. The method of clause 68, wherein L' is of the formula XI or XII 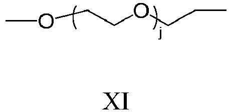 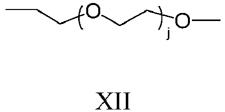 wherein j is 7.
72. The method of any one of clauses 56 to 71, wherein the N-terminal protecting group is a photoprotecting group.
73. The method of any one of clauses 56 to 72, wherein the N-terminal protecting group is 2-(2-nitrophenyl)propyloxycarbonyl.
74. The method of any one of clauses 23 to 54 and 56 to 73, wherein at least one of a label-free and an affinity analysis of the matured, extended core binder sequence peptides is performed.
75. The method of any one of clauses 23 to 54 and 56 to 74, wherein the first or second peptide microarray comprises at least one of glass, plastic, and carbon composite.
76. The method any one of clauses 23 to 54 and 56 to 75, wherein the functionalized peptides and the cyclic peptides on the first or the second peptide microarray comprise the same number of amino acids.
77. The method of any one of clauses 23 to 54 and 56 to 76, wherein the functionalized peptides and the cyclic peptides on the first or the second peptide microarray do not include the amino acid cysteine or methionine, or histidine-proline-glutamine motifs, or amino acid repeats of 2 or more amino acids.
78. The method of any one of clauses 23 to 54 and 56 to 77, wherein the population of matured, extended core binder sequence peptides includes at least one of an N-terminal wobble synthesis oligopeptide and a C-terminal wobble synthesis oligopeptide.
79. The method of any one of clauses 23 to 54 and 56 to 78 wherein the first or second peptide microarray comprises one or more linear peptides and wherein the method further comprises the step of contacting the one or more linear peptides on the first or second peptide microarray with a protease capable of digesting the one or more linear peptides.
80. The method of clause 79 wherein the protease is an amino protease or a mixture of amino proteases.
81. The method of clause 79 wherein the protease is dipeptidyl peptidase IV, aminopeptidase m, or a combination thereof.
82. An mRNA-displayed cow antibody scaffold polypeptide comprising a peptide of interest.
83. The mRNA-displayed cow antibody scaffold polypeptide of clause 82 comprising the sequence MGCTSVHQETKKYQS(X*)_{c}SYTYNYEHVDVWGCG (SEQ ID NO: 1) wherein (X*)_{c} is a random sequence of amino acids, and wherein X* is an amino acid sequence and c is the number of amino acids in the random sequence of amino acids.
84. The mRNA-displayed cow antibody scaffold polypeptide of clause 82 comprising the sequence MGCTSVHQETKKYQS(X*)_{c}SYTYNYEHVDVWGCGSADYKDDDDK (SEQ ID NO: 2) wherein (X*)_{c} is a random sequence of amino acids, and wherein X* is an amino acid sequence and c is the number of amino acids in the random sequence of amino acids.
85. The mRNA-displayed cow antibody scaffold polypeptide of clause 82 comprising the sequence MGCTSVHQETKKYQS(X*)_{c}SYTYNYEHVDVWGCGSADYKDDDDKKK (SEQ ID NO: 3) wherein (X*)_{c} is a random sequence of amino acids, and wherein X* is an amino acid sequence and c is the number of amino acids in the random sequence of amino acids.
86. The mRNA-displayed cow antibody scaffold polypeptide of any one of clauses 83 to 85 wherein X* comprises a natural amino acid.
87. The mRNA-displayed cow antibody scaffold polypeptide of any one of clauses 83 to 85 wherein X* comprises a non-natural amino acid.
88. The mRNA-displayed cow antibody scaffold polypeptide of any one of clauses 83 to 87 wherein c is an integer from 1 to 40.
89. The mRNA-displayed cow antibody scaffold polypeptide of any one of clauses 83 to 87 wherein c is an integer from 1 to 10.
90. The mRNA-displayed cow antibody scaffold polypeptide of any one of clauses 83 to 89 wherein (X*)_{c} comprises the sequence of the peptide of interest.
91. The mRNA-displayed cow antibody scaffold polypeptide of clause 90 wherein the peptide of interest is a therapeutic peptide.
92. A peptide microarray comprising a cow antibody scaffold polypeptide comprising a peptide of interest.
93. The peptide microarray of clause 92 wherein the cow antibody scaffold polypeptide comprises the sequence MGCTSVHQETKKYQS(X*)_{c}SYTYNYEHVDVWGCG (SEQ ID NO: 1) wherein (X*)_{c} is a random sequence of amino acids, and wherein X* is an amino acid sequence and c is the number of amino acids in the random sequence of amino acids.
94. The peptide microarray of clause 92 wherein the cow antibody scaffold polypeptide comprises the sequence MGCTSVHQETKKYQS(X*)_{c}SYTYNYEHVDVWGCGSADYKDDDDK (SEQ ID NO: 2) wherein (X*)_{c} is a random sequence of amino acids, and wherein X* is an amino acid sequence and c is the number of amino acids in the random sequence of amino acids.
95. The peptide microarray of clause 92 wherein the cow antibody scaffold polypeptide comprises the sequence MGCTSVHQETKKYQS(X*)_{c}SYTYNYEHVDVWGCGSADYKDDDDKKK (SEQ ID NO: 3) wherein (X*)_{c} is a random sequence of amino acids, and wherein X* is an amino acid sequence and c is the number of amino acids in the random sequence of amino acids.
96. The peptide microarray of any one of clauses 93 to 95 wherein X* comprises a natural amino acid.
97. The peptide microarray of any one of clauses 93 to 95 wherein X* comprises a non-natural amino acid.
98. The peptide microarray of any one of clauses 93 to 97 wherein c is an integer from 1 to 40.
99. The peptide microarray of any one of clauses 93 to 97 wherein c is an integer from 1 to 10.
100. The peptide microarray of any one of clauses 93 to 99 wherein (X*)_{c} comprises the sequence of the peptide of interest.
101. The peptide microarray of clause 100 wherein the peptide of interest is a therapeutic peptide.
102. The method, mRNA-displayed cow antibody scaffold polypeptide, or peptide microarray of any one of clauses 1 to 101 wherein the cow antibody scaffold polypeptide comprises a random amino acid sequence cassette and the random amino acid sequence cassette comprises the sequence of the peptide of interest.
103. The method, mRNA-displayed cow antibody scaffold polypeptide, or peptide microarray of clause 102 wherein the random amino acid sequence cassette does not include the sequence of an antibody, a fragment of an antibody, or a peptide fragment derived from an antibody.
104. The method of clause 43 or 44 wherein the butelase 1 recognition sequence is NHV.
105. The method of clause 45, 46, 66, or 67 wherein the glutamine side chain is part of the sequence [WY][DE][DE][YW]ALQ[GST]YD (SEQ ID NO:4) and the lysine side chain is part of the sequence RSKLG (SEQ ID NO:5).
106. The method of any one of clauses 1 to 22 further comprising the step of synthesizing the cow antibody scaffold polypeptide comprising the peptide of interest on a peptide microarray to mature and/or extend the peptide of interest or the step of synthesizing the peptide of interest on a peptide microarray to mature and/or extend the peptide of interest.
107. The method, mRNA-displayed cow antibody scaffold polypeptide, or peptide microarray of any one of clauses 1 to 101 wherein the cow antibody scaffold polypeptide comprises a random amino acid sequence cassette wherein the random amino acid sequence cassette comprises the sequence of the peptide of interest and wherein the random amino acid sequence cassette is used for selection of peptides of interest.
108. A cow antibody scaffold polypeptide comprising a random amino acid sequence cassette.

In any of the various embodiments described herein, the following features may be present where applicable, providing additional embodiments of the invention. For all of the embodiments, any applicable combination of embodiments is also contemplated.

### mRNA Display Using Cow Antibody Scaffold Polypeptides

In one embodiment of the methods and compositions described herein, mRNA display can be used as a method for selecting a peptide of interest. In alternative embodiments, other display techniques can be used to select peptides of interest including, but not limited to, phage display, yeast surface display, cDNA display, and ribosome display.

mRNA display is a display and selection technique used for *in vitro* identification of peptides, for example, that have desired properties and can bind to a target molecule of interest with high affinity. mRNA display results in translated peptides that are associated with their template mRNA via a peptidyl acceptor linkage. To achieve such a complex, a peptidyl acceptor linker can be used, such as puromycin, which is a terminal analog of acylated tRNA. For example, puromycin can be linked to the 3'-end of mRNA via a suitable linker, and the linked conjugate is added to an *in vitro* translation reaction to incorporate puromycin to site A of the ribosome, and to form a covalent bond between puromycin and a peptide in the process of elongation. The mRNA-peptide fusion conjugates (e.g., the mRNA-cow antibody scaffold polypeptide fusion conjugates described herein) can be reverse transcribed to DNA (e.g., to form the mRNA-DNA duplexes of the mRNA-cow antibody scaffold polypeptide fusion conjugates described herein). The mRNA-DNA duplexes of the mRNA-cow antibody scaffold polypeptide fusion conjugates can bind to an immobilized target molecule or a target molecule in solution in a selection step, such as affinity chromatography. mRNA-DNA duplexes of the mRNA-cow antibody scaffold polypeptide fusion conjugates that bind strongly to the target molecule can be isolated, and their sequence amplified via PCR. The result is a nucleic acid sequence that encodes a peptide that can bind with high affinity to the target molecule. Using mRNA display, diverse peptide libraries with large numbers of random sequences can be created (e.g., on the order of 10¹³ random sequences or 10¹³ random sequences with ten random amino acids).

Methods for mRNA display are well known in the art and are described in Roberts et al., Proc. Natl. Acd. Sci. USA, 1997, 94: 12297-12302, Nemoto et al., FEBS Lett., 1997, 414: 405-408, Keefe et al., Nature, 2001, 410:715-718, Wilson et al., Proc. Natl. Acad. Sci. USA, 2001, 98: 3750-3755; Cho et al., J Mol Biol., 2000, 297: 309-319, U.S. Pat. Nos. 6,258,558, 6,261,804, 6,214,553, 6,281,344, 6,207,446, and 6,518,018, WO 98/16636, WO 00/34784, WO 01/64942, WO 02/032925 and WO 98/31700,-Recombinant DNA technology methods, including, but not limited to, preparation of DNA libraries, *in vitro* transcription and *in vitro* translation methods, methods of reverse transcription, and regeneration of DNA used in mRNA display are also well-known in the art and are described in Sambrook et al., "Molecular Cloning: A Laboratory Manual", 3rd Edition, Cold Spring Harbor Laboratory Press, (2001).

In one aspect, the *in vitro* transcription and translation may be carried out in a cell-free system such as, for example, a wheat germ extract, a rabbit reticulocyte lysate, an Escherichia coli S30 extract, or a commercially available system (e.g., PURExpress In Vitro Protein Synthesis, New England BioLabs, Inc.; TNT T7 Quick Coupled Transcription/Translation System (Promega, Madison, Wisconsin)).

In one embodiment, the translated peptides in mRNA display are associated with their template mRNA via a peptidyl acceptor linkage. A peptidyl acceptor linkage can refer to any molecule capable of being added to the C-terminus of a growing peptide or protein chain by the catalytic activity of a ribosomal peptidyl transferase. Typically, such linkages contain (i) a nucleotide or nucleotide-like moiety (for example, puromycin and analogues thereof), (ii) an amino acid or amino acid-like moiety (for example, any of the 20 D- or L-amino acids or any amino acid analog thereof (for example, 0-methyl tyrosine or any of the analogs described by Ellman et al., Meth. Enzymol., 202:301 (1991), incorporated herein by reference), and (iii) a linkage between the two (for example, an ester, amide, or ketone linkage at the 3' position or, less preferably, the 2' position).

In some embodiments, the peptidyl acceptor linkage is a tRNA-like structure other than puromycin. Such compounds include, without limitation, any compound which possesses an amino acid linked to an adenine or an adenine-like compound, such as the amino acid nucleotides, phenylalanyl-adenosine (A-Phe), tyrosyl adenosine (A-Tyr), and alanyl adenosine (A-Ala), as well as amide-linked structures, such as phenylalanyl 3' deoxy 3' amino adenosine, alanyl 3' deoxy 3' amino adenosine, and tyrosyl 3' deoxy 3' amino adenosine; in any of these compounds, any of the naturally-occurring L-amino acids or their analogs may be used. In addition, a combined tRNA-like 3' structure-puromycin conjugate may also be used.

In some embodiments, the mRNA-peptide fusion conjugates (e.g., the mRNA-cow antibody scaffold polypeptide fusion conjugates described herein) are purified before binding to the target molecule, for example, by affinity chromatography. In certain embodiments, the mRNA-peptide fusion conjugates conjugates (e.g., the mRNA-cow antibody scaffold polypeptide fusion conjugates described herein) are purified by binding to an antibody specific for an epitope present in the peptide component of the fusion conjugates. The epitope, for example, may be an amino acid sequence tag, for example, FLAG or HA tags, incorporated into the amino acid sequence of the peptide component of the mRNA-peptide fusion conjugates (e.g., the mRNA-cow antibody scaffold polypeptide fusion conjugates described herein), for example, at the N-terminal or C-terminal region.

In some aspects, before binding to the target molecule, the mRNA-cow antibody scaffold polypeptide fusion conjugates can be reverse transcribed. In another embodiment, to bind the mRNA-DNA duplexes of the mRNA-cow antibody scaffold polypeptide fusion conjugates (formed after reverse transcription) to the target molecule of interest, the library of mRNA-DNA duplexes of the mRNA-cow antibody scaffold polypeptide fusion conjugates is incubated with the target molecule. Typically, the target molecule is immobilized on a solid support such as a bead, but the mRNA-DNA duplexes of the mRNA-cow antibody scaffold polypeptide fusion conjugates may be contacted with the target molecule in solution, or a combination of these methods may be used. After the mRNA-DNA duplexes of the mRNA-cow antibody scaffold polypeptide fusion conjugates are incubated with the target molecule, for example, immobilized on a solid support, the mRNA-DNA duplexes of the mRNA-cow antibody scaffold polypeptide fusion conjugates that bind to the target molecule remain associated with the target molecule, and the non-binding molecules are removed by washing. Exemplary solid supports include, for example, an epoxy resin, an agarose column, a SEPHAROSE™ column, or a BIACORE™ chip. In some embodiments, PCR can then be used to amplify the captured DNA (i.e., referred to herein as regenerating the DNA), now enriched for sequences encoding peptides that bind with high affinity to the target molecule. A person skilled in the art can determine what constitutes high affinity binding for any specific peptide-target molecule interaction.

In one illustrative aspect, the enriched population can then be introduced into additional rounds of mRNA display selection. In any of the embodiments described herein, additional DNA and peptide libraries can be made for additional rounds of selection by generating mutants by using, for example, error-prone PCR. These additional libraries can be transcribed and translated *in vitro* following similar steps as for the first round of selection. The additional mRNA-displayed peptides of interest can be subject to additional rounds of screening against the same target molecule to select for peptides of interest with higher affinity than the one(s) selected from the first round of selection. In one aspect, multiple rounds of selection can be performed. In one illustrative embodiment, the DNA encoding a peptide of interest (i.e., a peptide that binds to the target molecule with high affinity) may be sequenced and the peptide may be generated and characterized.

In one embodiment described herein, a method of selecting a peptide of interest is provided. The method comprises the steps of a) preparing a peptide library using an mRNA-displayed cow antibody scaffold polypeptide comprising a peptide of interest to be identified, b) selecting the peptide of interest from the peptide library by contacting a target molecule with the peptide of interest wherein the target molecule is immobilized on a solid support or is in solution, and c) identifying the amino acid sequence of the peptide of interest.

In one illustrative aspect, mRNA display can include the steps of 1) preparing a peptide library by *in vitro* transcription of a DNA library to form an mRNA library, 2) digesting the DNA library with DNase to remove the DNA, conjugating the mRNA from the mRNA library to a linker, such as a puromycin oligonucleotide linker, 3) translating *in vitro* the mRNA from the mRNA library to form mRNA-cow antibody scaffold polypeptide fusion conjugates (e.g., where the cow antibody scaffold polypeptide is linked to the mRNA by a linker, such as a puromycin oligonucleotide linker), 4) purifying the mRNA-cow antibody scaffold polypeptide fusion conjugates using a purification tag that is part of the cow antibody scaffold polypeptide, 5) reverse transcribing the mRNA from the mRNA library to form mRNA-DNA duplexes of the mRNA-cow antibody scaffold polypeptide fusion conjugates, 6) contacting the mRNA-DNA duplexes of the mRNA-cow antibody scaffold polypeptide fusion conjugates with the target molecule to select a peptide of interest, and 7) regenerating the DNA from the mRNA-DNA duplexes of the mRNA-cow antibody scaffold polypeptide fusion conjugates.

In this mRNA display embodiment, the cow antibody scaffold polypeptide can comprise the sequence MGCTSVHQETKKYQS(X*)_{c}SYTYNYEHVDVWGCGSADYKDDDDKKK (SEQ ID NO: 3)
wherein (X*)_{c} is a random sequence of amino acids, and wherein X* is an amino acid sequence and c is the number of amino acids in the random sequence of amino acids. In one embodiment, X* can comprise a natural amino acid or a non-natural amino acid. In another embodiment, c can be an integer from 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, 1 to 20, 1 to 21, 1 to 22, 1 to 23, 1 to 24, 1 to 25, 1 to 26, 1 to 27, 1 to 28, 1 to 29, 1 to 30, 1 to 31, 1 to 32, 1 to 33, 1 to 34, 1 to 35, 1 to 36, 1 to 37, 1 to 38, 1 to 39, or 1 to 40. In one illustrative embodiment, (X*)_{c} can comprise the sequence of the peptide of interest. In another embodiment, the peptide of interest is a therapeutic peptide.

In one embodiment, the cow antibody scaffold polypeptides described herein comprise a random amino acid sequence cassette. The random amino acid sequence cassette includes a random sequence of amino acids for selection of peptides of interest, and is denoted (X*)_{c} in the exemplary embodiment above.

In this mRNA display embodiment, the DNA library can be a cDNA library, *in vitro* transcription can be performed using an RNA polymerase, such as T7 RNA polymerase, the linker, such as a puromycin oligonucleotide linker, can be linked to the mRNA at the 3' end of the mRNA, the purification tag that is part of the cow antibody scaffold polypeptide can be any suitable purification tag, such as a FLAG tag, an AviTag, an HA-tag, a His-tag, or any other suitable purification tag known in the art, and can be at the N-terminus or the C-terminus of the cow antibody scaffold polypeptide, and the members of the DNA library can comprise an RNA polymerase promoter sequence, an enhancer sequence, and a purification tag sequence.

In another embodiment, an mRNA-displayed cow antibody scaffold polypeptide comprising a peptide of interest is provided. In various aspects, the cow antibody scaffold polypeptide can comprise the sequence MGCTSVHQETKKYQS(X*)_{c}SYTYNYEHVDVWGCGSADYKDDDDKKK (SEQ ID NO: 3)
wherein (X*)_{c} is a random sequence of amino acids, and wherein X* is an amino acid sequence and c is the number of amino acids in the random sequence of amino acids. In one embodiment, X* can comprise a natural amino acid or a non-natural amino acid. In another embodiment, c can be an integer from 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, 1 to 20, 1 to 21, 1 to 22, 1 to 23, 1 to 24, 1 to 25, 1 to 26, 1 to 27, 1 to 28, 1 to 29, 1 to 30, 1 to 31, 1 to 32, 1 to 33, 1 to 34, 1 to 35, 1 to 36, 1 to 37, 1 to 38, 1 to 39, or 1 to 40. In one illustrative embodiment, (X*)_{c} can comprise the sequence of the peptide of interest. In another embodiment, the peptide of interest is a therapeutic peptide. In one embodiment, the cow antibody scaffold polypeptides described herein comprise a random amino acid sequence cassette. The random amino acid sequence cassette includes a random sequence of amino acids for selection of peptides of interest, and is denoted (X*)_{c} in the exemplary embodiment above.

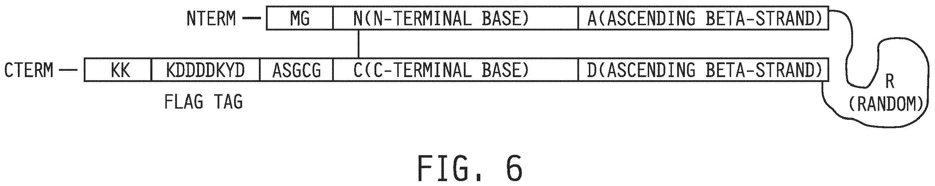

In other embodiments, other cow antibody scaffold polypeptides can be used as follows. In one embodiment, the cow antibody scaffold polypeptide can comprise an N (N-terminal base) motif, an A (ascending beta-strand) motif, an R (random) motif, a D (descending beta-strand) motif, and a C (C-terminal base) motif (see Figure 6 for example). In one embodiment, the N motif may comprise a peptide selected from the group consisting of CTTVHQ (SEQ ID NO:6), CTSVHQ (SEQ ID NO:7), CSSVTQ (SEQ ID NO:8), CSTVHQ (SEQ ID NO:9), CATVRQ (SEQ ID NO:10), CSPVHQ (SEQ ID NO:11), CATVYQ (SEQ ID NO:12), CTAVYQ (SEQ ID NO:13), CTNVHQ (SEQ ID NO:14), CATVHQ (SEQ ID NO:15), CTTVRQ (SEQ ID NO:16), CSTVYQ (SEQ ID NO:17), CTIVHQ (SEQ ID NO:18), CAIVYQ (SEQ ID NO:19), CTTVYQ (SEQ ID NO:20), CTTVFQ (SEQ ID NO:21), CAAVFQ (SEQ ID NO:22), CGTVHQ (SEQ ID NO:23), CASVHQ (SEQ ID NO:24), CTAVFQ (SEQ ID NO:25), CATVFQ (SEQ ID NO:26), CAAAHQ (SEQ ID NO:27), CVVVYQ (SEQ ID NO:28), CGTVFQ (SEQ ID NO:29), CGAVHQ (SEQ ID NO:30), CATKKQ (SEQ ID NO:31), CITVHQ (SEQ ID NO:32), CTIVHQ (SEQ ID NO:33), CITAHQ (SEQ ID NO:34), CVIVHQ (SEQ ID NO:35), CTIVNQ (SEQ ID NO:36), CAAVHQ (SEQ ID NO:37), CGTVYQ (SEQ ID NO:38), CVTVHQ (SEQ ID NO:39), CTTVLQ (SEQ ID NO:40), CTTTHQ (SEQ ID NO:41), and CTTDYQ (SEQ ID NO:42). In another embodiment, the A motif may comprise a peptide selected from the group consisting of ETKKYQS (SEQ ID NO:43), ETRKT (SEQ ID NO:44), RTHVSRS (SEQ ID NO:45), KTTRKTC (SEQ ID NO:46), IF (SEQ ID NO:47), KTRTTQGNT (SEQ ID NO:48), TTLRD (SEQ ID NO:49), KTRTTQGEYLSLMVTLLKDD (SEQ ID NO:50), KTRTTQGNNLSLMVTLLKDD (SEQ ID NO:51), KTRTTQGNT (SEQ ID NO:52), KPGQHKGILVLMVTLLKDD (SEQ ID NO:53), KTRTTQGILVLMVTLLKDD (SEQ ID NO:54), ETKKN (SEQ ID NO:55), EIRKC (SEQ ID NO:56), QTRKC (SEQ ID NO:57), QTRKS (SEQ ID NO:58), KTNQSKN (SEQ ID NO:59), TTHQIHT (SEQ ID NO:60), KTTSIRS (SEQ ID NO:61), KTKKT (SEQ ID NO:62), KTKKL (SEQ ID NO:63), HTNKKR (SEQ ID NO:64), HTNQNR (SEQ ID NO:65), KTNER (SEQ ID NO:66), KTNERC (SEQ ID NO:67), KTNRERC (SEQ ID NO:68), STNKKD (SEQ ID NO:69), ETLIR (SEQ ID NO:70), KTRTT (SEQ ID NO:71), KTNREMS (SEQ ID NO:72), ETKRS (SEQ ID NO:73), RTRQR (SEQ ID NO:74), KTETR (SEQ ID NO:75), KTNKKES (SEQ ID NO:76), KSRKESS (SEQ ID NO:77), ETRTN (SEQ ID NO:78), KTEKH (SEQ ID NO:79), KTKEL (SEQ ID NO:80), HTEPT (SEQ ID NO:81), ETRKS (SEQ ID NO:82), ETRKD (SEQ ID NO:83), ETKKS (SEQ ID NO:84), KTRTTQGNT (SEQ ID NO:85), KTNSQKS (SEQ ID NO:86), QTHKVRD (SEQ ID NO:87), RTGQK (SEQ ID NO:88), KTKQN (SEQ ID NO:89), QTHEKRS (SEQ ID NO:90), QTKRKSG (SEQ ID NO:91), ETKRT (SEQ ID NO:92), ETQKS (SEQ ID NO:93), ETHKR (SEQ ID NO:94), ETHKN (SEQ ID NO:95), QTHATRR (SEQ ID NO:96), RTEGQQS (SEQ ID NO:97), ETKTKSG (SEQ ID NO:98), HTKEIKT (SEQ ID NO:99), ETHQQRG (SEQ ID NO:100), KTEKK (SEQ ID NO:101), RTQKS (SEQ ID NO:102), QTNKR (SEQ ID NO:103), ETQRTS (SEQ ID NO:104), KDKH (SEQ ID NO:105), QTTEKGKT (SEQ ID NO:106), KTDVT (SEQ ID NO:107), ETHTQRT (SEQ ID NO:108), KTEKS (SEQ ID NO:109), KTNQKWG (SEQ ID NO:110), ETRTN (SEQ ID NO:111), KTTTTKS (SEQ ID NO:112), KTEQR (SEQ ID NO:113), MTIKT (SEQ ID NO:114), KTESVRS (SEQ ID NO:115), QTTNR (SEQ ID NO:116), LTKKT (SEQ ID NO:117), KTTQQS (SEQ ID NO:118), HTNKKR (SEQ ID NO:119), QTRKS (SEQ ID NO:120), KTARS (SEQ ID NO:121), IC (SEQ ID NO:122), QTTKR (SEQ ID NO:123), LTRAH (SEQ ID NO:124), RTEKS (SEQ ID NO:125), RTKRS (SEQ ID NO:126), ITHKE (SEQ ID NO:127), HTTTKNT (SEQ ID NO:128), KTLEKT (SEQ ID NO:129), EVQKKT (SEQ ID NO:130), KTQRS (SEQ ID NO:131), ETKTRST (SEQ ID NO:132), RTTTERS (SEQ ID NO:133), KTQRT (SEQ ID NO:134), and KTRTTQGNT (SEQ ID NO:135). In yet another embodiment, the D can comprise a peptide selected from the group consisting of CYTYNYEF (SEQ ID NO:136), HYTYTYDF (SEQ ID NO:137), HYTYTYEW (SEQ ID NO:138), KHRYTYEW (SEQ ID NO:139), NYIYKYSF (SEQ ID NO:140), PYIYTYQF (SEQ ID NO:141), SFTYTYEW (SEQ ID NO:142), SYIYIYQW (SEQ ID NO:143), SYNYTYSW (SEQ ID NO:144), SYSYSYEY (SEQ ID NO:145), SYTYNYDF (SEQ ID NO:146), SYTYNYEW (SEQ ID NO:147), SYTYNYQF (SEQ ID NO:148), SYVWTHNF (SEQ ID NO:149), TYKYVYEW (SEQ ID NO:150), TYTYTYEF (SEQ ID NO:151), TYTYTYEW (SEQ ID NO:152), VFTYTYEF (SEQ ID NO:153), AYTYEW (SEQ ID NO:154), DYIYTY (SEQ ID NO:155), IHSYEF (SEQ ID NO:156), SFTYEF (SEQ ID NO:157), SHSYEF (SEQ ID NO:158), THTYEF (SEQ ID NO:159), TWTYEF (SEQ ID NO:160), TYNYEW (SEQ ID NO:161), TYSYEF (SEQ ID NO:162), TYSYEH (SEQ ID NO:163), TYTYDF (SEQ ID NO:164), TYTYEF (SEQ ID NO:165), TYTYEW (SEQ ID NO:166), AYEF (SEQ ID NO:167), AYSF (SEQ ID NO:168), AYSY (SEQ ID NO:169), CYSF (SEQ ID NO:170), DYTY (SEQ ID NO:171), KYEH (SEQ ID NO:172), KYEW (SEQ ID NO:173), MYEF (SEQ ID NO:174), NWIY (SEQ ID NO:175), NYDY (SEQ ID NO:176), NYQW (SEQ ID NO:177), NYSF (SEQ ID NO:178), PYEW (SEQ ID NO:179), RYNW (SEQ ID NO:180), RYTY (SEQ ID NO:181), SYEF (SEQ ID NO:182), SYEH (SEQ ID NO:183), SYEW (SEQ ID NO:184), SYKW (SEQ ID NO:185), SYTY (SEQ ID NO:186), TYDF (SEQ ID NO:187), TYEF (SEQ ID NO:188), TYEW (SEQ ID NO:189), TYQW (SEQ ID NO:190), TYTY (SEQ ID NO:191), and VYEW (SEQ ID NO:192). In still another illustrative embodiment, the C motif can comprise a peptide selected from the group consisting of YVDAW (SEQ ID NO:193), HVDVW (SEQ ID NO:194), HVDAW (SEQ ID NO:195), GVDAW (SEQ ID NO:196), YIDAW (SEQ ID NO:197), HVDSW (SEQ ID NO:198), HIDAW (SEQ ID NO:199), YVDTW (SEQ ID NO:200), NVDAW (SEQ ID NO:201), HVNAW (SEQ ID NO:202), YVTAW (SEQ ID NO:203), YITAW (SEQ ID NO:204), YVEAW (SEQ ID NO:205), HVDEW (SEQ ID NO:206), HVDTW (SEQ ID NO:207), YADAW (SEQ ID NO:208), YGDAW (SEQ ID NO:209), HADAW (SEQ ID NO:210), YVEAW (SEQ ID NO:211), NVDSW (SEQ ID NO:212), NVDAW (SEQ ID NO:213), GVDAW (SEQ ID NO:214), NIDAW (SEQ ID NO:215), RIDVM (SEQ ID NO:216), YVETW (SEQ ID NO:217), YVNAW (SEQ ID NO:218), HADVW (SEQ ID NO:219), HVESW (SEQ ID NO:220), HVETW (SEQ ID NO:221), NVEAW (SEQ ID NO:222), YVDSW (SEQ ID NO:223), and RVDTW (SEQ ID NO:224). In another aspect, the R motif can be a random sequence of amino acids (X*)_{c}, wherein X* is an amino acid sequence and c is the number of amino acids in the random sequence of amino acids.

In the various embodiments described herein, the target molecule may be any molecule, including, but not limited to, a biomacromolecule such as a protein, a peptide, a nucleic acid (e.g., DNA or RNA), a polycarbohydrate, or a small molecule such as an organic compound or an organometallic complex, or any other molecule that contributes to a disease, such as the diseases listed below (e.g., a receptor for the therapeutic peptide, an enzyme inhibited or activated by the therapeutic peptide, or any other molecule wherein the activity of the molecule is altered by the therapeutic peptide). In one embodiment, the target molecule can be a molecule involved in a disease state and the peptide of interest or the cyclic peptide can be a therapeutic peptide.

In the embodiment where the peptide of interest or the cyclic peptide is a therapeutic peptide, the disease that is treated can be selected from the group consisting of cancer, an infectious disease, heart disease (e.g., atherosclerosis) and other cholesterol-related diseases, stroke, wounds, pain, an inflammatory disease, such as arthritis (e.g., rheumatoid arthritis), inflammatory bowel disease, psoriasis, diabetes mellitis, or an autoimmune disease, a respiratory disease, such as asthma or chronic obstructive pulmonary disease, diarrheal diseases, a genetic disease, a neurological disorder, such as Alzheimer's disease, muscular dystrophy, or Parkinson's disease, a mental disorder, or any other type of disease capable of being treated with a therapeutic peptide (e.g., a cyclic peptide).

In other embodiments, the disease can be a cancer selected from the group consisting of a carcinoma, a sarcoma, a lymphoma, a melanoma, a mesothelioma, a nasopharyngeal carcinoma, a leukemia, an adenocarcinoma, and a myeloma. In yet other embodiments, the disease can be a cancer selected from the group consisting of lung cancer, bone cancer, pancreatic cancer, skin cancer, cancer of the head or neck, melanoma, uterine cancer, ovarian cancer, endometrial cancer, rectal cancer, stomach cancer, colon cancer, breast cancer, cancer of the cervix, Hodgkin's Disease, cancer of the esophagus, non-small cell lung cancer, prostate cancer, leukemia, lymphoma, mesothelioma, cancer of the bladder, Burkitt's lymphoma, kidney cancer, and brain cancer, or any other type of cancer that can be treated with a therapeutic peptide (e.g., a cyclic peptide).

### Peptide Microarrays and Their Use

In one embodiment, a peptide microarray is provided comprising a cow antibody scaffold polypeptide comprising a peptide of interest. In another embodiment, a peptide microarray is provided comprising the peptide of interest identified using the cow antibody scaffold polypeptide.

In various aspects, the cow antibody scaffold polypeptide can comprise the sequence MGCTSVHQETKKYQS(X*)_{c}SYTYNYEHVDVWGCG (SEQ ID NO: 1), MGCTSVHQETKKYQS(X*)_{c}SYTYNYEHVDVWGCGSADYKDDDDK (SEQ ID NO: 2), or MGCTSVHQETKKYQS(X*)_{c}SYTYNYEHVDVWGCGSADYKDDDDKKK (SEQ ID NO: 3) wherein (X*)_{c} is a random sequence of amino acids, and wherein X* is an amino acid sequence and c is the number of amino acids in the random sequence of amino acids. In one embodiment, X* can comprise a natural amino acid or a non-natural amino acid. In another embodiment, c can be an integer from 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, 1 to 20, 1 to 21, 1 to 22, 1 to 23, 1 to 24, 1 to 25, 1 to 26, 1 to 27, 1 to 28, 1 to 29, 1 to 30, 1 to 31, 1 to 32, 1 to 33, 1 to 34, 1 to 35, 1 to 36, 1 to 37, 1 to 38, 1 to 39, or 1 to 40. In one illustrative embodiment, (X*)_{c} can comprise the sequence of the peptide of interest. In another embodiment, the peptide of interest is a therapeutic peptide.

In one embodiment, the cow antibody scaffold polypeptides described herein comprise a random amino acid sequence cassette. The random amino acid sequence cassette includes a random sequence of amino acids for selection of peptides of interest, and is denoted (X*)_{c} in the exemplary embodiment above.

In these embodiments, the term "natural amino acid" refers to one of the 20 amino acids typically found in proteins and used for protein biosynthesis as well as other amino acids which can be incorporated into proteins during translation (including pyrrolysine and selenocysteine). The 20 natural amino acids include histidine, alanine, valine, glycine, leucine, isoleucine, aspartic acid, glutamic acid, serine, glutamine, asparagine, threonine, arginine, proline, phenylalanine, tyrosine, tryptophan, cysteine, methionine and lysine.

In these embodiments, the term "non-natural amino acid" refers to an organic compound that is not among those encoded by the standard genetic code, or incorporated into proteins during translation. Therefore, non-natural amino acids include, but are not limited to, amino acids or analogs of amino acids, the D-isostereomers of amino acids, the beta-amino-analogs of amino acids, citrulline, homocitrulline, homoarginine, hydroxyproline, homoproline, ornithine, 4-amino-phenylalanine, cyclohexylalanine, α-aminoisobutyric acid, N-methylalanine, N-methyl-glycine, norleucine, N-methyl-glutamic acid, tert-butylglycine, α-aminobutyric acid, tert-butylalanine, 2-aminoisobutyric acid, α-ammoisobutyric acid, 2-aminoindane-2-carboxylic acid, selenomethionine, dehydroalanine, lanthionine, γ-amino butyric acid, and derivatives thereof wherein the amine nitrogen has been mono- or di-alkylated.

In the embodiment where the peptide of interest or the cyclic peptide is a therapeutic peptide, the disease that is treated can be selected from the group consisting of cancer, an infectious disease, heart disease (e.g., atherosclerosis) and other cholesterol-related diseases, stroke, wounds, pain, an inflammatory disease, such as arthritis (e.g., rheumatoid arthritis), inflammatory bowel disease, psoriasis, diabetes mellitis, or an autoimmune disease, a respiratory disease, such as asthma or chronic obstructive pulmonary disease, diarrheal diseases, a genetic disease, a neurological disorder, such as Alzheimer's disease, muscular dystrophy, or Parkinson's disease, a mental disorder, or any other type of disease capable of being treated with a therapeutic peptide (e.g., a cyclic peptide).

In other embodiments, the disease can be a cancer selected from the group consisting of a carcinoma, a sarcoma, a lymphoma, a melanoma, a mesothelioma, a nasopharyngeal carcinoma, a leukemia, an adenocarcinoma, and a myeloma. In yet other embodiments, the disease can be a cancer selected from the group consisting of lung cancer, bone cancer, pancreatic cancer, skin cancer, cancer of the head or neck, melanoma, uterine cancer, ovarian cancer, endometrial cancer, rectal cancer, stomach cancer, colon cancer, breast cancer, cancer of the cervix, Hodgkin's Disease, cancer of the esophagus, non-small cell lung cancer, prostate cancer, leukemia, lymphoma, mesothelioma, cancer of the bladder, Burkitt's lymphoma, kidney cancer, and brain cancer, or any other type of cancer that can be treated with a therapeutic peptide (e.g., a cyclic peptide).

In the embodiment where the peptide of interest or the cyclic peptide is a therapeutic peptide, the target molecule can be, for example, a protein or other molecule that contributes to a disease, such as the diseases listed above (e.g., a receptor for the therapeutic peptide, an enzyme inhibited or activated by the therapeutic peptide, or any other molecule wherein the activity of the molecule is altered by the therapeutic peptide).

In another embodiment, mRNA display as described herein above using the cow antibody scaffold polypeptide to select a peptide of interest is followed by synthesizing the cow antibody scaffold polypeptide comprising the peptide of interest on one or more peptide microarrays or synthesizing the peptide of interest, identified using the cow antibody scaffold polypeptide, on one or more peptide microarrays. In various embodiments, this method can comprise any of the methods of clauses 56 to 73 described above in this Detailed Description section of the application.

In yet another embodiment, the peptide of interest, identified using mRNA display, is then synthesized on a peptide microarray and is matured and/or extended and is cyclized *in situ* on the peptide microarray...

In one illustrative embodiment, the maturation/extension/cyclization method comprises the steps of:
a) synthesizing the peptide of interest, or derivatives thereof, on a first peptide microarray, wherein the derivatives of the peptide of interest include at least one alteration in the sequence of the peptide of interest selected from a single amino acid substitution, a double amino acid substitution, a deletion of one or more amino acids, and an insertion of one or more amino acids, whereby functionalized peptides are generated on the first peptide microarray;
b) forming from the functionalized peptides, wherein the functionalized peptides are in linear form, cyclic peptides of formula I 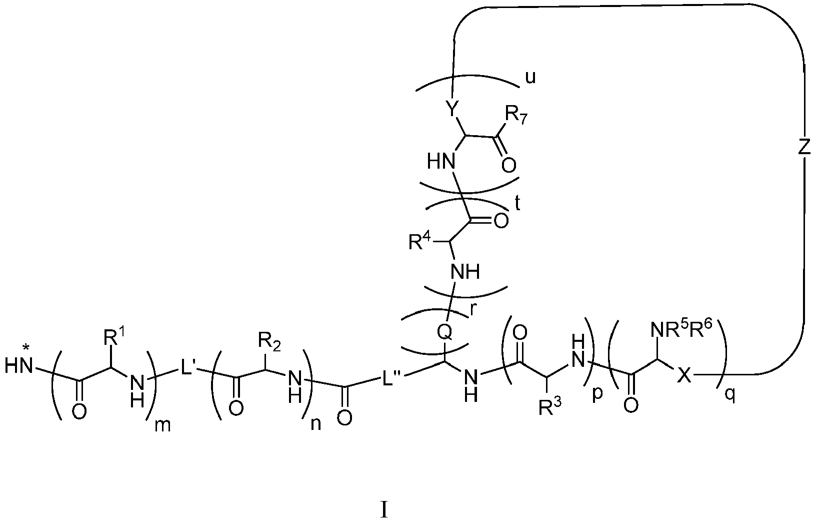
   wherein each R¹, R², R³ and R⁴ is independently a natural amino acid side chain or a non-natural amino acid side chain;
   each R⁵ and R⁶ is independently hydrogen or an N-terminal capping group;
   each R⁷ is independently -OH or a C-terminal capping group;
   Q is selected from the group consisting of a carbonyl, a natural amino acid side chain, and a non-natural amino acid side chain;
   each X and Y is independently selected from the group consisting of a bond, a natural amino acid side chain covalently attached to Z, and a non-natural amino acid side chain covalently attached to Z;
   Z is a group comprising a moiety selected from the group consisting of an amide bond, a disulfide bond, an isopeptide bond, a 1,2,3-triazole, and an optionally substituted 1,2-quinone;
   L' and L" are each independently an optional bivalent linking group or a bond;
   m is an integer from 0 to 6;
   n is an integer from 0 to 6;
   p is an integer from 0 to 100;
   q is 0 or 1;
   r is 0 or 1;
   t is an integer from 0 to 100;
   u is 0 or 1; and * is a point of connection connecting the cyclic peptide to an array support having a reactive surface;
   the method comprising the step of reacting a functionalized peptide of formula II under conditions that cause Z to form 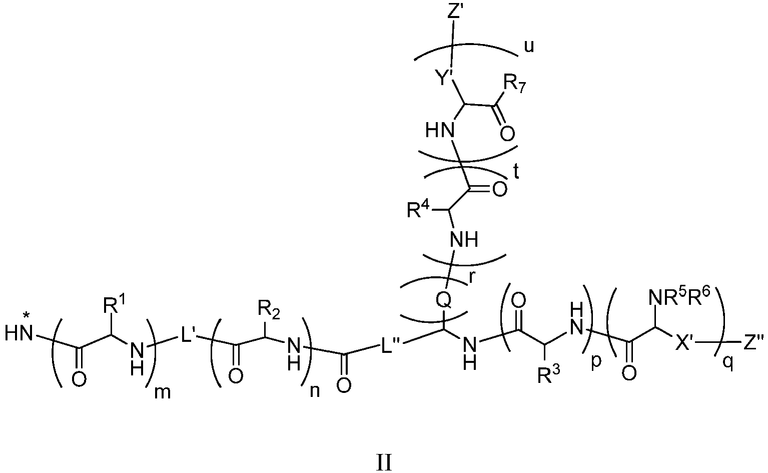
   wherein R¹, R² R³, R⁴, R⁵, R⁶, Q, m, n, p, q, r, t, u, and * are as defined for formula I;
   each R⁷ is independently selected from the group consisting of -OH, a C-terminal capping group, and 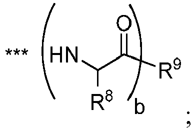
   each R⁸ is independently a natural amino acid side chain or a non-natural amino acid side chain;
   each R⁹ is independently -OH or a C-terminal capping group;
   each X' is independently selected from the group consisting of a bond, a natural amino acid side chain covalently attached to Z", and a non-natural amino acid side chain covalently attached to Z";
   each Y' is independently selected from the group consisting of a bond, a natural amino acid side chain covalently attached to Z', and a non-natural amino acid side chain covalently attached to Z';
   Z' and Z" are each independently selected from the group consisting of a bond, -OH, hydrogen, a thiol, an amine, a carboxylic acid, an amide, an alkyne, an azide, an optionally substituted aminophenol, a natural amino acid side chain, a non-natural amino acid side chain, an N-terminal protecting group, and a C-terminal protecting group, provided that Z' and Z" are complementary groups that combine to form Z;
   b is an integer from 0 to 50;
   and *** is a point of connection to the rest of the functionalized peptide;
   wherein the functionalized peptides and the cyclic peptides are immobilized to the reactive surface;
c) exposing the cyclic peptides to the target molecule, whereby the target molecule binds to at least one cyclic peptide;
d) identifying one or more of the cyclic peptides demonstrating strong binding to the target molecule, whereby a matured core binder sequence is determined;
e) performing at least one of N-terminal and C-terminal extension of the matured core binder sequence determined in step d to provide a matured, extended core binder sequence on a second peptide microarray;
f) exposing the target molecule to the second peptide microarray comprising a population of matured, extended core binder sequence peptides generated in step e wherein the population of matured, extended core binder sequence peptides comprises cyclic peptides formed as in step b; and
g) identifying a matured, extended cyclic peptide with strong binding to the target molecule.

In one aspect, Z comprises a moiety selected from the group consisting of an amide bond, 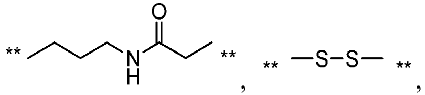 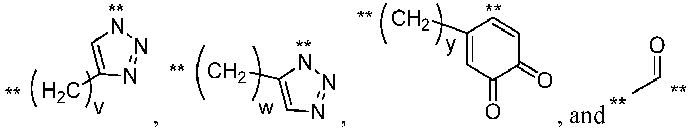 wherein v is an integer from 0 to 6, w is an integer from 0 to 6, and y is an integer from 0 to 6, and ** is a point of connection to the rest of the cyclic peptide. In another aspect, Z comprises a peptide bond, Z" comprises an N-terminal protecting group, Q is a carbonyl, q is 0, r is 1 and u is 0.

In another embodiment, the method further comprises removing Z" from the rest of the functionalized peptide to cause the peptide bond to form.

In yet another embodiment, X and Y are bonds to Z, Z comprises **-S-S-**, X' is a bond to Z", Y' is a bond to Z', Z' and Z" comprise cysteine side chains, q is 1, and u is 1. In another aspect, the method further comprises subjecting the functionalized peptide to oxidative conditions to cause **-S-S-** to form.

In still another embodiment, Y is a bond to Z, Z comprises 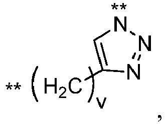 Y' is a bond to Z', Z' comprises 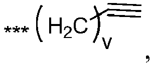 Z" comprises an azide, u is 1, and v is 1. In this embodiment, the method can further comprise contacting the functionalized peptide with a copper catalyst to cause 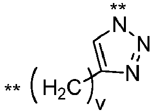 to form.

In another illustrative aspect, Y is a bond to Z, Z comprises 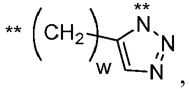 Y' is a bond to Z', Z' comprises 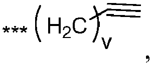 Z" comprises an azide, u is 1, and v is 1. In this aspect, the method can further comprise contacting the functionalized peptide with a copper catalyst to cause 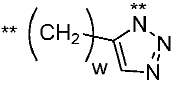 to form.

In another embodiment, Z comprises 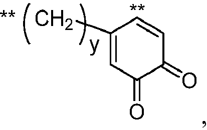 Y' is a bond to Z', Z' comprises 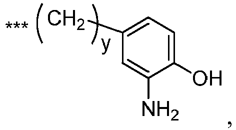 r is 0, u is 1, and y is 1. In this embodiment, the method can further comprise contacting the functionalized peptide with a potassium ferricyanide to cause 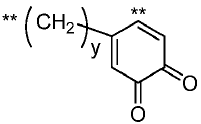 to form.

In still another embodiment, R³ and R⁸ are defined such that the functionalized peptide comprises a butelase 1 recognition sequence, Y is a bond to Z, Z comprises 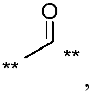 Y' is a bond to Z', Z' is an asparagine or aspartic acid side chain, q is 0, and u is 1. In this embodiment, the method can further comprise contacting the functionalized peptide with butelase 1 to cause 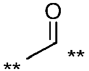 to form.

In another aspect, X and Y are bonds to Z, Z comprises 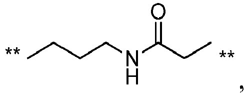 X' is a bond to Z", Y' is a bond to Z', Z' is a glutamine side chain and Z" is a lysine side chain or Z' is a lysine side chain and Z" is a glutamine side chain, q is 1, and u is 1. In this aspect, the method can further comprise contacting the functionalized peptide with a microbial transglutaminase to cause 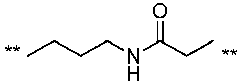 to form.

In other illustrative embodiments of this *in situ* cyclization method, 1) at least one of a label-free and an affinity analysis of the matured, extended core binder sequence peptide can be performed, 2) the functionalized peptides and the cyclic peptides on the first or the second peptide microarray can comprise the same number of amino acids, 3) the functionalized peptides and the cyclic peptides on the first or the second peptide microarray do not include the amino acid cysteine or methionine, or histidine-proline-glutamine motifs, or amino acid repeats of 2 or more amino acids, and/or 4) the population of matured, extended core binder sequence peptides can include at least one of an N-terminal wobble synthesis oligopeptide and a C-terminal wobble synthesis oligopeptide.

In one embodiment for the derivatives of peptides of interest described in step a) above, the peptide of interest can be modified by a single amino acid substitution, a double amino acid substitution, a deletion of one or more amino acids, or an insertion of one or more amino acids, whereby functionalized peptides are generated on the first peptide microarray. In this embodiment, the amino acids in the peptides of interest can be substituted with any of the 19 other natural amino acids or with any suitable non-natural amino acid. In another illustrative embodiment, the peptides of interest or cyclic peptides described herein can comprise natural or non-natural amino acids, or a combination thereof.

In one embodiment, a further step can be performed to increase the yield of cyclic peptides on the peptide microarray. In this aspect, the first or second peptide microarray comprises one or more linear peptides, along with cyclic peptides due to the inefficiency of cyclization. Thus, in this aspect, the cyclization method can further comprise the step of contacting the one or more linear peptides on the first or second peptide microarray with a protease capable of digesting the one or more linear peptides. In this embodiment, the steps of the maturation/extension/cyclization method described above can then be repeated to increase the yield of cyclic peptides on the peptide microarray. In one illustrative embodiment, the protease can be an aminoprotease, such as aminopeptidase m, cystinyl aminopeptidase, glutamyl aminopeptidase, leucyl aminopeptidase, or pyroglutamyl peptidase, or a mixture of aminoproteases. In another illustrative aspect, the protease can be a dipeptidase, such as dipeptidyl peptidase IV, a carboxypeptidase, a tripeptidylpeptidase, a metalloexopeptidase, or a combination thereof.

In one embodiment of the maturation/extension/cyclization method described above, an isopeptide bond can be formed to cyclize peptides on a peptide microarray. In one aspect, the amino acids that can be linked can be a glutamine residue and a lysine residue in the same peptide, and the linkage can be formed using a transglutaminase.

In this embodiment, the glutamine-containing portion of the peptide can comprise a sequence motif of GDYALQGPG (SEQ ID NO:225). In the embodiment where the sequence motif is GDYALQGPG (SEQ ID NO:225), the glutamine-containing portion of the peptide can comprise a sequence selected from the group consisting of CGGDYALQGPG (SEQ ID NO:226), WGGDYALQGPG (SEQ ID NO:227), YGGDYALQGPG (SEQ ID NO:228), DGGDYALQGPG (SEQ ID NO:229), GDGDYALQGPG (SEQ ID NO:230), NGGDYALQGPG (SEQ ID NO:231), GCGDYALQGPG (SEQ ID NO:232), EGGDYALQGPG (SEQ ID NO:233), PGGDYALQGPG (SEQ ID NO:234), TGGDYALQGPG (SEQ ID NO:235), QGGDYALQGPG (SEQ ID NO:236), IGGDYALQGPG (SEQ ID NO:237), FGGDYALQGPG (SEQ ID NO:238), HGGDYALQGPG (SEQ ID NO:239), LGGDYALQGPG (SEQ ID NO:240), VGGDYALQGPG (SEQ ID NO:241), RGGDYALQGPG (SEQ ID NO:242), GWGDYALQGPG (SEQ ID NO:243), MGGDYALQGPG (SEQ ID NO:244), SGGDYALQGPG (SEQ ID NO:245), AGGDYALQGPG (SEQ ID NO:246), GYGDYALQGPG (SEQ ID NO:247), GEGDYALQGPG (SEQ ID NO:248), GPGDYALQGPG (SEQ ID NO:249), GHGDYALQGPG (SEQ ID NO:250), and GNGDYALQGPG (SEQ ID NO:251), or a combination thereof. In another embodiment, the glutamine-containing portion of the peptide can comprise the sequence DYALQ (SEQ ID NO:252).

In another embodiment, the glutamine-containing portion of the peptide can comprise a sequence selected from the group consisting of GGGDYALQGGG (SEQ ID NO:253), WDGDYALQGGG (SEQ ID NO:254), GGGGDYALQGGGG (SEQ ID NO:255), and GGGDYALQGGGG (SEQ ID NO:256), or a combination thereof. In another embodiment, the glutamine-containing portion of the peptide can comprise the sequence GGGDYALQGGG (SEQ ID NO:253).

In yet another embodiment, the glutamine-containing portion of the peptide can comprise a sequence motif of [YF][VA]LQG (SEQ ID NO:257). In this embodiment, the glutamine-containing portion of the peptide can comprise a sequence selected from the group consisting of DYALQ (SEQ ID NO:252), DYVLQ (SEQ ID NO:258), NYALQ (SEQ ID NO:259), EYALQ (SEQ ID NO:260), PYALQ (SEQ ID NO:261), EYVLQ (SEQ ID NO:262), DFALQ (SEQ ID NO:263), FYALQ (SEQ ID NO:264), NYVLQ (SEQ ID NO:265), RYALQ (SEQ ID NO:266), YFALQ (SEQ ID NO:267), PYVLQ (SEQ ID NO:268), WYALQ (SEQ ID NO:269), SYALQ (SEQ ID NO:270), HYALQ (SEQ ID NO:271), EFALQ (SEQ ID NO:272), and NFVLQ (SEQ ID NO:273), or a combination thereof.

In still another illustrative aspect, the glutamine-containing portion of the peptide can comprise a sequence selected from the group consisting of DYFLQ (SEQ ID NO:274), EYVAQ (SEQ ID NO:275), DYVAQ (SEQ ID NO:276), DFYLQ (SEQ ID NO:277), EYFLQ (SEQ ID NO:278), or a combination thereof.

In yet another embodiment, the peptide can contain a lysine and the lysine-containing portion of the peptide can comprise a sequence motif of SK[LS]K (SEQ ID NO:279) or [KR][ST]KL (SEQ ID NO:280). In this embodiment, the lysine-containing portion of the peptide can comprise a sequence selected from the group consisting of ARSKL (SEQ ID NO:281), KSKLA (SEQ ID NO:282), TKSKL (SEQ ID NO:283), KLSKL (SEQ ID NO:284), RSKLG (SEQ ID NO:285), RGSKL (SEQ ID NO:286), RSKSK (SEQ ID NO:287), SKSKL (SEQ ID NO:288), PKTKL (SEQ ID NO:289), RSKLA (SEQ ID NO:290), GRSKL (SEQ ID NO:291), SKLSK (SEQ ID NO:292), FTKSK (SEQ ID NO:293), RLKSK (SEQ ID NO:294), KLGAK (SEQ ID NO:295), QRSKL (SEQ ID NO:296), LSKLK (SEQ ID NO:297), NRTKL (SEQ ID NO:298), QRTKL (SEQ ID NO:299), GGGRSKLAGGG (SEQ ID NO:300), and GGGARSKLGGGG (SEQ ID NO:301), or a combination thereof.

In another illustrative embodiment, the peptide can contain a lysine and the lysine-containing portion of the peptide can comprise a sequence selected from the group consisting of RGTKL (SEQ ID NO:302), FPKLK (SEQ ID NO:303), KLKYK (SEQ ID NO:304), RAKYK (SEQ ID NO:305), KTKYK (SEQ ID NO:306), and GYKLK (SEQ ID NO:307), or a combination thereof.

In still another embodiment, the peptide can comprise a transglutaminase glutamine substrate peptide and a transglutaminase lysine substrate peptide. In yet another embodiment, the transglutaminase glutamine and/or lysine substrate peptide can comprise a sequence of DYALQ (SEQ ID NO:252) or can have a sequence motif comprising [FY][FYT]LQ (SEQ ID NO:308), [YF]VAQ (SEQ ID NO:309), K[YLS]K (SEQ ID NO:310), or TKL (SEQ ID NO:311).

In another embodiment, transglutaminase substrate peptides are contemplated having about 60%, about 70%, about 75%, about 80%, about 85%, about 90%, about 95%, about 96%, about 97%, about 98%, or about 99% homology with any of SEQ ID NOS: 4 to 85. Determination of percent identity or similarity between sequences can be done, for example, by using the GAP program (Genetics Computer Group, software; available via Accelrys on http://www.accelrys.com), and alignments can be done using, for example, the ClustalW algorithm (VNTI software, InforMax Inc.). A sequence database can be searched using the peptide sequence to be compared. Algorithms for database searching are typically based on the BLAST software (Altschul et al., 1990).

In one illustrative embodiment, linking a transglutaminase glutamine substrate peptide and a transglutaminase lysine substrate peptide to form an isopeptide bond that results in cyclization of the peptide can be performed using a transglutaminase. In another embodiment, a microbial transglutaminase (e.g., a *Streptoverticillium sp.* transglutaminase) or a mammalian transglutaminase can be used. In the embodiment where the enzyme is a mammalian transglutaminase, the mammalian transglutaminase can be, for example, selected from the group consisting of Human Factor XIII A transglutaminase, Human Factor XIII B transglutaminase, a Factor XIII transglutaminase, a keratinocyte transglutaminase, a tissue-type transglutaminase, an epidermal transglutaminase, a prostate transglutaminase, a neuronal transglutaminase, a human transglutaminase 5, and a human transglutaminase 7.

In various illustrative aspects, the cyclic peptides or peptides of interest that make up the peptide microarrays described herein can be peptides of about 5 to about 19 amino acids, about 5 to about 18 amino acids, about 5 to about 17 amino acids, about 5 to about 16 amino acids, about 5 to about 15 amino acids, about 5 to about 14 amino acids, about 5 to about 13 amino acids, about 5 to about 12 amino acids, about 5 to about 11 amino acids, about 5 to about 10 amino acids, about 5 to about 9, about 5 to about 8, about 5 to about 7, or about 5 to about 6 amino acids. In other illustrative aspects, the cyclic peptides or peptides of interest described herein can be peptides of 5 to 19 amino acids, 5 to 18 amino acids, 5 to 17 amino acids, 5 to 16 amino acids, 5 to 15 amino acids, 5 to 14 amino acids, 5 to 13 amino acids, 5 to 12 amino acids, 5 to 11 amino acids, 5 to 10 amino acids, 5 to 9 amino acids, 5 to 8 amino acids, 5 to 7 amino acids, or 5 to 6 amino acids. In yet another illustrative embodiment, the cyclic peptides or peptides of interest can be selected from the group consisting of 5-mers, 6-mers, 7-mers, 8-mers, 9-mers, 10-mers, 11-mers, 12-mers, 13-mers, 14-mers, 15-mers, 16-mers, 17-mers, 18-mers, or 19-mers, or a combination thereof.

In various embodiments, the peptide microarrays described herein can have at least 1.6 x 10⁵ peptides, at least 2.0 x 10⁵ peptides, at least 3.0 x 10⁵ peptides, at least 4.0 x 10⁵ peptides, at least 5.0 x 10⁵ peptides, at least 6.0 x 10⁵ peptides, at least 7.0 x 10⁵ peptides, at least 8.0 x 10⁵ peptides, at least 9.0 x 10⁵ peptides, at least 1.0 x 10⁶ peptides, at least 1.2 x 10⁶ peptides, at least 1.4 x 10⁶ peptides, at least 1.6 x 10⁶ peptides, at least 1.8 x 10⁶ peptides, at least 1.0 x 10⁷ peptides, or at least 1.0 x 10⁸ peptides attached to the array support of the peptide microarray. In other embodiments, the peptide microarrays described herein can have about 1.6 x 10⁵ peptides, about 2.0 x 10⁵ peptides, about 3.0 x 10⁵ peptides, about 4.0 x 10⁵ peptides, about 5.0 x 10⁵ peptides, about 6.0 x 10⁵ peptides, about 7.0 x 10⁵ peptides, about 8.0 x 10⁵ peptides, about 9.0 x 10⁵ peptides, about 1.0 x 10⁶ peptides, about 1.2 x 10⁶ peptides, about 1.4 x 10⁶ peptides, about 1.6 x 10⁶ peptides, about 1.8 x 10⁶ peptides, about 1.0 x 10⁷ peptides, or about 1.0 x 10⁸ peptides attached to the array support of the peptide microarray. As described herein, a peptide microarray comprising a particular number of peptides can mean a single peptide microarray on a single array support, or the peptides can be divided and attached to more than one array support to obtain the number of peptides described herein.

In one embodiment, the cyclic peptides or peptides of interest for use in the peptide microarrays described herein can be synthetic. In one aspect, the cyclic peptides or peptides of interest on the peptide microarrays described herein can be synthesized as described in Example 1. Any appropriate protocols for synthesizing peptides for use on peptide microarrays that are well-known by persons of skill in the art can also be used.

In various embodiments, the cyclic peptides or peptides of interest attached to the peptide microarrays can lack cysteines, can lack amino acid repeats, can be unique (i.e., each peptide is different from the other peptides on the array), and/or can represent cyclic peptides, peptides of interest, or cyclic peptides of interest with a length selected from the group consisting of 5-mers, 6-mers, 7-mers, 8-mers, 9-mers, 10-mers, 11-mers, and 12-mers, or a combination thereof.

As described herein, a "peptide microarray" means an intentionally created collection of peptides that can be prepared synthetically. In one embodiment, the peptides on the peptide microarray can be different from each other. Methods for synthesizing peptide microarrays, including peptide microarrays made by maskless light-directed peptide array synthesis, are known in the art and exemplary methods are described in U.S. Patent Appl. Publication Nos. U.S 2004/0023367 and U.S. 2009/0176664 and U.S. Patent Nos. 6,375,903 and 5,143,854. Additional methods are described herein below in Example 1.

In one embodiment, the peptides on the peptide microarray are attached to an array support. An array support refers to a material or materials having a rigid or semi-rigid surface or surfaces. In some aspects, at least one surface of the array support will be substantially flat, although in some aspects regions may be physically separated for different peptides with, for example, wells, raised regions, pins, etched trenches, or the like. In yet another embodiment, the peptide microarray is made by maskless light-directed peptide array synthesis.

In various embodiments, array support materials may include, for example, silicon, bio-compatible polymers such as, for example poly(methyl methacrylate) (PMMA) and polydimethylsiloxane (PDMS), glass, plastic, SiO2, quartz, silicon nitride, functionalized glass, gold, platinum, carbon composite, or aluminum. Functionalized surfaces include for example, amino-functionalized glass, carboxy functionalized glass, and hydroxy functionalized glass. Additionally, an array support may optionally be coated with one or more layers to provide a surface for molecular attachment or functionalization, increased or decreased reactivity, binding detection, and the like. The appropriate array support material can be selected by a person skilled in the art.

In one embodiment, the peptide microarray can be made using maskless light-directed peptide array synthesis. Maskless light-directed peptide array synthesis may utilize micromirrors and projection optics which focus an image of the micromirrors on the array support where the reactions are conducted. In one embodiment, under the control of a computer, each of the micromirrors is selectively switched between a first position at which it projects light on the substrate through the optical system and a second position at which it deflects light away from the substrate. In this embodiment, the individually controllable mirrors can steer light beams to produce images or light patterns. In one embodiment, reactions at different regions on the array support can be modulated by providing irradiation of different strengths using a micromirror device. Such devices are available commercially. In one aspect, the controlled light irradiation allows control of the reactions to proceed at a desirable rate.

In one embodiment, the cyclic peptides or peptides of interest are attached covalently to the array support. In another embodiment, the cyclic peptides, peptides of interest, or cyclic peptides of interest are attached non-covalently to the array support. In yet another embodiment, the peptides are attached to the array support by a linker, such as a cleavable linker. In one illustrative embodiment, the linker is about 4 to about 40 atoms long. Exemplary linkers are aryl acetylene, ethylene glycol oligomers containing 2-10 monomer units (PEGs), diamines, diacids, amino acids, and the like, and combinations thereof.

In yet other embodiments of the embodiments described herein, the cow antibody scaffold in any embodiment described herein can be replaced with any antibody scaffold polypeptide, including an antibody scaffold polypeptide of a non-bovine animal. As used herein "antibody scaffold polypeptide" means a polypeptide comprising sequences from the CDR H3 region of an antibody of a non-bovine animal. The embodiments using an "antibody scaffold polypeptide" include the embodiments described below in clauses 109 to 193. Examples of non-bovine animals include, but are not limited to, a shark (e.g., immunoglobulin new antigen receptors (IgNARs) from shark serum), a camel (e.g., camel VnHs regions), and a human (e.g., human anti-influenza and anti-HIV antibodies with long CDR H3 regions).
109. A method of selecting a peptide of interest, the method comprising the steps of
   a) preparing a peptide library using an mRNA-displayed antibody scaffold polypeptide from an antibody CDR H3 region comprising the peptide of interest to be identified;
   b) selecting the peptide of interest from the peptide library by contacting a target molecule with the peptide of interest wherein the target molecule is immobilized on a solid support or is in solution; and
   c) identifying the amino acid sequence of the peptide of interest.
110. The method of clause 109 wherein the step of preparing the peptide library comprises the step of *in vitro* transcription of a DNA library to form an mRNA library.
111. The method of clause 109 or 110 further comprising the step of digesting the DNA library with DNase.
112. The method of clause 109 or 110 further comprising the step of conjugating the mRNA from the mRNA library to a puromycin oligonucleotide linker.
113. The method of any one of clauses 110 to 112 further comprising the step of translating *in vitro* the mRNA from the mRNA library to form mRNA-antibody scaffold polypeptide fusion conjugates comprising the antibody scaffold polypeptide wherein the antibody scaffold polypeptide comprises a purification tag.
114. The method of clause 113 further comprising the step of purifying the mRNA-antibody scaffold polypeptide fusion conjugates using the purification tag.
115. The method of clause 113 or 114 further comprising the step of reverse transcribing the mRNA from the mRNA library to form mRNA-DNA duplexes of the mRNA- antibody scaffold polypeptide fusion conjugates.
116. The method of clause 115 wherein the step of selecting comprises contacting the mRNA-DNA duplexes of the mRNA-antibody scaffold polypeptide fusion conjugates with the target molecule.
117. The method of clause 116 further comprising the step of regenerating the DNA from the mRNA-DNA duplexes of the mRNA-antibody scaffold polypeptide fusion conjugates.
118. The method of any one of clauses 110 to 117 wherein the DNA library is a cDNA library.
119. The method of any one of clauses 110 to 118 wherein the *in vitro* transcription is performed using T7 RNA polymerase.
120. The method of any one of clauses 112 to 119 wherein the puromycin oligonucleotide linker is linked to the mRNA at the 3' end of the mRNA.
121. The method of any one of clauses 113 to 120 wherein the purification tag is a FLAG tag.
122. The method of any one of clauses 110 to 121 wherein members of the DNA library comprise an RNA polymerase promoter sequence, an enhancer sequence, and a purification tag sequence.
123. The method of any one of clauses 113 to 122 wherein the purification tag is a C-terminal tag.
124. The method of any one of clauses 109 to 123 further comprising the steps of:
   a) synthesizing the peptide of interest, or derivatives thereof, on a first peptide microarray, wherein the derivatives of the peptide of interest include at least one alteration in the sequence of the peptide of interest selected from a single amino acid substitution, a double amino acid substitution, a deletion of one or more amino acids, and an insertion of one or more amino acids, whereby functionalized peptides are generated on the first peptide microarray;
   b) forming from the functionalized peptides, wherein the functionalized peptides are in linear form, cyclic peptides of formula I 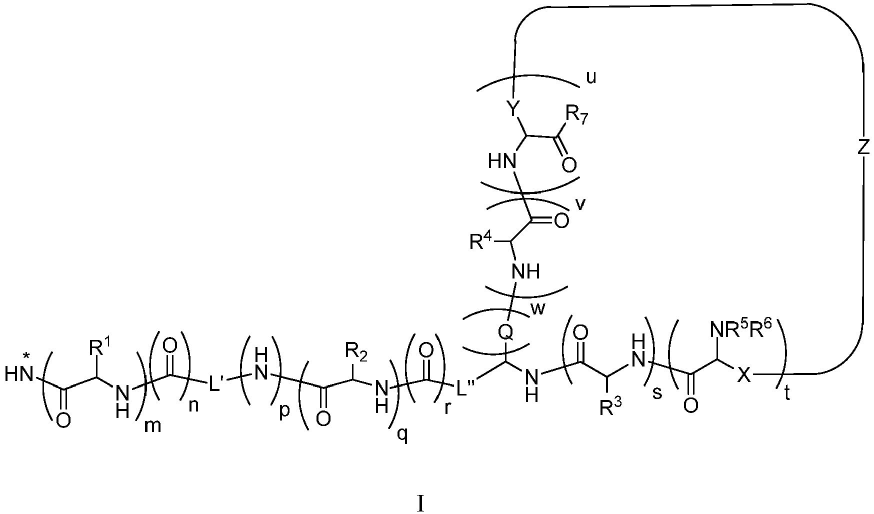
      wherein each R¹, R², R³ and R⁴ is independently a natural amino acid side chain or a non-natural amino acid side chain;
      each R⁵ and R⁶ is independently selected from the group consisting of hydrogen, an N-terminal capping group, and a N-terminal protecting group;
      R⁷ is selected from the group consisting of -OH, a C-terminal capping group, a C-terminal protecting group, and 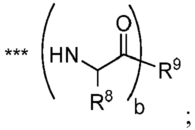
      each R⁸ is independently a natural amino acid side chain or a non-natural amino acid side chain;
      R⁹ is selected from the group consisting of -OH, a C-terminal capping group, and a C-terminal protecting group;
      Q is selected from the group consisting of a bond, a carbonyl, a natural amino acid side chain, and a non-natural amino acid side chain;
      each X and Y is independently selected from the group consisting of a bond, a natural amino acid side chain covalently attached to Z, and a non-natural amino acid side chain covalently attached to Z;
      Z is a group comprising a moiety selected from the group consisting of an amide bond, a disulfide bond, an isopeptide bond, a 1,2,3-triazole, and an optionally substituted 1,2-quinone;
      each L' and L" is independently an optional bivalent linking group or a bond;
      b is an integer from 0 to 50;
      m is an integer from 0 to 6;
      n is 0 or 1;
      p is 0 or 1;
      q is an integer from 0 to 6;
      r is 0 or 1;
      s is an integer from 0 to 100;
      t is 0 or 1;
      u is 0 or 1;
      v is an integer from 0 to 100;
      w is 0 or 1; and * is a point of connection connecting the cyclic peptide to an array support having a reactive surface; and *** is a point of connection to the rest of the functionalized peptide;
      the method comprising the step of reacting a functionalized peptide of formula II under conditions that cause Z to form 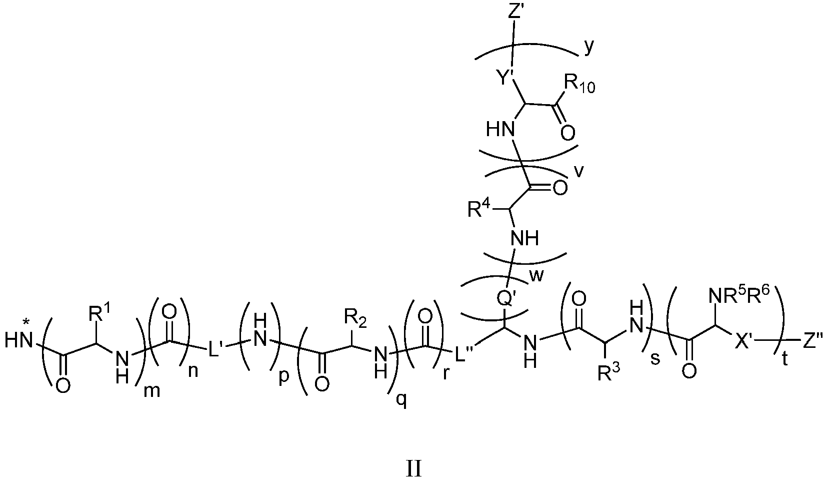
      wherein R¹, R² R³, R⁴, R⁵, R⁶, m, n, p, q, r, s, t, v, w, L', L", *, and *** are as defined for formula I;
      R¹⁰ is selected from the group consisting of -OH, a C-terminal capping group, a C-terminal protecting group, and 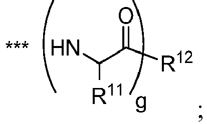
      each R¹¹ is independently a natural amino acid side chain or a non-natural amino acid side chain;
      R¹² is selected from the group consisting of -OH, a C-terminal capping group, and a C-terminal protecting group;
      Q' is selected from the group consisting of a bond, a carbonyl, a natural amino acid side chain covalently attached to Z", and a non-natural amino acid side chain covalently attached to Z";
      X' is selected from the group consisting of a bond, a natural amino acid side chain covalently attached to Z", and a non-natural amino acid side chain covalently attached to Z";
      Y' is selected from the group consisting of a bond, a natural amino acid side chain covalently attached to Z', and a non-natural amino acid side chain covalently attached to Z';
      each Z' and Z" is independently selected from the group consisting of a bond, -OH, hydrogen, a thiol, an amine, a carboxylic acid, an amide, an alkyne, an azide, an optionally substituted aminophenol, a natural amino acid side chain, a non-natural amino acid side chain, an N-terminal protecting group, and a C-terminal protecting group, provided that Z' and Z" are complementary groups that combine to form Z;
      g is an integer from 0 to 50; and
      y is 0 or 1;
      wherein the functionalized peptides and the cyclic peptides are immobilized to the reactive surface;
   c) exposing the cyclic peptides to the target molecule, whereby the target molecule binds to at least one cyclic peptide;
   d) identifying one or more of the cyclic peptides demonstrating strong binding to the target molecule, whereby a matured core binder sequence is determined;
   e) performing at least one of N-terminal and C-terminal extension of the matured core binder sequence determined in step d to provide a matured, extended core binder sequence on a second peptide microarray;
   f) exposing the target molecule to the second peptide microarray comprising a population of matured, extended core binder sequence peptides generated in step e wherein the population of matured, extended core binder sequence peptides comprises cyclic peptides formed as in step b; and
   g) identifying a matured, extended cyclic peptide with strong binding to the target molecule.
125. The method of clause 124, wherein Z comprises a moiety selected from the group consisting of an amide bond, 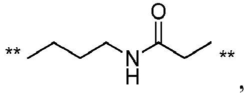 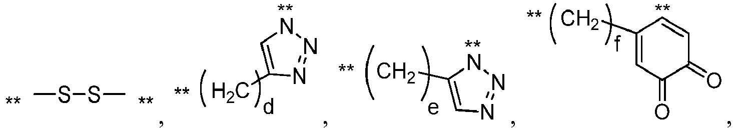 and 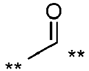 wherein d is an integer from 0 to 6, e is an integer from 0 to 6, and f is an integer from 0 to 6, and ** is a point of connection to the rest of the cyclic peptide.
126. The method of clause 124 or 125, wherein Z comprises a peptide bond, Z" comprises an N-terminal protecting group, t is 0, u is 0, and y is 0.
127. The method of clause 126, further comprising removing Z" from the rest of the functionalized peptide to cause the peptide bond to form.
128. The method of clause 124 or 125, wherein Q and X are bonds to Z, Z comprises **-S-S-**, X' is a bond to Z", Q' is a bond to Z', Z' and Z" comprise cysteine side chains, t is 1, u is 0, v is 0, w is 1, and y is 0.
129. The method of clause 128, further comprising subjecting the functionalized peptide to oxidative conditions to cause **-S-S-** to form.
130. The method of clause 124 or 125, wherein X and Y are bonds to Z, Z comprises **-S-S-**, X' is a bond to Z", Y' is a bond to Z', Z' and Z" comprise cysteine side chains, t is 1, u is 1, and y is 1.
131. The method of clause 130, further comprising subjecting the functionalized peptide to oxidative conditions to cause **-S-S-** to form.
132. The method of clause 124 or 125, wherein Q is a bond to Z, Z comprises 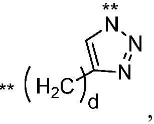 Q' is a bond to Z', Z' comprises 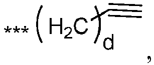 Z" comprises an azide, d is 1, u is 0, v is 0, w is 1, and y is 0.
133. The method of clause 132, further comprising contacting the functionalized peptide with a copper catalyst to cause 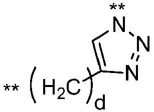 to form.
134. The method of clause 124 or 125, wherein Y is a bond to Z, Z comprises 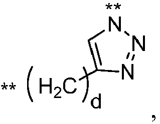 Y' is a bond to Z', Z' comprises 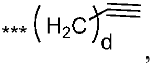 Z" comprises an azide, d is 1, u is 1, and y is 1.
135. The method of clause 134, further comprising contacting the functionalized peptide with a copper catalyst to cause 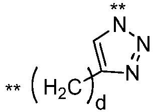 to form.
136. The method of clause 124 or 125, wherein Q is a bond to Z, Z comprises 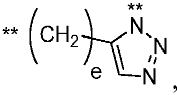 Q' is a bond to Z', Z' comprises 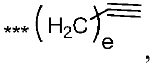 Z" comprises an azide, e is 1, u is 0, v is 0, w is 1, and y is 0.
137. The method of clause 136, further comprising contacting the functionalized peptide with a copper catalyst to cause 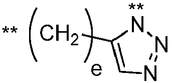 to form.
138. The method of clause 124 or 125, wherein Y is a bond to Z, Z comprises 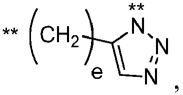 Y' is a bond to Z', Z' comprises 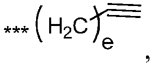 Z" comprises an azide, e is 1, u is 1, and y is 1.
139. The method of clause 138, further comprising contacting the functionalized peptide with a copper catalyst to cause 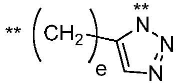 to form.
140. The method of clause 124 or 125, wherein Q is a bond to Z, Z comprises 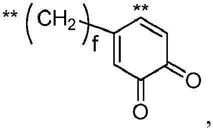 Q' is a bond to Z', Z' comprises 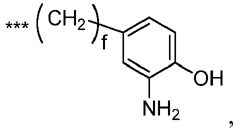 Z" comprises an amine, f is 1, u is 0, v is 0, w is 1, and y is 0.
141. The method of clause 140, further comprising contacting the functionalized peptide with a potassium ferricyanide to cause 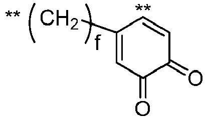 to form.
142. The method of clause 124 or 125, wherein Y is a bond to Z, Z comprises 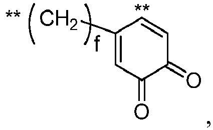 Z" comprises an amine, Y' is a bond to Z', Z' comprises 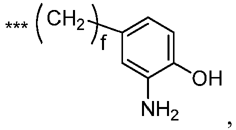 f is 1, u is 1, and y is 1.
143. The method of clause 142, further comprising contacting the functionalized peptide with a potassium ferricyanide to cause 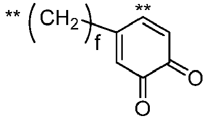 to form.
144. The method of clause 124 or 125, wherein R⁴, R¹⁰, and R¹¹ are defined such that the functionalized peptide comprises a butelase 1 recognition sequence, Y is a bond to Z, Z comprises 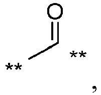 Y' is a bond to Z', Z' is an asparagine or aspartic acid side chain, u is 1, and y is 1.
145. The method of clause 144, further comprising contacting the functionalized peptide with butelase 1 to cause 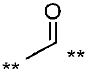 to form.
146. The method of clause 124 or 125, wherein Q and X are bonds to Z, Z comprises 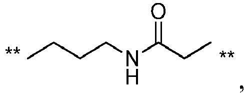 Q' is a bond to Z', X' is a bond to Z", Z' is a glutamine side chain and Z" is a lysine side chain or Z' is a lysine side chain and Z" is a glutamine side chain, t is 1, u is 0, v is 0, w is 1, and y is 0.
147. The method of clause 146, further comprising contacting the functionalized peptide with a microbial transglutaminase to cause 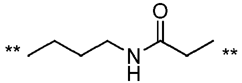 to form.
148. The method of clause 124 or 125, wherein X and Y are bonds to Z, Z comprises 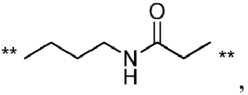 X' is a bond to Z", Y' is a bond to Z', Z' is a glutamine side chain and Z" is a lysine side chain or Z' is a lysine side chain and Z" is a glutamine side chain, t is 1, u is 1, and y is 1.
149. The method of clause 148, further comprising contacting the functionalized peptide with a microbial transglutaminase to cause 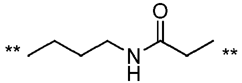 to form.
150. The method of any one of clauses 124 to 149, wherein each L' and L" is independently of the formula V 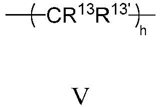 wherein each R¹³ and R^{13'} is independently selected from the group consisting of H, D, halogen, C₁-C₆ alkyl, C₂-C₆ alkenyl, C₂-C₆ alkynyl, C₃-C₆ cycloalkyl, 3- to 7-membered heterocycloalkyl, C₆-C₁₀ aryl, 5- to 7-membered heteroaryl, -OR¹⁴, OC(O)R¹⁴, -NR¹⁴R^{14'}, -NR¹⁴C(O)R¹⁵, -C(O)R¹⁴, -C(O)OR¹⁴, and -C(O)NR¹⁴R^{14'}, wherein each hydrogen atom in C₁-C₆ alkyl, C₂-C₆ alkenyl, C₂-C₆ alkynyl, C₃-C₆ cycloalkyl, 3- to 7-membered heterocycloalkyl, C₆-C₁₀ aryl and 5- to 7-membered heteroaryl is independently optionally substituted by halogen, C₁-C₆ alkyl, C₂-C₆ alkenyl, C₂-C₆ alkynyl, -OR¹⁶; each R¹⁴, R^{14'}, R¹⁵, and R¹⁶ is independently selected from the group consisting of H, D, hydroxyl, C₁-C₇ alkyl, C₂-C₇ alkenyl, C₂-C₇ alkynyl, C₃-C₆ cycloalkyl, 3- to 7-membered heterocycloalkyl, C₆-C₁₀ aryl and 5- to 7-membered heteroaryl; and h is an integer from 1 to 10; or the formula VI or VII 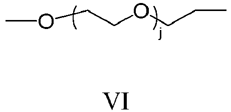 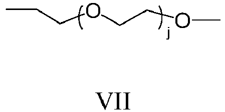 wherein j is an integer from 0 to 30.
151. The method of clause 150, wherein each R¹³ and R^{13'} is hydrogen.
152. The method of clause 150 or 151, wherein L' is present, h is 5, m is 0, n is 1, and p is 1.
153. The method of clause 150, wherein at least one of L' and L" is of the formula VI or VII 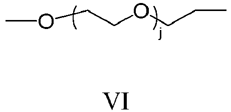 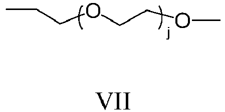 wherein j is 7.
154. The method of any one of clauses 124 to 153, wherein the N-terminal protecting group is a photoprotecting group.
155. The method of any one of clauses 124 to 154, wherein the N-terminal protecting group is 2-(2-nitrophenyl)propyloxycarbonyl.
156. The method of any one of clauses 109 to 123 further comprising the step of synthesizing the antibody scaffold polypeptide on one or more peptide microarrays wherein the antibody scaffold polypeptide comprises the peptide of interest.
157. The method of clause 156 comprising the steps of:
   a) synthesizing the antibody scaffold polypeptide comprising the peptide of interest, or derivatives of the peptide of interest, on a first peptide microarray, wherein the derivatives of the peptide of interest include at least one alteration in the sequence of the peptide of interest selected from a single amino acid substitution, a double amino acid substitution, a deletion of one or more amino acids, and an insertion of one or more amino acids, whereby functionalized peptides are generated on the first peptide microarray;
   b) forming from the functionalized peptides, wherein the functionalized peptides are in linear form, cyclic peptides of formula VIII 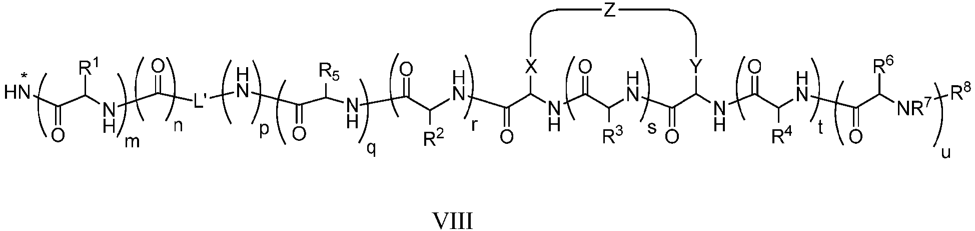
      wherein each R¹, R², R³, and R⁴ is independently a natural amino acid side chain or a non-natural amino acid side chain;
      each R⁵ and R⁶ is independently a natural amino acid side chain or a non-natural amino acid side chain selected such that 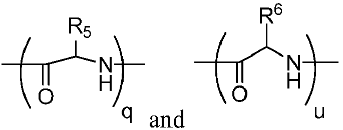 can form a beta-sheet;
      each R⁷ is independently selected from the group consisting of hydrogen, an N-terminal capping group, and an N-terminal protecting group;
      R⁸ is selected from the group consisting of hydrogen, an N-terminal capping group, and a protecting group;
      each X and Y is independently selected from the group consisting of a bond, a natural amino acid side chain covalently attached to Z, and a non-natural amino acid side chain covalently attached to Z;
      Z is a group comprising a moiety selected from the group consisting of an amide bond, a disulfide bond, an isopeptide bond, a 1,2,3-triazole, and an optionally substituted 1,2-quinone;
      L' is an optional bivalent linking group or a bond;
      m is an integer from 0 to 6;
      n is 0 or 1;
      p is 0 or 1;
      q is an integer from 0 to 50;
      r is an integer from 0 to 50;
      s is an integer from 0 to 50;
      t is an integer from 0 to 50;
      u is an integer from 0 to 50; and * is a point of connection connecting the cyclic peptide to an array support having a reactive surface;
      the method comprising the step of reacting a functionalized peptide of formula IX under conditions that cause Z to form 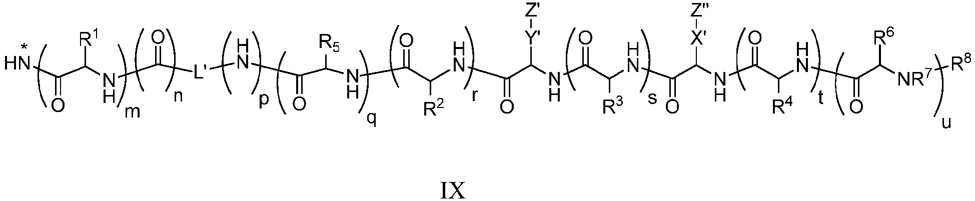
      wherein R¹, R² R³, R⁴, R⁵, R⁶, R⁷, and R⁸, m, n, p, q, r, s, t, u, L' and * are as defined for formula VIII;
      X' is selected from the group consisting of a bond, a natural amino acid side chain covalently attached to Z", and a non-natural amino acid side chain covalently attached to Z";
      Y' is selected from the group consisting of a bond, a natural amino acid side chain covalently attached to Z', and a non-natural amino acid side chain covalently attached to Z'; and
      each Z' and Z" is independently selected from the group consisting of a bond, -OH, hydrogen, a thiol, an amine, a carboxylic acid, an amide, an alkyne, an azide, an optionally substituted aminophenol, a natural amino acid side chain, a non-natural amino acid side chain, an N-terminal protecting group, and a C-terminal protecting group, provided that Z' and Z" are complementary groups that combine to form Z;
      wherein the functionalized peptides and the cyclic peptides are immobilized to the reactive surface;
   c) exposing the cyclic peptides to the target molecule, whereby the target molecule binds to at least one cyclic peptide;
   d) identifying one or more of the cyclic peptides demonstrating strong binding to the target molecule, whereby a matured core binder sequence is determined;
   e) performing at least one of N-terminal and C-terminal extension of the matured core binder sequence determined in step d to provide a matured, extended core binder sequence on a second peptide microarray;
   f) exposing the target molecule to the second peptide microarray comprising a population of matured, extended core binder sequence peptides generated in step e wherein the population of matured, extended core binder sequence peptides comprises cyclic peptides formed as in step b; and
   g) identifying a matured, extended cyclic peptide with strong binding to the target molecule.
158. The method of clause 157, wherein Z comprises a moiety selected from the group consisting of an amide bond, 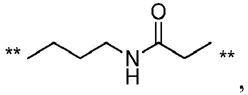 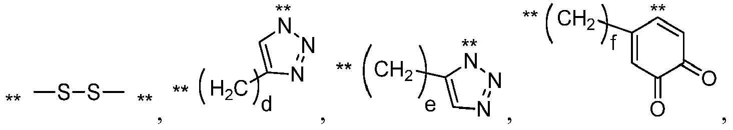 and 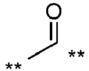 wherein d is an integer from 0 to 6, e is an integer from 0 to 6, and f is an integer from 0 to 6, and ** is a point of connection to the rest of the cyclic peptide.
159. The method of clause 157 or 158, wherein X and Y are bonds to Z, Z comprises **-S-S-**, X' is a bond to Z", Y' is a bond to Z', and Z' and Z" comprise cysteine side chains.
160. The method of clause 159, further comprising subjecting the functionalized peptide to oxidative conditions to cause **-S-S-** to form.
161. The method of clause 157 or 158, wherein Y is a bond to Z, Z comprises 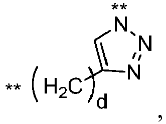 Y' is a bond to Z', Z' comprises 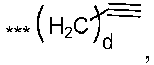 Z" comprises an azide, and d is 1.
162. The method of clause 161, further comprising contacting the functionalized peptide with a copper catalyst to cause 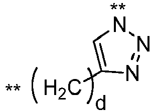 to form.
163. The method of clause 157 or 158, wherein Y is a bond to Z, Z comprises 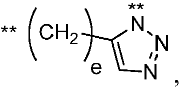 Y' is a bond to Z', Z' comprises 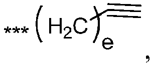 Z" comprises an azide, and e is 1.
164. The method of clause 163, further comprising contacting the functionalized peptide with a copper catalyst to cause 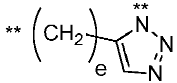 to form.
165. The method of clause 157 or 158, wherein Y is a bond to Z, Z comprises 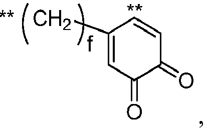 Y' is a bond to Z', Z' comprises 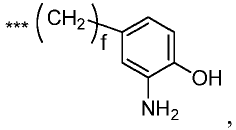 Z" comprises an amine, and f is 1.
166. The method of clause 165, further comprising contacting the functionalized peptide with a potassium ferricyanide to cause 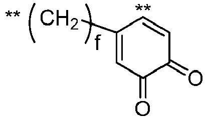 to form.
167. The method of clause 157 or 158, wherein X and Y are bonds to Z, Z comprises 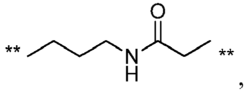 X' is a bond to Z", Y' is a bond to Z', and Z' is a glutamine side chain and Z" is a lysine side chain or Z' is a lysine side chain and Z" is a glutamine side chain.
168. The method of clause 167, further comprising contacting the functionalized peptide with a microbial transglutaminase to cause 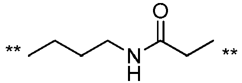 to form.
169. The method of any one of clauses 157 to 158, wherein L' is of the formula (X) 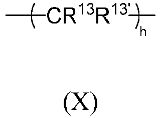 wherein each R¹³ and R^{13'} is independently selected from the group consisting of H, D, halogen, C₁-C₆ alkyl, C₂-C₆ alkenyl, C₂-C₆ alkynyl, C₃-C₆ cycloalkyl, 3- to 7-membered heterocycloalkyl, C₆-C₁₀ aryl, 5- to 7-membered heteroaryl, -OR¹⁴, -OC(O)R¹⁴, -NR¹⁴R^{14'}, -NR¹⁴C(O)R¹⁵, -C(O)R¹⁴, -C(O)OR¹⁴, and -C(O)NR¹⁴R^{14'}, wherein each hydrogen atom in C₁-C₆ alkyl, C₂-C₆ alkenyl, C₂-C₆ alkynyl, C₃-C₆ cycloalkyl, 3- to 7-membered heterocycloalkyl, C₆-C₁₀ aryl and 5- to 7-membered heteroaryl is independently optionally substituted by halogen, C₁-C₆ alkyl, C₂-C₆ alkenyl, C₂-C₆ alkynyl, -OR¹⁶; each R¹⁴, R^{14'}, R¹⁵, and R¹⁶ is independently selected from the group consisting of H, D, hydroxyl, C₁-C₇ alkyl, C₂-C₇ alkenyl, C₂-C₇ alkynyl, C₃-C₆ cycloalkyl, 3- to 7-membered heterocycloalkyl, C₆-C₁₀ aryl and 5- to 7-membered heteroaryl; and h is an integer from 1 to 10; or the formula XI or XII 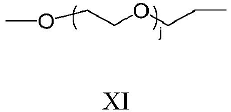 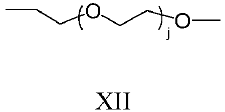 wherein j is an integer from 0 to 30.
170. The method of clause 169, wherein each R¹³ and R^{13'} is hydrogen.
171. The method of clause 169 or 170, wherein L' is present, h is 5, m is 0, n is 1, and p is 1.
172. The method of clause 169, wherein L' is of the formula XI or XII 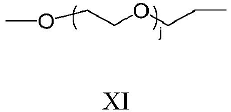 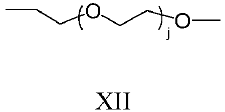 wherein j is 7.
173. The method of any one of clauses 157 to 172, wherein the N-terminal protecting group is a photoprotecting group.
174. The method of any one of clauses 157 to 173, wherein the N-terminal protecting group is 2-(2-nitrophenyl)propyloxycarbonyl.
175. The method of any one of clauses 124 to 155 and 157 to 174, wherein at least one of a label-free and an affinity analysis of the matured, extended core binder sequence peptides is performed.
176. The method of any one of clauses 124 to 155 and 157 to 175, wherein the first or second peptide microarray comprises at least one of glass, plastic, and carbon composite.
177. The method any one of clauses 124 to 155 and 157 to 176, wherein the functionalized peptides and the cyclic peptides on the first or the second peptide microarray comprise the same number of amino acids.
178. The method of any one of clauses 124 to 155 and 157 to 177, wherein the functionalized peptides and the cyclic peptides on the first or the second peptide microarray do not include the amino acid cysteine or methionine, or histidine-proline-glutamine motifs, or amino acid repeats of 2 or more amino acids.
179. The method of any one of clauses 124 to 155 and 157 to 178, wherein the population of matured, extended core binder sequence peptides includes at least one of an N-terminal wobble synthesis oligopeptide and a C-terminal wobble synthesis oligopeptide.
180. The method of any one of clauses 124 to 155 and 157 to 179 wherein the first or second peptide microarray comprises one or more linear peptides and wherein the method further comprises the step of contacting the one or more linear peptides on the first or second peptide microarray with a protease capable of digesting the one or more linear peptides.
181. The method of clause 180 wherein the protease is an amino protease or a mixture of amino proteases.
182. The method of clause 180 wherein the protease is dipeptidyl peptidase IV, aminopeptidase m, or a combination thereof.
183. An mRNA-displayed antibody scaffold polypeptide comprising a peptide of interest.
184. The mRNA-displayed antibody scaffold polypeptide of clause 183 wherein the peptide of interest is a therapeutic peptide.
185. A peptide microarray comprising an antibody scaffold polypeptide comprising a peptide of interest.
186. The peptide microarray of clause 185 wherein the peptide of interest is a therapeutic peptide.
187. The method, mRNA-displayed antibody scaffold polypeptide, or peptide microarray of any one of clauses 109 to 186 wherein the antibody scaffold polypeptide comprises a random amino acid sequence cassette and the random amino acid sequence cassette comprises the sequence of the peptide of interest.
188. The method, mRNA-displayed antibody scaffold polypeptide, or peptide microarray of clause 187 wherein the random amino acid sequence cassette does not include the sequence of an antibody, a fragment of an antibody, or a peptide fragment derived from an antibody.
189. The method of clause 144 or 145 wherein the butelase 1 recognition sequence is NHV.
190. The method of clause 146, 147, 167, or 168 wherein the glutamine side chain is part of the sequence [WY][DE][DE][YW]ALQ[GST]YD (SEQ ID NO:4)and the lysine side chain is part of the sequence RSKLG (SEQ ID NO:5).
191. The method of any one of clauses 109 to 123 further comprising the step of synthesizing the antibody scaffold polypeptide comprising the peptide of interest on a peptide microarray to mature and/or extend the peptide of interest.
192. The method, mRNA-displayed antibody scaffold polypeptide, or peptide microarray of any one of clauses 109 to 186 wherein the antibody scaffold polypeptide comprises a random amino acid sequence cassette wherein the random amino acid sequence cassette comprises the sequence of the peptide of interest and wherein the random amino acid sequence cassette is used for selection of peptides of interest.
193. An antibody scaffold polypeptide comprising a random amino acid sequence cassette.

In another embodiment, the methods, peptide microarrays, and mRNA-displayed cow antibody scaffold polypeptides and antibody scaffold polypeptides described herein include the following examples. The examples further illustrate additional features of the various embodiments of the invention described herein. However, it is to be understood that the examples are illustrative and are not to be construed as limiting other embodiments of the invention described herein. In addition, it is appreciated that other variations of the examples are included in the various embodiments of the invention described herein.

### Example 1

### Microarrays

Various methods for the production of microarrays are known in the state of the art. For example, spotting prefabricated peptides or *in-situ* synthesis by spotting reagents, e.g., on membranes, exemplify known methods. Other known methods used for generating peptide arrays of higher density are the so-called photolithographic techniques, where the synthetic design of the desired biopolymers is controlled by suitable photolabile protecting groups (PLPG) releasing the linkage site for the respective next component (e.g., amino acid) upon exposure to electromagnetic radiation, such as light (Fodor et al., (1993) Nature 364:555-556; Fodor et al., (1991) Science 251:767-773). Two different photolithographic techniques are known in the state of the art. The first is a photolithographic mask, used to direct light to specific areas of the synthesis surface effecting localized deprotection of the PLPG. "Masked" methods include the synthesis of polymers utilizing a mount (e.g., a "mask") which engages a substrate and provides a reactor space between the substrate and the mount. Exemplary embodiments of such "masked" array synthesis are described in, for example, U.S. Patent Nos. 5,143,854 ad 5,445,934. The second photolithographic technique is maskless photolithography, where light is directed to specific areas of the synthesis surface effecting localized deprotection of the PLPG by digital projection technologies, such as micromirror devices (Singh-Gasson et al., Nature Biotechn. 17 (1999) 974-978). It should be understood that the embodiments of the methods disclosed herein may comprise or utilize any of the various microarray synthesis techniques described above.

The use of PLPG (photolabile protecting groups), providing the basis for the photolithography based synthesis of peptide microarrays, is well known in the art. Commonly used PLPG for photolithography based biopolymer synthesis are for example α-methyl-6-nitropiperonyl-oxycarbonyl (MeNPOC) (Pease et al., Proc. Natl. Acad. Sci. USA (1994) 91:5022-5026), 2-(2-nitrophenyl)-propoxycarbonyl (NPPOC) (Hasan et al. (1997) Tetrahedron 53: 4247-4264), nitroveratryloxycarbonyl (NVOC) (Fodor et al. (1991) Science 251:767-773) and 2-nitrobenzyloxycarbonyl (NBOC) (Patchornik et al. (1970) 21:6333-6335).

Amino acids have been introduced in photolithographic solid-phase peptide synthesis of peptide microarrays, which were protected with NPPOC as a photolabile amino protecting group, wherein glass slides were used as a support (U.S. Patent Publication No. 2005/0101763 A1). The method using NPPOC protected amino acids has the disadvantage that the half-life upon irradiation with light of all (except one) protected amino acids is within the range of approximately 2 to 3 minutes under certain conditions. In contrast, under the same conditions, NPPOC-protected tyrosine exhibits a half-life of almost 10 minutes. As the velocity of the whole synthesis process depends on the slowest sub-process, this phenomenon increases the time of the synthesis process by a factor of 3 to 4. Concomitantly, the degree of damage by photogenerated radical ions to the growing oligomers increases with increasing and excessive light dose requirement.

A single peptide microarray or, in some cases, multiple microarrays (e.g., 3, 4, 5, or more microarrays) can be located on one array support. The size of the peptide microarrays depends on the number of microarrays on one array support. The higher the number of microarrays per array support, the smaller the arrays have to be to fit on the array support. The arrays can be designed in any shape, but preferably they are designed as squares or rectangles.

The term feature refers to a defined area on the surface of a peptide microarray. The feature comprises biomolecules, such as peptides, and the like. One feature can contain biomolecules with different properties, such as different sequences or orientations, as compared to other features. The size of a feature is determined by two factors: i) the number of features on a microarray, the higher the number of features on a microarray, the smaller is each single feature, ii) the number of individually addressable aluminum mirror elements which are used for the irradiation of one feature. The higher the number of mirror elements used for the irradiation of one feature, the bigger is each single feature. The number of features on a microarray may be limited by the number of mirror elements (pixels) present in the micro mirror device. For example, the state of the art micro mirror device from Texas Instruments, Inc. currently contains 4.2 million mirror elements (pixels), thus the number of features within such an exemplary microarray is therefore limited by this number. However, it should be understood that the micro mirror device from Texas Instruments, Inc. is provided only for exemplary purposes and higher density microarrays are possible.

It should be understood that the term array support refers to any solid material, having a surface area to which organic molecules can be attached through bond formation or absorbed through electronic or static interactions such as covalent bond or complex formation through a specific functional group. The array support can be a combination of materials such as plastic on glass, carbon on glass, and the like. The functional surface can be simple organic molecules but can also comprise of co-polymers, dendrimers, molecular brushes and the like. Plastic can be used as a support and preferably the plastic is a polyolefin with defined optical properties, like TOPAS® or ZEONOR/EX®.

The term "functional group" as used in this Example refers to any of numerous combinations of atoms that form parts of chemical molecules, that undergo characteristic reactions themselves, and that influence the reactivity of the remainder of the molecule. Typical functional groups include, but are not limited to, hydroxyl, carboxyl, aldehyde, carbonyl, amino, azide, alkynyl, thiol and nitril. Potentially reactive functional groups include, for example, amines, carboxylic acids, alcohols, double bonds, and the like. Preferred functional groups are potentially reactive functional groups of amino acids such as amino groups or carboxyl groups.

As understood by one of skill in the art, peptide microarrays comprise an assay principle whereby thousands (or millions) of peptides (in some embodiments presented in multiple copies) are linked or immobilized to the surface of an array support (which in some embodiments comprises a glass, carbon composite and/or plastic chip or slide). In some embodiments, the peptide microarray, after incubation with a target molecule, undergoes one or more washing steps, and then is exposed to a detection system, for example, utilizing fluorescence, chemiluminescence, colorimetric methods, or autoradiography.

In the case of binding events, after scanning the microarray slides, the scanner can record a 20-bit, 16-bit or 8-bit numeric image in tagged image file format (*.tif). The .tif-image enables interpretation and quantification of the data obtained from the scanned microarray slide. This quantitative data can be the basis for performing statistical analysis on measured binding events or peptide modifications on the microarray slide. For evaluation and interpretation of detected signals an allocation of the peptide spot (visible in the image) and the corresponding peptide sequence has to be performed. The data for allocation is usually saved in the GenePix Array List (.gal) file and supplied together with the peptide microarray. The .gal-file (a tab-separated text file) can be opened using microarray quantification software-modules or processed with a text editor (e.g. notepad) or Microsoft Excel. This 'gal' file is most often provided by the microarray manufacturer and is generated by input txt files and tracking software built into the robots that do the microarray manufacturing.

A peptide microarray is a planar slide with peptides spotted onto it or assembled directly on the surface by *in-situ* synthesis. Peptides are ideally covalently linked through a chemoselective bond leading to peptides with the same orientation for interaction profiling. Alternative procedures include unspecific covalent binding and adhesive immobilization.

After identification of a core binder sequence, a process of "peptide maturation" can be conducted whereby the core binder sequence is altered in various ways (through amino acid substitutions, deletions and insertions) at each position of the core binder sequence in order to further optimize/verify the proper core binder sequence. For example, according to some embodiments (for example, where the core binder sequence comprises a given number of, such as 5, amino acids), a maturation array is produced. The maturation array may have, for example, immobilized thereto, a population of core binder sequences whereby each amino acid in the core binder sequence has undergone an amino acid substitution at each position.

An example/hypothetical core binder sequence is described as consisting of a 5-mer peptide having the amino acid sequence -M1M2M3M4M5-(SEQ ID NO: 372). Hit maturation may involve any of, or a combination of any or all of, amino acid substitutions, deletions and insertions at positions 1, 2, 3, 4 and 5. For example, in regard to the hypothetical core binder sequence -M1M2M3M4M5-(SEQ ID NO: 372), embodiments may include the amino acid M at position 1 being substituted with each of the other 19 amino acids (e.g., A1M2M3M4M5-(SEQ ID NO: 373), P1M2M3M4M5- (SEQ ID NO: 374), V1M2M3M4M5- (SEQ ID NO: 375), Q1M2M3M4M5- (SEQ ID NO: 376), etc.). Each position (2, 3,4 and 5) would also have the amino acid M substituted with each of the other 19 amino acids (for example, with position 2 the substitutions would resemble, M1A2M3M4M5- (SEQ ID NO: 377), M1Q2M3M4M5- (SEQ ID NO: 378), M1P2M3M4M5- (SEQ ID NO: 379), M1N2M3M4M5- (SEQ ID NO: 380), etc.). It should be understood that a peptide (immobilized on an array) is created comprising the substituted and/or deleted and/or inserted sequences of the core

In some embodiments of maturation according to the instant disclosure, a double amino acid substitution may be performed. A double amino acid substation includes altering the amino acid at a given position (e.g.,a M→P substitution, for example at position 1) and then substituting the amino acid at position 2 with each of the other 19 amino acids the amino acid at position 2. This process is repeated until all possible combinations of positions 1 and 2 are combined. By way of example, referring back to the hypothetical core binder sequence having a 5-mer peptide with amino acid sequence -M1M2M3M4M5-(SEQ ID NO: 372), a double amino acid substitution with regard to positions 1 and 2 may include, for example, a M→P substitution at position 1, and then a substation of all 20 amino acids at position 2 (e.g, -P1A2M3M4M5- (SEQ ID NO: 381), - P 1F2M3M4M5- (SEQ ID NO: 382), -P1V2M3M4M5- (SEQ ID NO: 383), - P 1E2M3M4M5- (SEQ ID NO: 384), etc.), a M→V substitution at position 1, and then a substation of all 20 amino acids at position 2 (e.g, - V 1A2M3M4M5- (SEQ ID NO: 385), - V 1F2M3M4M5- (SEQ ID NO: 386), - P1V2M3M4M5- (SEQ ID NO: 387), - V 1E2M3M4M5- (SEQ ID NO: 388), etc.), M→A substitution at position 1, and then a substation of all 20 amino acids at position 2 (e.g, - A 1A2M3M4M5- (SEQ ID NO: 389), - A 1F2M3M4M5- (SEQ ID NO: 390), - A 1V2M3M4M5- (SEQ ID NO: 391), - A 1E2M3M4M5- (SEQ ID NO: 392), etc.).

In some embodiments of maturation, an amino acid deletion for each amino acid position of the core binder sequence may be performed. An amino acid deletion includes preparing a peptide including the core binder sequence, but deleting a single amino acid from the core binder sequence (such that a peptide is created in which the amino acid at each peptide is deleted). By way of example, referring back to the hypothetical core binder sequence having a 5-mer peptide with amino acid sequence -M1M2M3M4M5- (SEQ ID NO: 372), an amino acid deletion would include preparing a series of peptides having the following sequences -M2M3M4M5- (SEQ ID NO: 393); -M1M3M4M5- (SEQ ID NO: 393); -M1M2M4M5- (SEQ ID NO: 393); -M1M2M3M5- (SEQ ID NO: 393); and -M1M2M3M4- (SEQ ID NO: 393). It should be noted that, following an amino acid deletion of the hypothetical 5-mer, 5 new 4-mers are created. According to some embodiments an amino acid substitution or a double amino acid substation scan can be performed for each new 4-mer generated.

Similar to the amino acid deletion scan discussed above, some embodiments of maturation may include an amino acid insertion scan, whereby each of the 20 amino acids is inserted before and after every position of the core binder sequence. By way of example, referring back to the hypothetical core binder sequence having a 5-mer peptide with amino acid sequence - M1M2M3M4M5- (SEQ ID NO: 372), an amino acid insertion scan could include the following sequences, -XM1M2M3M4M5- (SEQ ID NO: 394); - M1XM2M3M4M5- (SEQ ID NO: 395); -M1M2XM3M4M5- (SEQ ID NO: 396); -M1M2M3XM4M5- (SEQ ID NO: 397); -M1M2M3M4XM5- (SEQ ID NO: 398); and -M1M2M3M4M5X- (SEQ ID NO: 399) (where X represents an individual amino, selected from the 20 known amino acids or a specific, defined subset of amino acids, whereby a peptide replicate will be created for each of the 20 or defined subset of amino acids).

It should also be understood that the amino acid substituted peptides, double amino acid substituted peptides, amino acid deletion scan peptides and amino acid insertion scan peptides described above may also include one, or both of, an N-terminal and C-terminal wobble amino acid sequence. As with the N-terminal and C-terminal wobble amino acid sequences, the N-terminal and C-terminal wobble amino acid sequences may comprise as few as 1 amino acid or as many as 15 or 20 amino acids, and the N-terminal wobble amino acid sequence may be the same length as, longer than or shorter than the C-terminal wobble amino acid sequence. Further, the N-terminal and C-terminal wobble amino acid sequences may comprise any defined group of amino acids at any given ratios (for example, glycine and serine in a 3:1 ratio).

Once the various substitution, deletion and insertion variations of the core binder sequence are prepared (for example, in immobilized fashion on an array support such as a microarray), a predetermined property of the purified target molecule is analyzed, for example, under appropriate reaction or binding conditions.

Upon maturation of the core binder sequence (such that a more optimal amino acid sequence of the core binder sequence is identified for binding the target molecule, for example), the N-terminal and/or C-terminal positions can undergo an extension step, whereby the length of the matured core binder sequence is further extended for increasing the specificity and affinity for the target molecule.

According to various embodiments of N-terminal extension of the instant disclosure, once the matured core binder sequence is identified through the maturation process, any specific amino acids, can be added (or synthesized onto) the N-terminal end of a matured core binder sequence, for example. Likewise, according to various embodiments of C-terminal extension of the instant disclosure, once the matured core binder sequence is identified through the maturation process, any specific amino acids can be added (or synthesized onto) the C-terminal end of a matured core binder sequence. According to some embodiments of the instant disclosure, the matured core binder sequence used in C-terminal extension and N-terminal extension may also include one, or both of, a N-terminal and C-terminal wobble amino acid sequence. The N-terminal and C-terminal wobble amino acid sequences may comprise as few as 1 amino acid or as many as 15 or 20 amino acids (or more), and the N-terminal wobble amino acid sequence may be the same length as, longer than, or shorter than the C-terminal wobble amino acid sequence. Further, the N-terminal and C-terminal wobble amino acid sequences may comprise any defined group of amino acids at any given ratios (for example, glycine and serine in a 3:1 ratio). The extensions described above may result in a mature, extended core binder sequence peptide.

In use, an extension array can be exposed to a concentrated, purified target molecule, whereby the target molecule may bind at any peptide of interest (e.g., a cyclic peptide), independent of the other peptides comprising the populations. After exposure to the target molecule, binding or activity, for example, of the target molecule can be assayed. Because the peptide sequence for each location on the array, is known, it is possible to chart/quantify/compare/contrast the sequences (and binding strengths or activity, for example) of the target molecule in relation to the specific peptide comprising the matured, extended core binder sequence peptide. An exemplary method is to review the binding strength in a principled analysis distribution-based clustering, such as described in, Standardizing and Simplifying Analysis of Peptide Library Data, Andrew D White et al, J Chem Inf Model, 2013, 53(2), pp 493-499, incorporated herein by reference. Clustering of binding to the respective peptides shown in a principled analysis distribution-based clustering indicates peptides having overlapping peptide sequences. As demonstrated in greater detail below, from the overlapping peptide sequences (of each cluster), an extended, matured core binder sequence peptide can be identified and constructed for further evaluation. In some embodiments of the instant application, an extended, matured core binder sequence peptide undergoes a subsequent maturation process.

Following identification of an extended, matured core binder sequence, a specificity analysis may be performed according to some embodiments of the instant disclosure. One example of a specificity analysis includes a "Biacore™" system analysis which is used for characterizing peptides of interest (e.g., cyclic peptides) in terms of the interaction specifically to a target molecule, the kinetic rates (of "on," binding, and "off," disassociation) and affinity (binding strength). Biacore™ is a trademark of General Electric Company. An overview of the Biacore™ system and process is available at www.biacore.com/lifesciences/ introduction/index.html. A benefit of "Biacore™," is the ability to perform the kinetic, specificity and affinity analyses in a label-free manner.

### EXAMPLE 2

### Design of a Cow Antibody CDR H3 Scaffold Polypeptide for mRNA display

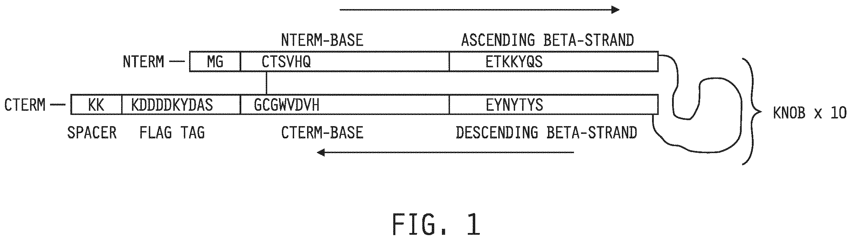

Ultralong bovine CDR H3s have certain consensus regions that define the boundary of the "stalk" and the "knob" domain of these polypeptides. For example, many antibodies (such as BLV1H12 and BLV5B8) have a "T(T/S)VHQ" (SEQ ID NO:312) motif at the base of their ascending strands. As for the descending strands of the stalk, they have alternating aromatics (for example, YXYXY) forming a ladder through stacking interactions. As a result, we determined the following sequences for a cow antibody scaffold polypeptide for use in mRNA display: MGCTSVHQETKKYQS(X)ioSYTYNYEHVDVWGCGSADYKDDDDKKK (SEQ ID NO:313). As shown in Figure 1, the N-terminal bases, the ascending beta strand, the descending beta strand and the C-terminal bases form the stalk region. The knob region of the polypeptide we produced contains 10 random residues X₁₀, where X can be any of the twenty natural amino acids (see Figure 1). A FLAG tag was appended at the C-terminus for easy recovery of the fusion molecules during the selection process. Theoretically, the number of unique amino acid sequences that could appear in a library using this cow antibody scaffold polypeptide is ∼ 10¹³ (20¹⁰).

### EXAMPLE 3

### Construction of a Synthetic DNA Library Coding the Combinatorial Peptide Library

To encode the above-mentioned full-length peptide library, a DNA template that contained more than two hundred bases was made. At this length, fidelity was often compensated with chemical synthesis. Therefore, a DNA template encoding part of the library was created first (ordered through IDT). High-fidelity PCR was then carried out to extend the library to its full length. Members of the resulting DNA library all contained a T7 RNA polymerase promoter, an enhancer sequence, a C-terminal FLAG tag coding sequence, and a random cassette encoding the ten consecutive random codons (see Figure 1). The random region used the sequence NNS (SEQ ID NO:314) (NNC (SEQ ID NO:315) or NNG (SEQ ID NO:316)) to minimize the appearance of stop codons. For a cow antibody scaffold polypeptide displaying a single peptide, the thrombin-binding peptide, the random amino acid sequence cassette was replaced with the following sequence:
TGCATTATCAAAAAGAGCCGCGATCCGGGCCGCTGC (SEQ ID NO:317), which encoded the peptide CIIKKSRDPGRC (SEQ ID NO:318).

### EXAMPLE 4

### Expression of a Thrombin-Binding Peptide and a Peptide Library

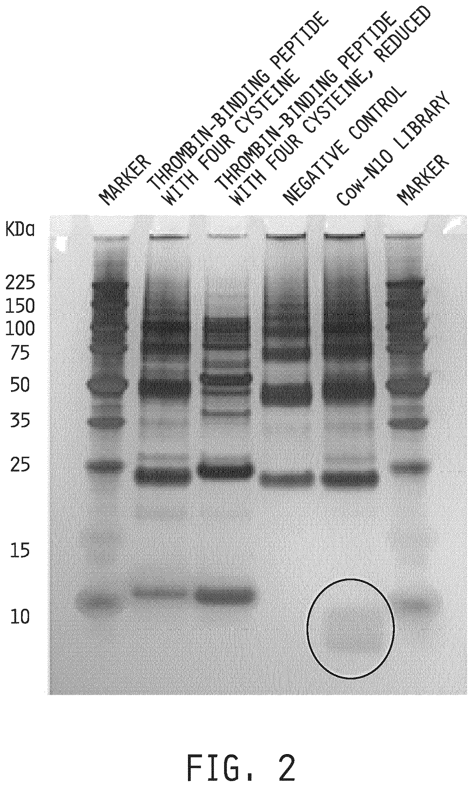

The DNA encoding the thrombin-binding peptide or the peptide library was used as a template in PURExpress In Vitro Protein Synthesis per the manufacturer's instructions. The resulting reaction mixture was checked on a denaturing SDS page gel and visualized by silver staining (see Figure 2). The band that corresponded to the thrombin-binding peptide fragment was further cut out, in-gel digested and sent for mass spec analysis (UW Biotechnology Center), which further confirmed the successful synthesis of the peptide as designed (see below).

| |
|---|
| Fixed modifications: carbamidomethyl (C) |
| Variable modifications: Deamidated (NQ),Oxidation (M) |
| Cleavage by Trypsin: cuts C-term side of KR unless next residue is P Sequence Coverage: **98%** |
| |
| Matched peptides shown in Bold Red |
| |
| |

### EXAMPLE 5

### Construction of an mRNA Display Library with Stalk-and-Knob Structures and Selection of High-Affinity Target (Thrombin) Binders

### 1) Sequence elements of the proposed mRNA display library

A novel mRNA display library encoding the polypeptide chain CTSVHQETKKYQS(X₁₀)SYTYNYEHVDVW (SEQ ID NO:319) was created. The amino acid sequence "CTSVHQETKKYQS...SYTYNYEHVDVW" (SEQ ID NOS: 320 and 370, respectively) was adopted from the stalk-and-knob structure of the CDR H3 region of cow antibodies. X₁₀ refers to ten random amino acids that are being displayed over the top of the knob region during selection. The peptide portion of the mRNA display library is expected to have the following sequences if they are at their full length: "DYKDDDDK" (SEQ ID NO:322) is a FLAG tag that was incorporated to enable efficient purification of the peptide/mRNA fusion molecules during the selection.

The mRNA library encoding the above-mentioned peptide sequences had the following sequences: where "AUG" (SEQ ID NO:324) is the start codon. The corresponding DNA library had the following sequences: ATGACGACGATAAGAAAAAA (SEQ ID NO:326)
where "TAATACGACTCACTATAGGG" (SEQ ID NO:327) is the T7 promoter site.

### 2) PCR amplification of 300-53-N10 library

300-53-N10:
300-48-1 :
300-22-2: TTTTTTCTTATCGTCGTCATCTTTGTAGTC (SEQ ID NO:330)

To synthesize the above-described DNA library, a 162 nucleotide initial DNA library 300-53-N10 and two primers 300-48-1 and 300-22-2 were ordered from IDT. The following PCR reaction was further carried out to amplify and achieve a full-length DNA library:

| PCR mix | |
|---|---|
| | |
| 5X HF buffer (NEB) | 200 ul |
| 100 uM 300-48-1 | 10 ul 1000 pmol |
| 100uM 300-22-2 | 10 ul 1000 pmol |
| 10 uM synthetic template 300-53-N10 | 10 ul, 100 pmol. |
| 10 mM dNTP | 20 ul |
| Water | 745 ul |
| Phusion polymerase (NEB) Hot start | 5 ul |

The mixture was PCR amplified for 6 cycles
98C 30"->(98C 20"->64C 30"->72C 30")6 cycles ->72C 2'

The PCR product was purified with two QIAgen columns, eluted with 26 ul H₂O each for a total of 52 ul. The DNA concentration was then measured and was 244.5 ng/ul.

### 3) Transcription of the above-generated DNA library

| T7 RiboMax (Promega) transcription in 50 ul. | |
|---|---|
| 5X T7 buffer | 10 ul |
| 25 mM rNTP each | 12.5 ul |
| 244.5 ng/ul DNA library | 22.5 ul |
| T7 Enzyme mix | 5 ul |

The reaction mixture was incubated at 37°C for 3h.

Then 5 ul RQ1 DNase was added directly into the reaction mixture, 37°C for 30 min.

The transcripts were purified with RNeasy mini (Qiagen), and eluted in 32 ul H₂O, 2939.6 ng/ul.

### 4) Puromycin spacer ligation

| | |
|---|---|
| 10X T4 RNA ligase buffer (NEB) | 5 ul |
| 10 mM ATP | 1.5 ul |
| 2939.6 ng/ul mRNA library | 10 ul |
| 100 uM puromycin spacer | 20 ul |
| T4 RNA ligase (NEB) | 10 ul |
| H2O | 3.5 ul |

The mRNA was heated at 75°C for 1 min before assembling the ligase reaction, which was then incubated at 15°C for 2 h. The mRNA was purified using an RNeasy column (QIAgen), and was eluted in 30 ul H₂O, 647.4 ng/ul, and was then used for PURExpress translation.

### 5) PURExpress deltaRF123 (NEB) translation of mRNA-spacer

| | |
|---|---|
| Solution A | 20 ul |
| Solution B (minus RF123) | 15 ul |
| RNasin | 1 ul |
| mRNA-spacer (647.4 ng/ul) | 15 ul |

The mixture was incubated at 37°C for 1.5 h. Then 5 ul 20% SDS was added to 2%.

The following steps used anti-FLAG beads to capture the fusion molecules and enabled on-beads washing and recovery

50 ul anti-FLAG M2 magnetic beads (Sigma) were washed with 1X TBSTE+ 2% BSA. The translation reaction was mixed with 350 ul 1X TBSTE + 2% BSA and blocked beads. The reaction was then incubated at 4°C for 30 min, and washed 3X in TE+0.2% Tween 20.

6) RT primer extension was done on the beads and fusion conjugates were eluted as follows:

**Resuspended in 40 ul:**

| | |
|---|---|
| 100 uM reverse primer 300-22-2 | 1 ul |
| 10 mM dNTP | 2 ul |
| Rnase free H2O | 23.5 ul |
| 5X RT buffer (Invitrogen) | 8 ul |
| 0.1 M DTT | 4 ul |
| RNasin (Promega) | 0.5 ul |
| Superscript III (Invitrogen) | 1 ul |

The mixture was incubated at 50°C for 30 min, washed 3X in TE+0.2% Tween 20, and transferred to a new tube.

The mixture was then eluted 2X with 50 ul 6M guanidine HCl in TE, 1 ul 10 mg/ml tRNA, ethanol precipitate, 30' at -20°C.

The mixture was then centrifuged and resuspended in 100 ul 1xTE, and 1xTBSTE/0.2% Tween was added to 1 ml.

### 7) Pre-selection

1 ml of the fusions from the previous step were added to 50 ul of M270 SA beads blocked with TBSTE + 2% BSA at room temperature, and were incubated at room temperature for 30 min, and the supernatant was removed for binding with biotinylated thrombin.

8) Pre-selected fusions were bound to 3 ul biotinylated human thrombin at 1 U/ul at 4°C for 1 h with rotation

### 9) M270 beads capture

25 ul of M270 SA bead suspension was washed and blocked with TBSTE + 2% BSA. The beads were then collected, mixed with 1 ml of each fusion/target complex, and incubated at room temperature for 30 min. The mixture was washed 3X in TBSTE, transferred to a new tube, and the cDNA was eluted with 50 ul of 0.1 N NaOH at room temperature for 3 min. Then 2.5 ul 3M NaAc, pH5.5. BioRad spin column was added and the volume was adjusted to 147 ul with H₂O.

### 10) Amplify library by Phusion PCR

This step of PCR was carried out to amplify the library for the next round of selection, and also to help determine how successful the selection was
PCR mix (200 ul) was prepared:

| | |
|---|---|
| 5X HF buffer (NEB) | 40 ul |
| 10 uM forward primer 300-48-1 | 4 ul |
| 10 uM reverse primer 300-22-2 | 4 ul |
| 10 mM dNTP | 4 ul |
| cDNA | 147 ul |
| Phusion polymerase | 1 ul |

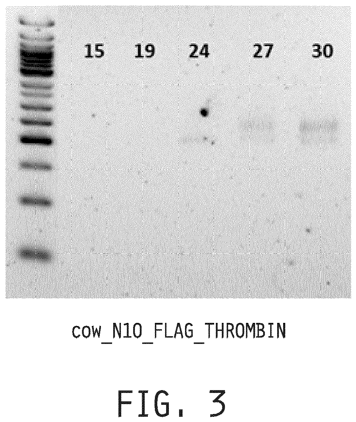

Various PCR cycles were tested and agarose gel electrophoresis was carried out to help determine the most appropriate cycle numbers where enough amplification products can be detected without over-amplification. As illustrated in Figure 3, in this particular case, we tested cycle numbers 15, 19, 24, 27 and 30, and cycle number 24 was used for further PCR amplification.

### 11) Iterative rounds of selections -

The following rounds of selections (3-5) were carried out following the above procedure with increased stringency (e.g. lower amounts of fusion molecule input and increased number of washing steps). After each round of selection, similar PCR amplification was done to prepare the DNA library for the next round of selection.

In summary, for example, one round of mRNA display consisted of the following steps: in vitro transcription, DNase digestion, conjugation with the puromycin oligo linker, in vitro translation/fusion formation, FLAG fusion molecule purification, reverse transcription, pre-selection or functional selection, and regeneration of the selected sequences. Briefly, the cDNA library was in vitro transcribed using T7 RNA polymerase, the mRNA templates with puromycin at the 3' ends were generated by crosslinking with an oligonucleotide containing a psoralen residue and a puromycin residue at its 5' and 3' ends, respectively, and in vitro translation was performed using an NEB Purexpress Δ123 kit. The desired mRNA-protein fusions were then isolated from the translation reaction mixture using anti-FLAG magnetic beads. The fusion molecules were then converted into DNA/RNA hybrids through reverse transcription. This step removed secondary mRNA structures that might interfere with the subsequent selection. The resulting mRNA-displayed synthetic peptide library was then used for selection.

### 12) Results and analysis

To analyze the results of the selection, the DNA libraries were amplified after each round of selection using specially-designed sequencing primers that harbor Illumina next-generation-sequencing adapters. The libraries were mixed, further purified and next generation sequencing was performed. Peptide sequences recovered after each round of selection were called out according to their unique sequencing barcode and further comparison and analysis was done. Next-generation-sequencing provided the advantages of high throughput and a more complete overview of the selected species.

### Example 6

### Selection Progress Against Thrombin and Sequences of Selected Peptides

When thrombin was used as a target, a total of three rounds of selections were carried out. The selection pressure was increased by increasing the washing stringency (from three times to five times then to ten times for selection round 1, 2 and 3). Table 1 illustrates the top binders (ranked according to their frequency) after each round of selection. After selection round 3, more than 30% of the selected peptides contained the conserved "RDPGR" (SEQ ID NO:331) motif The most abundant peptide "RDPGRLIFQS" (SEQ ID NO:332) was chosen for further analysis.

**Table 1. Top binders for human thrombin**

| **Round 1** | | | **Round 2** | | | **Round 3** | | |
|---|---|---|---|---|---|---|---|---|
| **sequence** | **counts** | **percentage (%)** | **sequence** | **counts** | **percentage (%)** | **sequence** | **counts** | **percentage (%)** |
| (SEQ ID NO:333) SQSERATPRY | 47 | 1.5E-03 | (SEQ ID NO:343) RDPGRLIFQS | 2642 | 0.1608 | (SEQ ID NO:353) RDPGRLIFQS | 170914 | 20.512 |
| (SEQ ID NO:334) RFHLFILGTS | 46 | 1.4E-03 | (SEQ ID NO:344) RDPGRVIFEI | 1632 | 0.0993 | (SEQ ID NO:354) RDPGRVIFEI | 83638 | 10.038 |
| (SEQ ID NO:335) PPVLPQL VLL | 44 | 1.4E-03 | (SEQ ID NO:345) RDPGRIVFNN | 781 | 0.0475 | (SEQ ID NO:355) RDPGRIVFNN | 9534 | 1.144 |
| (SEQ ID NO:336) PLIHPRRGYA | 43 | 1.3E-03 | (SEQ ID NO:346) RDPYNVLISL | 278 | 0.0170 | (SEQ ID NO:356) RDPYNVLISL | 702 | 0.084 |
| (SEQ ID NO:337) HRLQIATVYT | 42 | 1.3E-03 | (SEQ ID NO:347) HRLQIATVYT | 179 | 0.0109 | (SEQ ID NO:357) RSHGQFSFTF | 258 | 0.031 |
| (SEQ ID NO:338) TQPKLTIEQR | 42 | 1.3E-03 | (SEQ ID NO:348) ETRIYIRFHT | 86 | 0.0052 | (SEQ ID NO:358) SLFVEYRITY | 224 | 0.027 |
| (SEQ ID NO:339) HCDTLELRPA | 40 | 1.2E-03 | (SEQ ID NOS: 349 and 371, respectively) RTKIK*NGL* | 86 | 0.0052 | (SEQ ID NO:359) QMSFRFEVRV | 211 | 0.025 |
| (SEQ ID NO:340) LKQTDFRTPI | 39 | 1.2E-03 | (SEQ ID NO:350) VTSTYMFMYA | 84 | 0.0051 | (SEQ ID NO:360) SIEFGLTFSF | 195 | 0.023 |
| (SEQ ID NO:341) KCCTSMVIQL | 38 | 1.2E-03 | (SEQ ID NO:351) AAVRLNSSS* | 75 | 0.0046 | (SEQ ID NO:361) VTSTYMFMYA | 175 | 0.021 |
| (SEQ ID NO:342) LQRAISSYSR | 38 | 1.2E-03 | (SEQ ID NO:352) VVEFHFKWCI | 75 | 0.0046 | (SEQ ID NO:362) QFHLEF AFTL | 164 | 0.020 |

### Example 7

### Binding Characteristics of Selected Thrombin Binders

Linear DNA template 300-53-C encoding the peptide MGCTSVHQETKKYQSRDPGRLIFQSSYTYNYEHVDVWGCGSAWSHPQFE KGS (SEQ ID NO:363) was ordered as purified synthetic single-stranded DNA (ssDNA) from IDT.com as follows:
Primer PC1:
Primer PC2: 5'-TTAACTACCCTTTTCGAACTGCGGATGGCTCCA-3'(SEQ ID NO:366).

The ssDNA (SEQ ID NO:363) was further amplified and converted into double-stranded DNA (dsDNA) by PCR with a high fidelity polymerase according to the following protocol

| | |
|---|---|
| 5X HF buffer (NEB) | 200 ul |
| 100 uM PC1 | 10 ul 1000 pmol |
| 100 uM PC2 | 10 ul 1000 pmol |
| 10 uM synthetic template | 10 ul 100 pmol. |
| 10 mM dNTP | 20 ul |
| Water | 745 ul |
| Phusion polymerase Hot start | 5 ul |

The mixture was PCR amplified for 6 cycles: 98°C 30"->(98°C 20"->64°C 60"->72°C 30") 6 cycles ->72C 2'. The PCR product was then purified with two QIAGEN columns and eluted with 25 ul H₂O each for a total of 50 ul. The DNA concentration was then measured and was adjusted to a final concentration of 250 ng/ul.

An in vitro translation reaction using PURExpress (NEB, E6800) was then assembled on ice in the following order:

| | |
|---|---|
| Solution A: | 10 µl |
| Solution B: | 7.5 µl |
| RNAsin Ribonuclease Inhibitor: | 1 µl |
| Nuclease-free H₂O: | 4.5 µl |
| Template dsDNA: | 2 µl, 250 ng/ul |

The samples were incubated at 37°C for 3 hour. The reaction was then stopped by placing the tube(s) on ice, and the samples were stored at -20°C for future use.

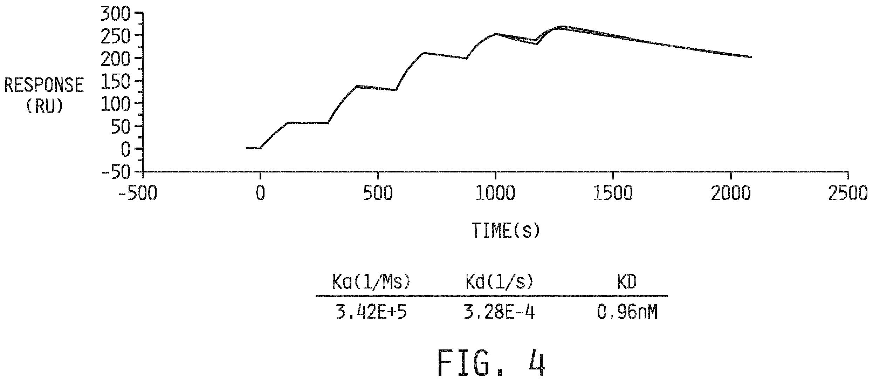

Binding kinetics of crude peptides from the translation mixture were determined by measuring surface plasmon resonance on a BIAcore X100. In brief, 50 ug/ml of strepMAB-Immo (Strep-tag® II specific monoclonal antibody from IBA) in 10 mM NaAc was immobilized onto a sensor chip CM5 that was pre-activated with NHS/EDC. After ethanolamine deactivation, crude translation mixture (10 ul diluted into 113 ul of 1xHBS/EP+ running buffer) was injected. The peptides expressed in the mixture contained a strep-tag II, and were thus captured by the StrepMAB-Immo modified surface. Thrombin with a range of different concentrations (0 nM, 3.125 nM, 6.25 nM, 12.5 nM, 25 nM and 50 nM) was then flown across the surface according to the standard single-cycle kinetics protocol, with each step consisting of a 120-second injection association phase and an 800-second dissociation phase. The sensogram was recorded (see Figure 4) and kinetic constants were derived by global fitting to a 1:1 Langmuir binding model using the BIAcore X100 Evaluation Software.

Two peptides were further prepared (synthesized and purified by Peptide 2.0) with the following sequences: a) Biotin-GCTSVHQETKKYQSRDPGRLIFQSSYTYNYEHVDVW GCG (SEQ ID NO:367) and b) Biotin - GGGGSGGGGSRDPGRLIFQS (SEQ ID NO:368). The binding affinities of each peptide towards thrombin were tested on a Biacore X100 system.

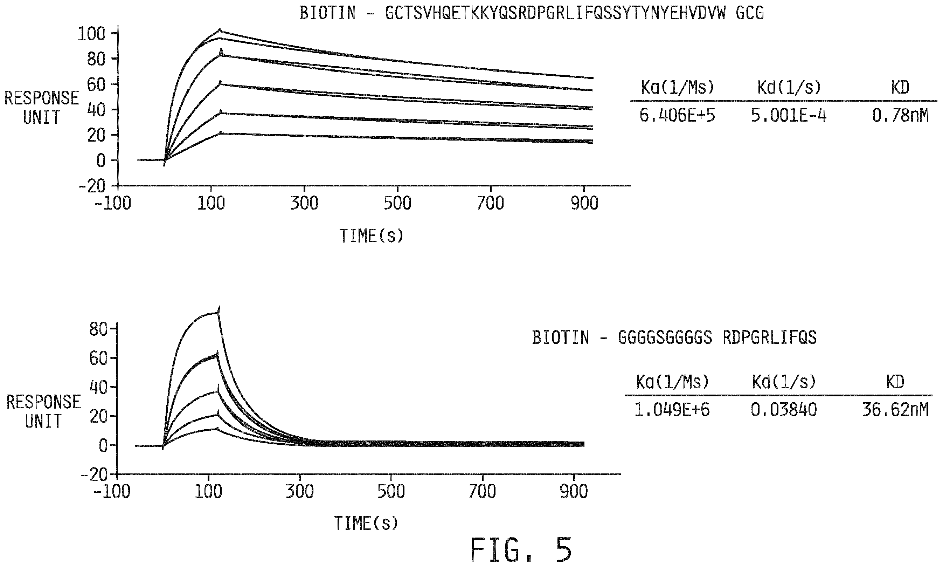

In brief, 50 ul of 100 ug/ml of streptavidin (in 10 mM NaAc, 0.1 mM EDTA, 1 mM NaCl, 1 mM DTT pH 4.6) was immobilized onto a sensor chip CM5 that was pre-activated with NHS/EDC at a flow rate of 5 µl/min. After ethanolamine deactivation, biotinylated peptides (10 ng/ml) were injected and captured. Following the standard protocol for multiple-cycle kinetics, a series of injections of thrombin were then performed at 0 nM, 3.125 nM, 6.25 nM, 12.5 nM, 25 nM and 50 nM. After each injection, the sensor surface was regenerated for 2 min with 10mM Glycine-HCl, pH 1.7 (from GE). The sensogram was recorded (see Figure 5) and kinetic constants were derived by global fitting to a 1:1 Langmuir binding model using the BIAcore X100 Evaluation Software.

As can be seen from the data, when the selected conserved core sequence is put in the context of the cow antibody scaffold, its binding performance towards thrombin was greatly enhanced, as demonstrated by a more than forty-fold increase in the K_{D} values when compared to linear peptide without cow antibody scaffold sequences. The cow antibody scaffold may provide a cyclized conformation (due to the existence of its β strand "stalk"), which in turn increases the affinity of the peptide for its target by decreasing the entropic cost of binding.

## Claims

1. A method of selecting a peptide of interest, the method comprising the steps of:
a) preparing a peptide library using an mRNA-displayed cow antibody scaffold polypeptide comprising the peptide of interest to be identified;
b) selecting the peptide of interest from the peptide library by contacting a target molecule with the peptide of interest wherein the target molecule is immobilized on a solid support or is in solution; and
c) identifying the amino acid sequence of the peptide of interest,
wherein the cow antibody scaffold polypeptide comprises the sequence MGCTSVHQETKKYQS(X*)_{c}SYTYNYEHVDVWGCGSADYKDDDDKKK (SEQ ID NO: 3)
wherein (X*)_{c} is a random sequence of amino acids, and wherein X* is an amino acid sequence and c is the number of amino acids in the random sequence of amino acids.

2. The method of claim 1 wherein the step of preparing the peptide library comprises the step of *in vitro* transcription of a DNA library to form an mRNA library.

3. The method of claim 2 wherein members of the DNA library comprise an RNA polymerase promoter sequence, an enhancer sequence, and a purification tag sequence.

4. The method of claim 2 further comprising the step of translating *in vitro* the mRNA from the mRNA library to form mRNA-cow antibody scaffold polypeptide fusion conjugates comprising the cow antibody scaffold polypeptide wherein the cow antibody scaffold polypeptide comprises a purification tag.

5. The method of claim 4 further comprising the step of purifying the mRNA-cow antibody scaffold polypeptide fusion conjugates using the purification tag.

6. The method of claim 5 further comprising the step of reverse transcribing the mRNA from the mRNA library to form mRNA-DNA duplexes of the mRNA-cow antibody scaffold polypeptide fusion conjugates.

7. The method of claim 6 wherein the step of selecting comprises contacting the mRNA-DNA duplexes of the mRNA-cow antibody scaffold polypeptide fusion conjugates with the target molecule.

8. The method of claim 1 further comprising one of:
i) synthesizing the cow antibody scaffold polypeptide comprising the peptide of interest on a peptide microarray to at least one of mature and extend the peptide of interest; and
ii) synthesizing the peptide of interest on a peptide microarray to mature and/or extend the peptide of interest.

9. The method of claim 8 comprising the steps of:
a) synthesizing the cow antibody scaffold polypeptide comprising the peptide of interest, or derivatives of the peptide of interest, on a first peptide microarray, wherein the derivatives of the peptide of interest include at least one alteration in the sequence of the peptide of interest selected from a single amino acid substitution, a double amino acid substitution, a deletion of one or more amino acids, and an insertion of one or more amino acids, whereby functionalized peptides are generated on the first peptide microarray;
b) forming from the functionalized peptides, wherein the functionalized peptides are in linear form, cyclic peptides of formula VIII 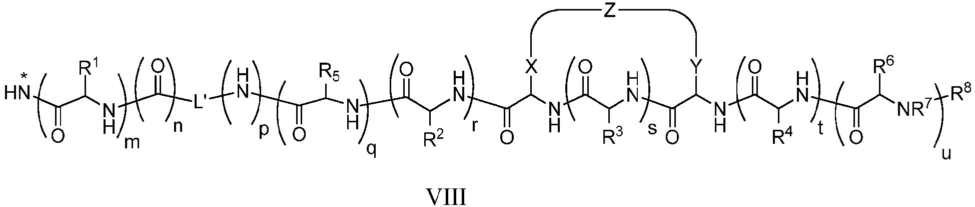
wherein each R¹, R², R³, and R⁴ is independently a natural amino acid side chain or a non-natural amino acid side chain;
each R⁵ and R⁶ is independently a natural amino acid side chain or a non-natural amino acid side chain selected such that 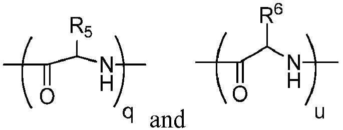 can form a beta-sheet;
each R⁷ is independently selected from the group consisting of hydrogen, an N-terminal capping group, and an N-terminal protecting group;
R⁸ is selected from the group consisting of hydrogen, an N-terminal capping group, and a protecting group;
each X and Y is independently selected from the group consisting of a bond, a natural amino acid side chain covalently attached to Z, and a non-natural amino acid side chain covalently attached to Z;
Z is a group comprising a moiety selected from the group consisting of an amide bond, a disulfide bond, an isopeptide bond, a 1,2,3-triazole, and an optionally substituted 1,2-quinone;
L' is an optional bivalent linking group or a bond;
m is an integer from 0 to 6;
n is 0 or 1;
p is 0 or 1;
q is an integer from 0 to 50;
r is an integer from 0 to 50;
s is an integer from 0 to 50;
t is an integer from 0 to 50;
u is an integer from 0 to 50; and * is a point of connection connecting the cyclic peptide to an array support having a reactive surface;
the method comprising the step of reacting a functionalized peptide of formula IX under conditions that cause Z to form 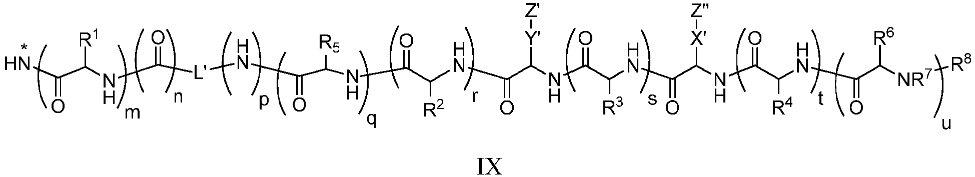
wherein R¹, R² R³, R⁴, R⁵, R⁶, R⁷, and R⁸, m, n, p, q, r, s, t, u, L' and * are as defined for formula VIII;
X' is selected from the group consisting of a bond, a natural amino acid side chain covalently attached to Z", and a non-natural amino acid side chain covalently attached to Z";
Y' is selected from the group consisting of a bond, a natural amino acid side chain covalently attached to Z', and a non-natural amino acid side chain covalently attached to Z'; and
each Z' and Z" is independently selected from the group consisting of a bond, -OH, hydrogen, a thiol, an amine, a carboxylic acid, an amide, an alkyne, an azide, an optionally substituted aminophenol, a natural amino acid side chain, a non-natural amino acid side chain, an N-terminal protecting group, and a C-terminal protecting group, provided that Z' and Z" are complementary groups that combine to form Z;
wherein the functionalized peptides and the cyclic peptides are immobilized to the reactive surface;
c) exposing the cyclic peptides to the target molecule, whereby the target molecule binds to at least one cyclic peptide;
d) identifying one or more of the cyclic peptides demonstrating strong binding to the target molecule, whereby a matured core binder sequence is determined;
e) performing at least one of N-terminal and C-terminal extension of the matured core binder sequence determined in step d to provide a matured, extended core binder sequence on a second peptide microarray;
f) exposing the target molecule to the second peptide microarray comprising a population of matured, extended core binder sequence peptides generated in step e wherein the population of matured, extended core binder sequence peptides comprises cyclic peptides formed as in step b; and
g) identifying a matured, extended cyclic peptide with strong binding to the target molecule.

10. The method of claim 9, wherein Z comprises a moiety selected from the group consisting of an amide bond, 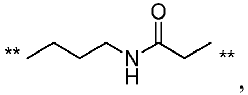 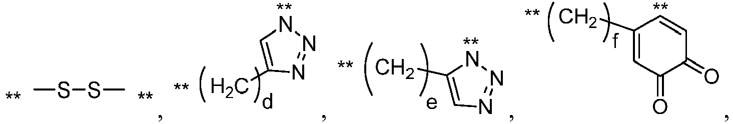 and 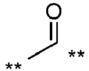 wherein d is an integer from 0 to 6, e is an integer from 0 to 6, and f is an integer from 0 to 6, and ** is a point of connection to the rest of the cyclic peptide.

11. The method of claim 10 further comprising the steps of:
a) synthesizing the peptide of interest, or derivatives thereof, on a first peptide microarray, wherein the derivatives of the peptide of interest include at least one alteration in the sequence of the peptide of interest selected from a single amino acid substitution, a double amino acid substitution, a deletion of one or more amino acids, and an insertion of one or more amino acids, whereby functionalized peptides are generated on the first peptide microarray;
b) forming from the functionalized peptides, wherein the functionalized peptides are in linear form, cyclic peptides of formula I 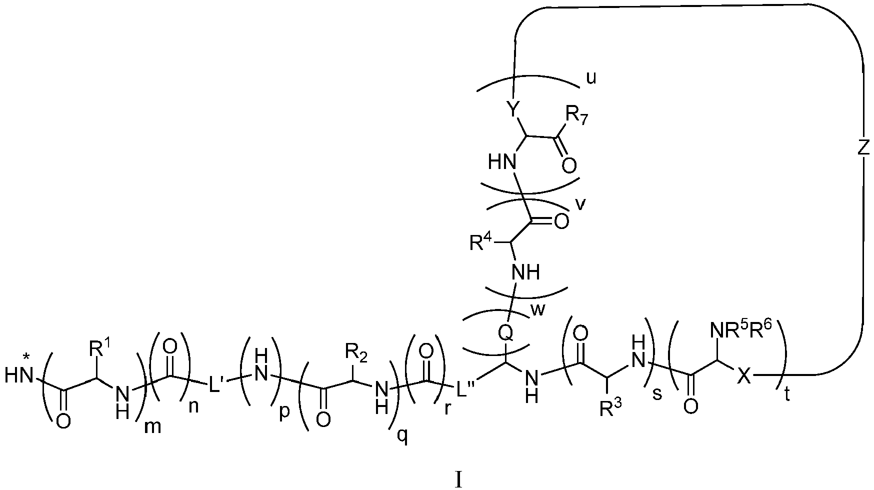
wherein each R¹, R², R³ and R⁴ is independently a natural amino acid side chain or a non-natural amino acid side chain;
each R⁵ and R⁶ is independently selected from the group consisting of hydrogen, an N-terminal capping group, and a N-terminal protecting group;
R⁷ is selected from the group consisting of -OH, a C-terminal capping group, a C-terminal protecting group, and 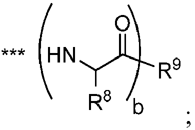
each R⁸ is independently a natural amino acid side chain or a non-natural amino acid side chain;
R⁹ is selected from the group consisting of-OH, a C-terminal capping group, and a C-terminal protecting group;
Q is selected from the group consisting of a bond, a carbonyl, a natural amino acid side chain, and a non-natural amino acid side chain;
each X and Y is independently selected from the group consisting of a bond, a natural amino acid side chain covalently attached to Z, and a non-natural amino acid side chain covalently attached to Z;
Z is a group comprising a moiety selected from the group consisting of an amide bond, a disulfide bond, an isopeptide bond, a 1,2,3-triazole, and an optionally substituted 1,2-quinone;
each L' and L" is independently an optional bivalent linking group or a bond;
b is an integer from 0 to 50;
m is an integer from 0 to 6;
n is 0 or 1;
p is 0 or 1;
q is an integer from 0 to 6;
r is 0 or 1;
s is an integer from 0 to 100;
t is 0 or 1;
u is 0 or 1;
v is an integer from 0 to 100;
w is 0 or 1; and * is a point of connection connecting the cyclic peptide to an array support having a reactive surface; and *** is a point of connection to the rest of the functionalized peptide;
the method comprising the step of reacting a functionalized peptide of formula II under conditions that cause Z to form 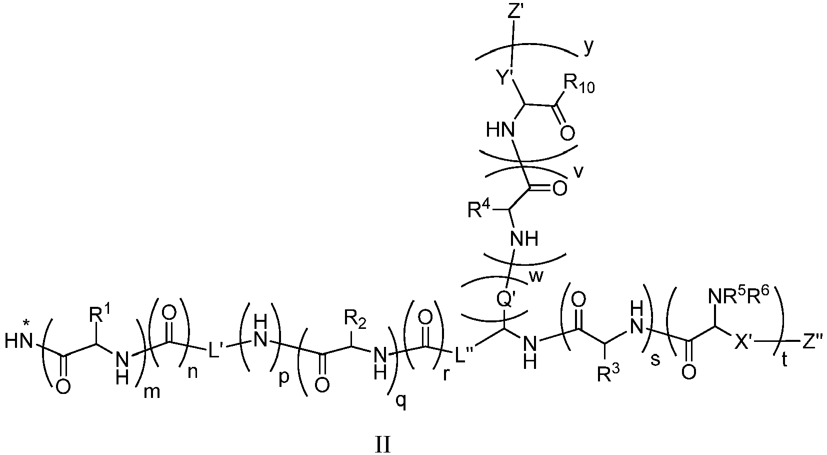
wherein R¹, R² R³, R⁴, R⁵, R⁶, m, n, p, q, r, s, t, v, w, L', L", *, and *** are as defined for formula I;
R¹⁰ is selected from the group consisting of -OH, a C-terminal capping group, a C-terminal protecting group, and 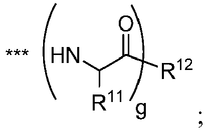
each R¹¹ is independently a natural amino acid side chain or a non-natural amino acid side chain;
R¹² is selected from the group consisting of -OH, a C-terminal capping group, and a C-terminal protecting group;
Q' is selected from the group consisting of a bond, a carbonyl, a natural amino acid side chain covalently attached to Z", and a non-natural amino acid side chain covalently attached to Z";
X' is selected from the group consisting of a bond, a natural amino acid side chain covalently attached to Z", and a non-natural amino acid side chain covalently attached to Z";
Y' is selected from the group consisting of a bond, a natural amino acid side chain covalently attached to Z', and a non-natural amino acid side chain covalently attached to Z';
each Z' and Z" is independently selected from the group consisting of a bond, -OH, hydrogen, a thiol, an amine, a carboxylic acid, an amide, an alkyne, an azide, an optionally substituted aminophenol, a natural amino acid side chain, a non-natural amino acid side chain, an N-terminal protecting group, and a C-terminal protecting group, provided that Z' and Z" are complementary groups that combine to form Z;
g is an integer from 0 to 50; and
y is 0 or 1;
wherein the functionalized peptides and the cyclic peptides are immobilized to the reactive surface;
c) exposing the cyclic peptides to the target molecule, whereby the target molecule binds to at least one cyclic peptide;
d) identifying one or more of the cyclic peptides demonstrating strong binding to the target molecule, whereby a matured core binder sequence is determined;
e) performing at least one of N-terminal and C-terminal extension of the matured core binder sequence determined in step d to provide a matured, extended core binder sequence on a second peptide microarray;
f) exposing the target molecule to the second peptide microarray comprising a population of matured, extended core binder sequence peptides generated in step e wherein the population of matured, extended core binder sequence peptides comprises cyclic peptides formed as in step b; and
g) identifying a matured, extended cyclic peptide with strong binding to the target molecule.

12. The method of claim 11, wherein Z comprises a moiety selected from the group consisting of an amide bond, 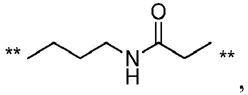 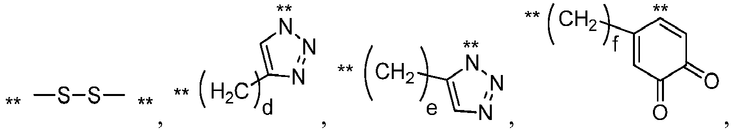 and 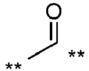 wherein d is an integer from 0 to 6, e is an integer from 0 to 6, and f is an integer from 0 to 6, and ** is a point of connection to the rest of the cyclic peptide.

13. An mRNA-displayed cow antibody scaffold polypeptide comprising a peptide of interest, whereine mRNA-displayed cow antibody scaffold polypeptide comprising the sequence MGCTSVHQETKKYQS(X*)_{c}SYTYNYEHVDVWGCGSADYKDDDDKKK (SEQ ID NO: 3) wherein (X*)_{c} is a random sequence of amino acids, and wherein X* is an amino acid sequence and c is the number of amino acids in the random sequence of amino acids.

## Patentansprüche

1. Verfahren zur Auswahl eines Peptids von Interesse, wobei das Verfahren die folgenden Schritte umfasst:
a) Herstellen einer Peptidbibliothek unter Verwendung eines mRNA-Display-Kuh-Antikörper-Gerüst-Polypeptids, umfassend das zu identifizierende Peptid von Interesse;
b) Auswählen des Peptids von Interesse aus der Peptidbibliothek durch Inkontaktbringen eines Zielmoleküls mit dem Peptid von Interesse, wobei das Zielmolekül auf einem festen Träger immobilisiert oder in Lösung ist; und
c) Identifizieren der Aminosäuresequenz des Peptids von Interesse,
wobei das Kuh-Antikörper-Gerüst-Polypeptid die Sequenz MGCTSVHQETKKYQS(X*)_{c}SYTYNYEHVDVWGCGSADYKDDDDKKK (SEQ ID NO:3) umfasst,
wobei (X*)_{c} eine Zufallssequenz von Aminosäuren ist, und wobei X* eine Aminosäuresequenz ist und c die Anzahl von Aminosäuren in der Zufallssequenz von Aminosäuren ist.

2. Verfahren nach Anspruch 1, wobei der Schritt der Herstellung der Peptidbibliothek den Schritt der *In-vitro*-Transkription einer DNA-Bibliothek zur Bildung einer mRNA-Bibliothek umfasst.

3. Verfahren nach Anspruch 2, wobei Mitglieder der DNA-Bibliothek eine RNA-Polymerase-Promotorsequenz, eine Enhancersequenz und eine Reinigungs-Tag-Sequenz umfassen.

4. Verfahren nach Anspruch 2, ferner umfassend den Schritt der In-vitro-Translation der mRNA aus der mRNA-Bibliothek unter Bildung von mRNA-Kuh-Antikörper-Gerüst-Polypeptid-Fusionskonjugaten, umfassend das Kuh-Antikörper-Gerüst-Polypeptid, wobei das Kuh-Antikörper-Gerüst-Polypeptid einen Reinigungs-Tag umfasst.

5. Verfahren nach Anspruch 4, ferner umfassend den Schritt der Reinigung der mRNA-Kuh-Antikörper-Gerüst-Polypeptid-Fusionskonjugate unter Verwendung des Reinigungs-Tags.

6. Verfahren nach Anspruch 5, ferner umfassend den Schritt der reversen Transkription der mRNA aus der mRNA-Bibliothek zur Bildung von mRNA-DNA-Doppelsträngen der mRNA-Kuh-Antikörper-Gerüst-Polypeptid-Fusionskonjuga

7. Verfahren nach Anspruch 6, wobei der Schritt des Auswählens das Inkontaktbringen der mRNA-DNA-Doppelstränge der mRNA-Kuh-Antikörper-Gerüst-Polypeptid-Fusionskonjugate mit dem Zielmolekül umfasst.

8. Verfahren nach Anspruch 1, ferner umfassend eines von:
i) Synthetisieren des Kuh-Antikörper-Gerüst-Polypeptids, umfassend das Peptid von Interesse, auf einem Peptid-Mikroarray, um das Peptid von Interesse mindestens zur Reife zu bringen und/oder zu verlängern; und
ii) Synthetisieren des Peptids von Interesse auf einem Peptid-Mikroarray, um das Peptid von Interesse zur Reife zu bringen und/oder zu verlängern.

9. Verfahren nach Anspruch 8, umfassend die folgenden Schritte:
a) Synthetisieren des Kuh-Antikörper-Gerüst-Polypeptids, umfassend das Peptid von Interesse oder Derivate des Peptids von Interesse, auf einem ersten Peptid-Mikroarray, wobei die Derivate des Peptids von Interesse mindestens eine Veränderung in der Sequenz des Peptids von Interesse umfassen, ausgewählt aus einer einzelnen Aminosäuresubstitution, einer doppelten Aminosäuresubstitution, einer Deletion einer oder mehrerer Aminosäuren und einer Insertion einer oder mehrerer Aminosäuren, wodurch funktionalisierte Peptide auf dem erstem Peptid-Mikroarray erzeugt werden;
b) Bilden, aus den funktionalisierten Peptiden, wobei die funktionalisierten Peptide in linearer Form vorliegen, von cyclischen Peptiden der Formel VIII 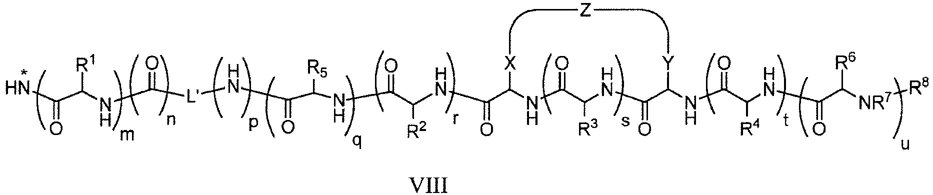
wobei jeder R¹, R², R³ und R⁴ unabhängig voneinander eine natürliche Aminosäureseitenkette oder eine nicht natürliche Aminosäureseitenkette ist;
jeder R⁵ und R⁶ unabhängig voneinander eine natürliche Aminosäureseitenkette oder eine nicht natürliche Aminosäureseitenkette ist, so ausgewählt, dass 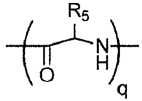 und 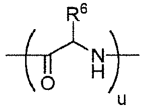 ein Beta-Faltblatt bilden können;
jeder R⁷ unabhängig voneinander ausgewählt ist aus der Gruppe, bestehend aus Wasserstoff, einer N-terminalen Capping-Gruppe und einer N-terminalen Schutzgruppe;
R⁸ ausgewählt ist aus der Gruppe, bestehend aus Wasserstoff, einer N-terminalen Capping-Gruppe und einer Schutzgruppe;
jeder X und Y unabhängig voneinander ausgewählt ist aus der Gruppe, bestehend aus einer Bindung, einer natürlichen Aminosäureseitenkette, die kovalent an Z gebunden ist, und einer nicht natürlichen Aminosäureseitenkette, die kovalent an Z gebunden ist;
Z eine Gruppe ist, die eine Einheit umfasst, die ausgewählt ist aus der Gruppe, bestehend aus einer Amidbindung, einer Disulfidbindung, einer Isopeptidbindung, einem 1,2,3-Triazol und einem gegebenenfalls substituierten 1,2-Chinon;
L' eine optionale zweiwertige Kupplungsgruppe oder eine Bindung ist;
m eine ganze Zahl von 0 bis 6 ist;
n 0 oder 1 ist;
p 0 oder 1 ist;
q eine ganze Zahl von 0 bis 50 ist;
r eine ganze Zahl von 0 bis 50 ist;
s eine ganze Zahl von 0 bis 50 ist;
t eine ganze Zahl von 0 bis 50 ist;
u eine ganze Zahl von 0 bis 50 ist; und * ein Verbindungspunkt ist, der das cyclische Peptid an einen Arrayträger mit einer reaktiven Oberfläche bindet;
wobei das Verfahren den Schritt des Umsetzens eines funktionalisierten Peptids der Formel IX unter Bedingungen umfasst, die -Z dazu bringen, 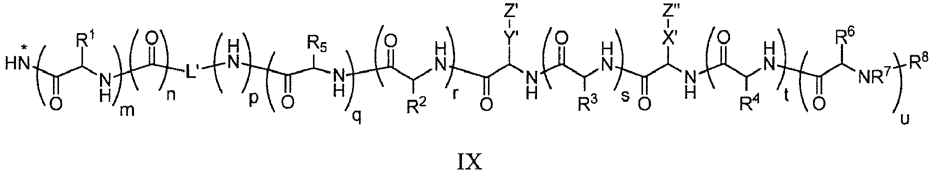 zu bilden,
wobei R¹, R², R³, R⁴, R⁵, R⁶, R⁷ und R⁸, m, n, p, q, r, s, t, u, L' und * wie für Formel VIII definiert sind;
X' ausgewählt ist aus der Gruppe, bestehend aus einer Bindung, einer natürlichen Aminosäureseitenkette, die kovalent an Z" gebunden ist, und einer nicht natürlichen Aminosäureseitenkette, die kovalent an Z" gebunden ist;
Y' ausgewählt ist aus der Gruppe, bestehend aus einer Bindung, einer natürlichen Aminosäureseitenkette, die kovalent an Z' gebunden ist, und einer nicht natürlichen Aminosäureseitenkette, die kovalent an Z' gebunden ist; und
jeder Z' und Z" unabhängig voneinander ausgewählt ist aus der Gruppe, bestehend aus einer Bindung, -OH, Wasserstoff, einem Thiol, einem Amin, einer Carbonsäure, einem Amid, einem Alkin, einem Azid, einem gegebenenfalls substituierten Aminophenol, einer natürlichen Aminosäureseitenkette, einer nicht natürlichen Aminosäureseitenkette, einer N-terminalen Schutzgruppe und einer C-terminalen Schutzgruppe, mit der Maßgabe, dass Z' und Z" komplementäre Gruppen sind, die sich unter Bildung von Z vereinigen;
wobei die funktionalisierten Peptide und die cyclischen Peptide an der reaktiven Oberfläche immobilisiert sind;
c) Exponieren der cyclischen Peptide dem Zielmolekül, wobei das Zielmolekül an mindestens ein cyclisches Peptid bindet;
d) Identifizieren eines oder mehrerer der cyclischen Peptide, die eine starke Bindung an das Zielmolekül demonstrieren, wobei eine gereifte Kernbindersequenz bestimmt wird;
e) Durchführen mindestens eines von N-terminaler und C-terminaler Verlängerung der in Schritt d bestimmten gereiften Kernbindersequenz unter Bereitstellung einer gereiften, verlängerten Kernbindersequenz auf einem zweiten Peptid-Mikroarray;
f) Exponieren des Zielmoleküls dem zweiten Peptid-Mikroarray, umfassend eine Population von in Schritt e erzeugten gereiften, verlängerten Kernbindersequenzpeptiden, wobei die Population gereifter, verlängerter Kernbindersequenzpeptide cyclische Peptide, wie in Schritt b gebildet, umfasst; und
g) Identifizieren eines gereiften, verlängerten cyclischen Peptids mit starker Bindung an das Zielmolekül.

10. Verfahren nach Anspruch 9, wobei Z eine Einheit, ausgewählt aus der Gruppe, bestehend aus einer Amidbindung, 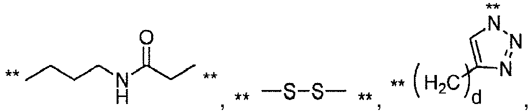 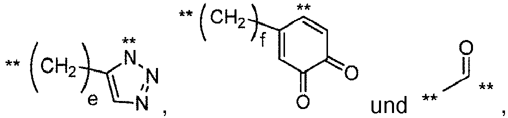 umfasst, wobei d eine ganze Zahl von 0 bis 6 ist, e eine ganze Zahl von 0 bis 6 ist und f eine ganze Zahl von 0 bis 6 ist, und ** ein Verbindungspunkt zum Rest des cyclischen Peptids ist.

11. Verfahren nach Anspruch 10, ferner umfassend die folgenden Schritte:
a) Synthetisieren des Peptids von Interesse oder Derivaten davon auf einem ersten Peptid-Mikroarray, wobei die Derivate des Peptids von Interesse mindestens eine Veränderung in der Sequenz des Peptids von Interesse, ausgewählt aus einer einzelnen Aminosäuresubstitution, einer doppelten Aminosäuresubstitution, einer Deletion einer oder mehrerer Aminosäuren und einer Insertion einer oder mehrerer Aminosäuren, umfassen, wobei funktionalisierte Peptide auf dem ersten Peptid-Mikroarray erzeugt werden;
b) Bilden, aus den funktionalisierten Peptiden, wobei die funktionalisierten Peptide in linearer Form vorliegen, von cyclischen Peptiden der Formel I 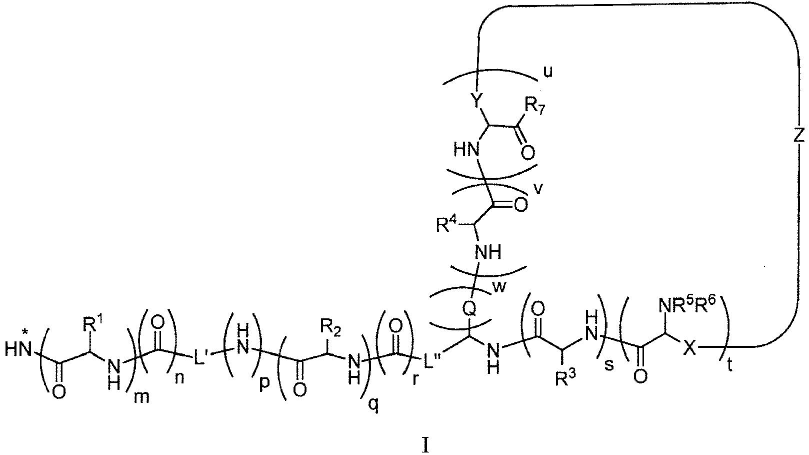
wobei jeder R¹, R², R³ und R⁴ unabhängig voneinander eine natürliche Aminosäureseitenkette oder eine nicht natürliche Aminosäureseitenkette ist;
jeder R⁵ und R⁶ unabhängig voneinander ausgewählt ist aus der Gruppe, bestehend aus Wasserstoff, einer N-terminalen Capping-Gruppe und einer N-terminalen Schutzgruppe;
R⁷ ausgewählt ist aus der Gruppe, bestehend aus -OH, einer C-terminalen Capping-Gruppe, einer C-terminalen Schutzgruppe und 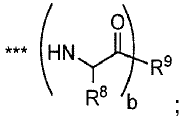
jeder R⁸ unabhängig voneinander eine natürliche Aminosäureseitenkette oder eine nicht natürliche Aminosäureseitenkette ist;
R⁹ ausgewählt ist aus der Gruppe, bestehend aus -OH, einer C-terminalen Capping-Gruppe und einer C-terminalen Schutzgruppe;
Q ausgewählt ist aus der Gruppe, bestehend aus einer Bindung, einem Carbonyl, einer natürlichen Aminosäureseitenkette und einer nicht natürlichen Aminosäureseitenkette;
jeder X und Y unabhängig voneinander ausgewählt ist aus der Gruppe, bestehend aus einer Bindung, einer natürlichen Aminosäureseitenkette, die kovalent an Z gebunden ist, und einer nicht natürlichen Aminosäureseitenkette, die kovalent an Z gebunden ist;
Z eine Gruppe ist, die eine Einheit umfasst, die ausgewählt ist aus der Gruppe, bestehend aus einer Amidbindung, einer Disulfidbindung, einer Isopeptidbindung, einem 1,2,3-Triazol und einem gegebenenfalls substituierten 1,2-Chinon;
jeder L' und L" unabhängig voneinander eine optionale zweiwertige Kupplungsgruppe oder eine Bindung ist;
b eine ganze Zahl von 0 bis 50 ist;
m eine ganze Zahl von 0 bis 6 ist;
n 0 oder 1 ist;
p 0 oder 1 ist;
q eine ganze Zahl von 0 bis 6 ist;
r 0 oder 1 ist;
s eine ganze Zahl von 0 bis 100 ist;
t 0 oder 1 ist;
u 0 oder 1 ist;
v eine ganze Zahl von 0 bis 100 ist;
w 0 oder 1 ist; und * ein Verbindungspunkt ist, der das cyclische Peptid an einen Arrayträger mit einer reaktiven Oberfläche bindet; und *** ein Verbindungspunkt zum Rest des funktionalisierten Peptids ist;
wobei das Verfahren den Schritt der Umsetzung eines funktionalisierten Peptids der Formel II unter Bedingungen umfasst, die Z dazu bringen, 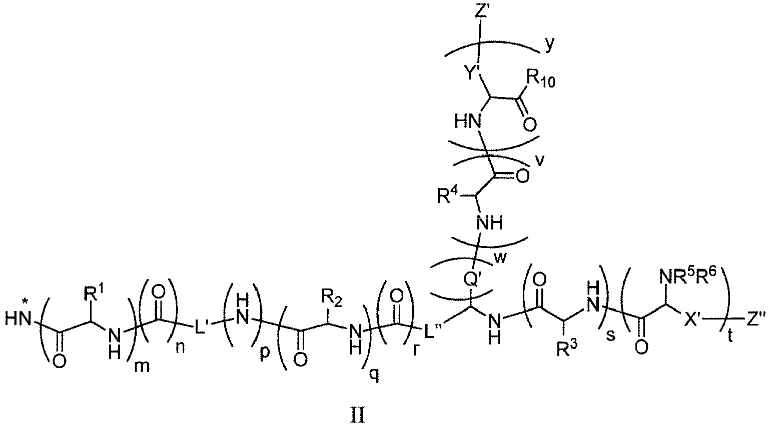 zu bilden
wobei R¹, R², R³, R⁴, R⁵, R⁶, m, n, p, q, r, s, t, v, w, L', L", * und *** wie für Formel I definiert sind;
R¹⁰ ausgewählt ist aus der Gruppe, bestehend aus -OH, einer C-terminalen Capping-Gruppe, einer C-terminalen Schutzgruppe und 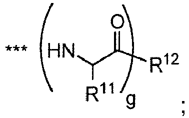
jeder R¹¹ unabhängig voneinander eine natürliche Aminosäureseitenkette oder eine nicht natürliche Aminosäureseitenkette ist;
R¹² ausgewählt ist aus der Gruppe, bestehend aus -OH, einer C-terminalen Capping-Gruppe und einer C-terminalen Schutzgruppe;
Q' ausgewählt ist aus der Gruppe, bestehend aus einer Bindung, einem Carbonyl, einer natürlichen Aminosäureseitenkette, die kovalent an Z" gebunden ist, und einer nicht natürlichen Aminosäureseitenkette, die kovalent an Z" gebunden ist;
X' ausgewählt ist aus der Gruppe, bestehend aus einer Bindung, einer natürlichen Aminosäureseitenkette, die kovalent an Z" gebunden ist, und einer nicht natürlichen Aminosäureseitenkette, die kovalent an Z" gebunden ist;
Y' ausgewählt ist aus der Gruppe, bestehend aus einer Bindung, einer natürlichen Aminosäureseitenkette, die kovalent an Z' gebunden ist, und einer nicht natürlichen Aminosäureseitenkette, die kovalent an Z' gebunden ist;
jeder Z' und Z" unabhängig voneinander ausgewählt ist aus der Gruppe, bestehend aus einer Bindung, -OH, Wasserstoff, einem Thiol, einem Amin, einer Carbonsäure, einem Amid, einem Alkin, einem Azid, einem gegebenenfalls substituierten Aminophenol, einer natürlichen Aminosäureseitenkette, einer nicht natürlichen Aminosäureseitenkette, einer N-terminalen Schutzgruppe und einer C-terminalen Schutzgruppe, mit der Maßgabe, dass Z' und Z" komplementäre Gruppen sind, die sich unter Bildung von Z vereinigen;
g eine ganze Zahl von 0 bis 50 ist und
y 0 oder 1 ist;
wobei die funktionalisierten Peptide und die cyclischen Peptide an der reaktiven Oberfläche immobilisiert sind;
c) Exponieren der cyclischen Peptide dem Zielmolekül, wobei das Zielmolekül an mindestens ein cyclisches Peptid bindet;
d) Identifizieren eines oder mehrerer der cyclischen Peptide, die eine starke Bindung an das Zielmolekül demonstrieren, wobei eine gereifte Kernbindersequenz bestimmt wird;
e) Durchführen mindestens eines von N-terminaler und C-terminaler Verlängerung der in Schritt d bestimmten gereiften Kernbindersequenz unter Bereitstellung einer gereiften, verlängerten Kernbindersequenz auf einem zweiten Peptid-Mikroarray;
f) Exponieren des Zielmoleküls dem zweiten Peptid-Mikroarray, umfassend eine Population von in Schritt e erzeugten gereiften, verlängerten Kernbindersequenzpeptiden, wobei die Population gereifter, verlängerter Kernbindersequenzpeptide cyclische Peptide, wie in Schritt b gebildet, umfasst; und
g) Identifizieren eines gereiften, verlängerten cyclischen Peptids mit starker Bindung an das Zielmolekül.

12. Verfahren nach Anspruch 11, wobei Z eine Einheit umfasst, die ausgewählt ist aus der Gruppe, bestehend aus einer Amidbindung, 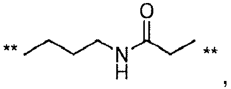 ** -S-S- ** , 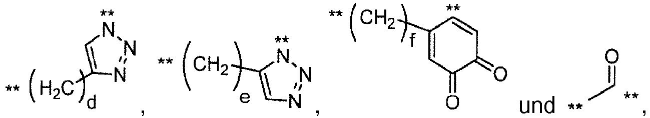 wobei d eine ganze Zahl von 0 bis 6 ist, e eine ganze Zahl von 0 bis 6 ist und f eine ganze Zahl von 0 bis 6 ist, und ** ein Verbindungspunkt zum Rest des cyclischen Peptids ist.

13. mRNA-Display-Kuh-Antikörper-Gerüst-Polypeptid, umfassend ein Peptid von Interesse, wobei das mRNA-Display-Kuh-Antikörper-Gerüst-Polypeptid die Sequenz MGCTSVHQETKKYQS(X*)_{c}SYTYNYEHVDVWGCGSADYKDDDDKKK (SEQ ID NO:3) umfasst, wobei (X*)_{c} eine Zufallssequenz von Aminosäuren ist, und wobei X* eine Aminosäuresequenz ist und c die Anzahl von Aminosäuren in der Zufallssequenz von Aminosäuren ist.

## Revendications

1. Procédé de sélection d'un peptide d'intérêt, le procédé comprenant les étapes de :
a) préparation d'une banque de peptides à l'aide d'un polypeptide d'échafaudage d'anticorps bovin produit par mRNA-display comprenant le peptide d'intérêt à identifier ;
b) sélection du peptide d'intérêt à partir de la banque de peptides par la mise en contact d'une molécule cible avec le peptide d'intérêt où la molécule cible est immobilisée sur un support solide ou est en solution ; et
c) identification de la séquence d'acides aminés du peptide d'intérêt,
dans lequel le polypeptide d'échafaudage d'anticorps bovin comprend la séquence MGCTSVHQETKKYQS(X*)_{c}SYTYNYEHVDVWGCGSADYKDDDDKKK (SEQ ID NO : 3)
où (X*)_{c} est une séquence aléatoire d'acides aminés, et où X* est une séquence d'acides aminés et c est le nombre d'acides aminés dans la séquence aléatoire d'acides aminés.

2. Procédé selon la revendication 1, dans lequel l'étape de préparation de la banque de peptides comprend l'étape de transcription *in vitro* d'une banque d'ADN pour former une banque d'ARNm.

3. Procédé selon la revendication 2 dans lequel des membres de la banque d'ADN comprennent une séquence promotrice d'ARN polymérase, une séquence activatrice et une séquence d'étiquette de purification.

4. Procédé selon la revendication 2 comprenant en outre l'étape de traduction *in vitro* de l'ARNm depuis la banque d'ARNm pour former des conjugués de fusion de polypeptide d'échafaudage d'anticorps bovin d'ARNm comprenant le polypeptide d'échafaudage d'anticorps bovin où le polypeptide d'échafaudage d'anticorps bovin comprend une étiquette de purification.

5. Procédé selon la revendication 4 comprenant en outre l'étape de purification des conjugués de fusion de polypeptide d'échafaudage d'anticorps bovin d'ARNm à l'aide de l'étiquette de purification.

6. Procédé selon la revendication 5 comprenant en outre l'étape de transcription inverse de l'ARNm de la banque d'ARNm pour former des duplexes d'ARNm-ADN des conjugués de fusion de polypeptide d'échafaudage d'anticorps bovin d'ARNm.

7. Procédé selon la revendication 6 dans lequel l'étape de sélection comprend la mise en contact des duplexes d'ARNm-ADN des conjugués de fusion de polypeptide d'échafaudage d'anticorps bovin d'ARNm avec la molécule cible.

8. Procédé selon la revendication 1 comprenant en outre l'une de :
i) la synthèse du polypeptide d'échafaudage d'anticorps bovin comprenant le peptide d'intérêt sur un microréseau peptidique pour au moins une parmi la maturation et l'extension du peptide d'intérêt ; et
ii) la synthèse du peptide d'intérêt sur un microréseau peptidique pour maturer et/ou étendre le peptide d'intérêt.

9. Procédé selon la revendication 8 comprenant les étapes de :
a) synthèse du polypeptide d'échafaudage d'anticorps bovin comprenant le peptide d'intérêt, ou de dérivés du peptide d'intérêt, sur un premier microréseau peptidique, dans lequel les dérivés du peptide d'intérêt comprennent au moins une altération dans la séquence du peptide d'intérêt sélectionnée parmi une seule substitution d'acide aminé, une double substitution d'acides aminés, une délétion d'un ou de plusieurs acides aminés et une insertion d'un ou de plusieurs acides aminés, où les peptides fonctionnalisés sont générés sur le premier microréseau peptidique ;
b) formation à partir des peptides fonctionnalisés, où les peptides fonctionnalisés sont sous forme linéaire, des peptides cycliques de formule VIII 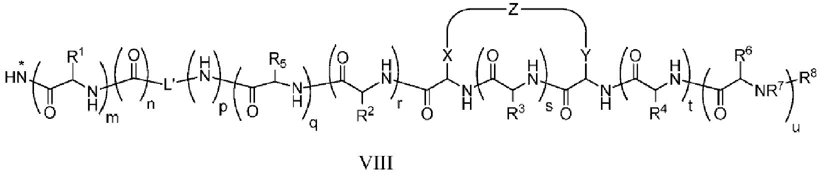
dans laquelle chaque R¹, R², R³ et R⁴ est indépendamment une chaîne latérale d'acides aminés naturelle ou une chaîne latérale d'acides aminés non naturelle ;
chaque R⁵ et R⁶ est indépendamment une chaîne latérale d'acides aminés naturelle ou une chaîne latérale d'acides aminés non naturelle sélectionnée de telle sorte que 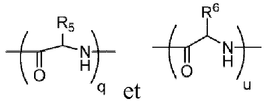 puissent former un feuillet bêta ;
chaque R⁷ est sélectionné indépendamment dans le groupe constitué par l'hydrogène, un groupe de coiffage N-terminal et un groupe de protection N-terminal ;
R⁸ est sélectionné dans le groupe constitué par l'hydrogène, un groupe de coiffage N-terminal et un groupe de protection ;
chaque X et Y est sélectionné indépendamment dans le groupe constitué par une liaison, une chaîne latérale d'acides aminés naturelle attachée de façon covalente à Z, et une chaîne latérale d'acides aminés non naturelle attachée de façon covalente à Z ;
Z est un groupe comprenant un fragment sélectionné dans le groupe constitué par une liaison amide, une liaison disulfure, une liaison isopeptidique, un 1,2,3-triazole et une 1,2-quinone optionnellement substituée ;
L' est un groupe de liaison bivalente optionnel ou une liaison ;
m est un entier de 0 à 6 ;
n représente 0 ou 1 ;
p représente 0 ou 1 ;
q est un entier de 0 à 50 ;
r est un entier de 0 à 50 ;
s est un entier de 0 à 50 ;
t est un entier de 0 à 50 ;
u est un entier de 0 à 50 ; et * est un point de connexion connectant le peptide cyclique à un support de réseau ayant une surface réactive ;
le procédé comprenant l'étape de réaction d'un peptide fonctionnalisé de formule IX dans des conditions qui provoquent Z à former 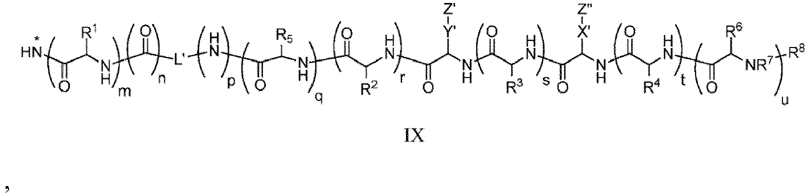
dans laquelle R¹, R², R³, R⁴, R⁵, R⁶, R⁷ et R⁸, m, n, p, q, r, s, t, u, L' et * sont tels que définis pour la formule VIII ;
X' est sélectionné dans le groupe constitué par une liaison, une chaîne latérale d'acides aminés naturelle attachée de façon covalente à Z" et une chaîne latérale d'acides aminés non naturelle attachée de façon covalente à Z" ;
Y' est sélectionné dans le groupe constitué par une liaison, une chaîne latérale d'acides aminés naturelle attachée de manière covalente à Z' et une chaîne latérale d'acides aminés non naturelle attachée de manière covalente à Z' ; et
chaque Z' et Z" est sélectionné indépendamment dans le groupe constitué par une liaison, -OH, un hydrogène, un thiol, une amine, un acide carboxylique, un amide, un alcyne, un azide, un aminophénol optionnellement substitué, une chaîne latérale d'acides aminés naturelle, une chaîne latérale d'acides aminés non naturelle, un groupe protecteur N-terminal et un groupe protecteur C-terminal, à condition que Z' et Z" soient des groupes complémentaires qui se combinent pour former Z ;
où les peptides fonctionnalisés et les peptides cycliques sont immobilisés à la surface réactive ;
c) exposition des peptides cycliques à la molécule cible, de sorte que
la molécule cible se lie à au moins un peptide cyclique ;
d) identification d'un ou plusieurs des peptides cycliques démontrant une liaison forte à la molécule cible, où une séquence de liant de noyau mature est déterminée ;
e) réalisation d'au moins une extension N-terminale et C-terminale de la séquence de liant de noyau mature déterminée à l'étape d afin de fournir une séquence de liant de noyau étendue et mature sur un second microréseau peptidique ;
f) exposition de la molécule cible au second microréseau peptidique comprenant une population de peptides d'une séquence de liant de noyau étendus et matures générés à l'étape e où la population de peptides de séquence de liant de noyau étendus et matures comprend des peptides cycliques formés comme à l'étape b ; et
g) identification d'un peptide cyclique étendu et mature, avec une forte liaison à la molécule cible.

10. Procédé selon la revendication 9, dans lequel Z comprend un fragment sélectionné dans le groupe constitué par une liaison amide, 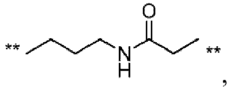 ** -S-S- **, 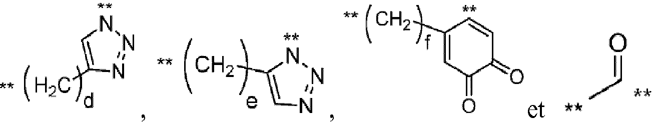 où d est un entier de 0 à 6, e est un entier de 0 à 6 et f est un entier de 0 à 6, et ** est un point de connexion au reste du peptide cyclique.

11. Procédé selon la revendication 10 comprenant en outre les étapes de :
a) synthèse du peptide d'intérêt, ou de dérivés de celui-ci, sur un premier microréseau peptidique, où les dérivés du peptide d'intérêt comprennent au moins une altération dans la séquence du peptide d'intérêt sélectionnée parmi une seule substitution d'acide aminé, une double substitution d'acide aminé, une délétion d'un ou plusieurs acides aminés et une insertion d'un ou plusieurs acides aminés, où les peptides fonctionnalisés sont générés sur le premier microréseau peptidique ;
b) formation à partir des peptides fonctionnalisés, où les peptides fonctionnalisés sont sous forme linéaire, de peptides cycliques de formule I 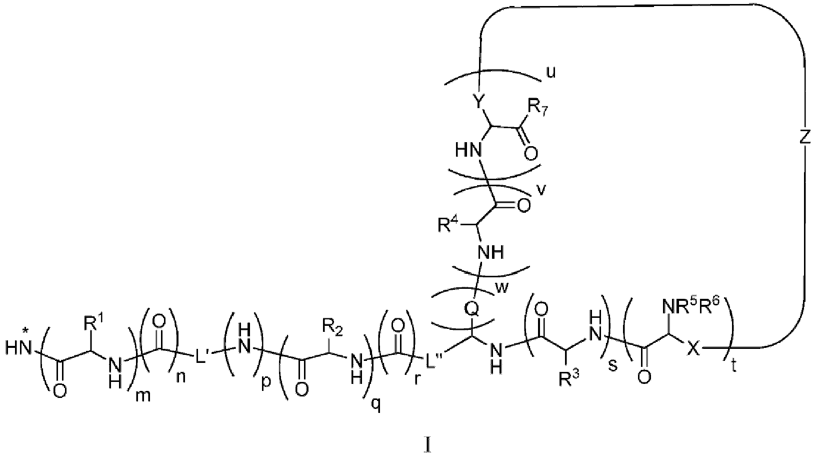
dans laquelle chaque R¹, R², R³ et R⁴ est indépendamment une chaîne latérale d'acides aminés naturelle ou une chaîne latérale d'acides aminés non naturelle ;
chaque R⁵ et R⁶ est indépendamment sélectionné dans le groupe constitué par l'hydrogène, un groupe de coiffage N-terminal et un groupe de protection N-terminal ;
R⁷ est sélectionné dans le groupe constitué par -OH, un groupe de coiffage C-terminal, un groupe de protection C-terminal, et 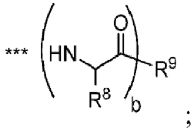
chaque R⁸ est indépendamment une chaîne latérale d'acides aminés naturelle ou une chaîne latérale d'acides aminés non naturelle ;
R⁹ est sélectionné dans le groupe constitué par -OH, un groupe de coiffage C-terminal et un groupe de protection C-terminal ;
Q est sélectionné dans le groupe constitué par une liaison, un carbonyle, une chaîne latérale d'acides aminés naturelle et une chaîne latérale d'acides aminés non naturelle ;
chaque X et Y est sélectionné indépendamment dans le groupe constitué par une liaison, une chaîne latérale d'acides aminés naturelle attachée de façon covalente à Z et une chaîne latérale d'acides aminés non naturelle attachée de façon covalente à Z ;
Z est un groupe comprenant un fragment sélectionné dans le groupe constitué par une liaison amide, une liaison disulfure, une liaison isopeptidique, un 1,2,3-triazole et une liaison 1,2-quinone optionnellement substituée ;
chaque L' et L" est indépendamment un groupe de liaison bivalente optionnelle ou une liaison ;
b est un entier de 0 à 50 ;
m est un entier de 0 à 6 ;
n représente 0 ou 1 ;
p représente 0 ou 1 ;
q est un entier de 0 à 6 ;
r représente 0 ou 1 ;
s est un entier de 0 à 100 ;
t représente 0 ou 1 ;
u représente 0 ou 1 ;
v est un entier de 0 à 100 ;
w représente 0 ou 1 ; et * est un point de connexion reliant le peptide cyclique à un support de réseau ayant une surface réactive ; et *** est un point de connexion au reste du peptide fonctionnalisé ;
le procédé comprenant l'étape de réaction d'un peptide fonctionnalisé de formule II dans des conditions qui provoquent Z à
former 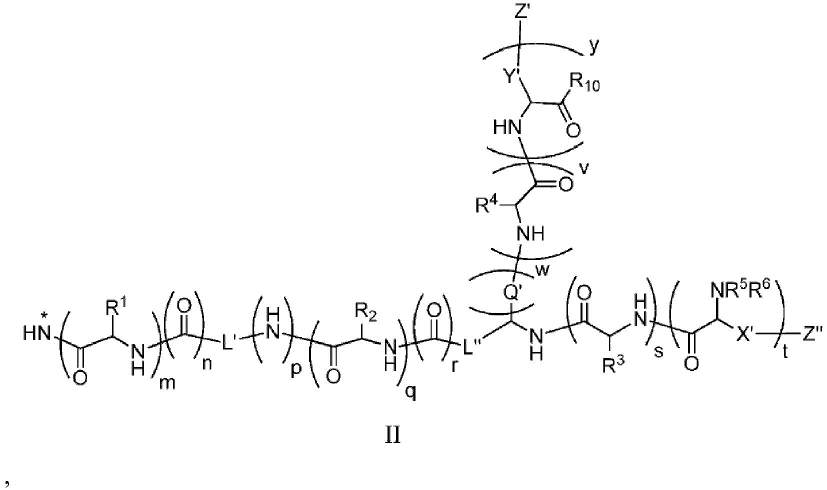
dans laquelle R¹, R², R³, R⁴, R⁵, R⁶, m, n, p, q, r, s, t, v, w, L' L", * et *** sont tels que définis pour la formule I ;
R¹⁰ est sélectionné dans le groupe constitué par -OH, un groupe de coiffage C-terminal, un groupe de protection C-terminal, et 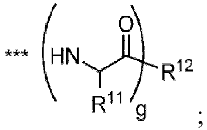
chaque R¹¹ est indépendamment une chaîne latérale d'acides aminés naturelle ou une chaîne latérale d'acide aminée non naturelle ;
R¹² est sélectionné dans le groupe constitué par -OH, un groupe de coiffage C-terminal et un groupe de protection C-terminal ;
Q' est sélectionné dans le groupe constitué par une liaison, d'un carbonyle, une chaîne latérale d'acides aminés naturelle attachée de manière covalente à Z" et une chaîne latérale d'acides aminés non naturelle attachée de manière covalente à Z" ;
X' est sélectionné dans le groupe constitué par une liaison, une chaîne latérale d'acides aminés naturel attachée de façon covalente à Z" et une chaîne latérale d'acides aminés non naturels attachée de façon covalente à Z" ;
Y' est sélectionné dans le groupe constitué d'une par une liaison, d'une chaîne latérale d'acides aminés naturelle attachée de manière covalente à Z' et une chaîne latérale d'acides aminés non naturels attachée de manière covalente à Z' ;
chaque Z' et Z" est sélectionné indépendamment dans le groupe constitué par une liaison, -OH, un hydrogène, un thiol, une amine, un acide carboxylique, un amide, un alcyne, un azide, un aminophénol optionnellement substitué, une chaîne latérale d'acides aminés naturelle, une chaîne latérale d'acides aminés non naturelle, un groupe protecteur N-terminal et un groupe protecteur C-terminal, à condition que Z' et Z" soient des groupes complémentaires qui se combinent pour former Z ;
g est un entier de 0 à 50 ; et
y représente 0 ou 1 ;
où les peptides fonctionnalisés et les peptides cycliques sont immobilisés à la surface réactive ;
c) exposition des peptides cycliques à la molécule cible, de sorte que la molécule cible se lie à au moins un peptide cyclique ;
d) identification d'un ou plusieurs des peptides cycliques démontrant une liaison forte à la molécule cible, où une séquence de liant de noyau mature est déterminée ;
e) réalisation d'au moins une des opérations suivantes : extension N-terminale et C-terminale de la séquence de liant de noyau mature déterminée à l'étape d afin de fournir une séquence de liant de noyau étendue et mature sur un second microréseau peptidique ;
f) exposition de la molécule cible au second microréseau peptidique comprenant une population de peptides d'une séquence de liant de noyau étendus et matures générés à l'étape e où la population de peptides de séquence de liant de noyau étendus et matures comprend des peptides cycliques formés comme à l'étape b ; et
g) identification d'un peptide cyclique étendu et mature, avec une forte liaison à la molécule cible.

12. Procédé selon la revendication 11, dans lequel Z comprend un fragment sélectionné dans le groupe constitué par une liaison amide, 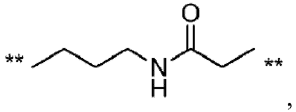 ** -S-S- **, 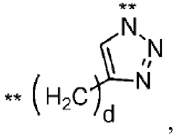 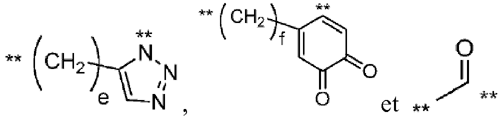 où d est un entier de 0 à 6, e est un entier de 0 à 6 et f est un entier de 0 à 6, et ** est un point de connexion au reste du peptide cyclique.

13. Polypeptide d'échafaudage d'anticorps de bovin produit par mRNA-display comprenant un peptide d'intérêt, le polypeptide d'échafaudage d'anticorps de bovin produit par mRNA-display comprenant la séquence MGCTSVHQETKKYQS(X*)_{c}SYTYNYEHVDVWGCGSADYKDDDDKKK (SEQ ID NO : 3) où (X*)_{c} est une séquence aléatoire d'acides aminés, et où X* est une séquence d'acides aminés et c est le nombre d'acides aminés dans la séquence aléatoire d'acides aminés.
